(19) Europäisches Patentamt / European Patent Office / Office européen des brevets

(11) EP 3 083 880 B1

(12) # EUROPÄISCHE PATENTSCHRIFT

(45) Veröffentlichungstag und Bekanntmachung des Hinweises auf die Patenterteilung:
**01.04.2020 Patentblatt 2020/14**

(21) Anmeldenummer: **14801936.7**

(22) Anmeldetag: **21.11.2014**

(51) Int Cl.:
***C09K 11/06*** *(2006.01)*

(86) Internationale Anmeldenummer:
**PCT/EP2014/003120**

(87) Internationale Veröffentlichungsnummer:
**WO 2015/090504 (25.06.2015 Gazette 2015/25)**

(54) **HETEROCYCLISCHE SPIROVERBINDUNGEN**

HETEROCYCLIC SPIRO COMPOUNDS

COMPOSÉS SPIRANIQUES HÉTÉROCYCLIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.12.2013 EP 13005938**

(43) Veröffentlichungstag der Anmeldung:
**26.10.2016 Patentblatt 2016/43**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **MONTENEGRO, Elvira**
  **69469 Weinheim (DE)**
- **JATSCH, Anja**
  **60489 Frankfurt am Main (DE)**
- **PFLUMM, Christof**
  **64291 Darmstadt (DE)**
- **KROEBER, Jonas Valentin**
  **60311 Frankfurt am Main (DE)**
- **GROSSMANN, Tobias**
  **64297 Darmstadt (DE)**
- **EBERLE, Thomas**
  **76829 Landau (DE)**
- **DOBELMANN, Lars**
  **64283 Darmstadt (DE)**

(56) Entgegenhaltungen:
**WO-A1-2013/100464**
**DE-A1-102009 032 922**
**JP-A- 2009 266 927**
**US-A1- 2013 256 645**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 083 880 B1

## Beschreibung

Die vorliegende Erfindung betrifft Spiroverbindungen mit mindestens einer elektronentransportierenden Gruppe, welche sich für den Einsatz in organischen Elektrolumineszenzvorrichtungen eignen. Weiterhin betrifft die vorliegende Erfindung Verfahren zu deren Herstellung und organische Elektrolumineszenzvorrichtungen.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische Feldeffekt-Transistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Elektronische Vorrichtungen, die Spiroverbindungen mit mindestens einer elektronentransportierenden Gruppe enthalten, werden unter anderem in der Druckschrift WO 2013/100464 A1 beschrieben. Die darin explizit dargelegten Spiroverbindungen sind lediglich an den 2', 7'- Positionen der Ringe mit elektronenleitenden Gruppen substituiert, wobei in dieser Zählweise die Spiroverbindung an Positionen 3 und 4 so substituiert ist, dass zusammen mit einem Arylring des Spirogrundgerüsts eine Dibenzofuran-Gruppe gebildet wird.

US 2013/256645A1 offenbart unter anderem Spirobilfuorene mit ankondensierten Benzothiophengruppen, die mit Triazinen weiter substituiert sind, sowie deren Verwendung in organischen lichtemittierenden Dioden.

WO 2013/100464 A1 offenbart unter anderem Spirobifluorene mit ankondensierten Benzofuran- oder Benzothiophengruppen, wobei die Spirobifluorene in spezifischer Weise aromatisch oder heteroaromatisch substituiert sind, sowie deren Verwendung in organischen optoelektronischen Elementen.

DE 102009032922 A1 offenbart Spirobifluorenderivate sowie deren Verwendung in organischen lichtemittierenden Dioden.

JP 2009/266927 A offenbart unter anderem kondensierte Spirobifluorenderivate sowie deren Verwendung in organischen elektrolumineszierenden Elementen.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern.

Zu diesen Eigenschaften gehört insbesondere die Verarbeitbarkeit der zur Herstellung der elektronischen Vorrichtungen eingesetzten Verbindungen. So sind bekannte Verbindungen relativ oxidationsempfindlich in Lösung. Weiterhin zeigen bekannte Verbindungen eine sehr begrenzte Löslichkeit. Ferner neigen bekannte Verbindungen bei hohen Temperaturen zu einer starken Zersetzung, die einer Sublimation entgegensteht. Ein weiteres Problem stellt die Energieeffizienz dar, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein. Ein weiteres Problem stellt insbesondere die Lebensdauer der elektronischen Vorrichtungen dar.

Aufgabe der vorliegenden Erfindung ist daher die Bereitstellung neuer Verbindungen, welche zu organischen

Elektrolumineszenzvorrichtungen mit verbesserten Eigenschaften führen. Insbesondere ist die Aufgabe Lochblockiermaterialien, Elektroneninjektionsmaterialien und/oder Elektronentransportmaterialien bereitzustellen, welche verbesserte Eigenschaften in Bezug auf Verarbeitbarkeit, Handhabung, Effizienz, Betriebsspannung und/oder Lebensdauer zeigen. Weiterhin sollten sich die Verbindungen möglichst einfach verarbeiten lassen, insbesondere eine gute Löslichkeit und Filmbildung zeigen.

Eine weitere Aufgabe kann darin gesehen werden, organische Elektrolumineszenzvorrichtungen mit einer ausgezeichneten Leistungsfähigkeit möglichst kostengünstig und in konstanter Qualität bereitzustellen.

Weiterhin sollten die organischen Elektrolumineszenzvorrichtungen für viele Zwecke eingesetzt oder angepasst werden können. Insbesondere sollte die Leistungsfähigkeit der organischen Elektrolumineszenzvorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Verbindungen mit allen Merkmalen des Patentanspruchs 1 gelöst werden. Zweckmäßige Abwandlungen der erfindungsgemäßen Verbindungen werden in den auf Anspruch 1 rückbezogenen abhängigen Ansprüchen unter Schutz gestellt.

Gegenstand der Erfindung ist somit eine Verbindung, umfassend mindestens eine Struktur der Formel (I)

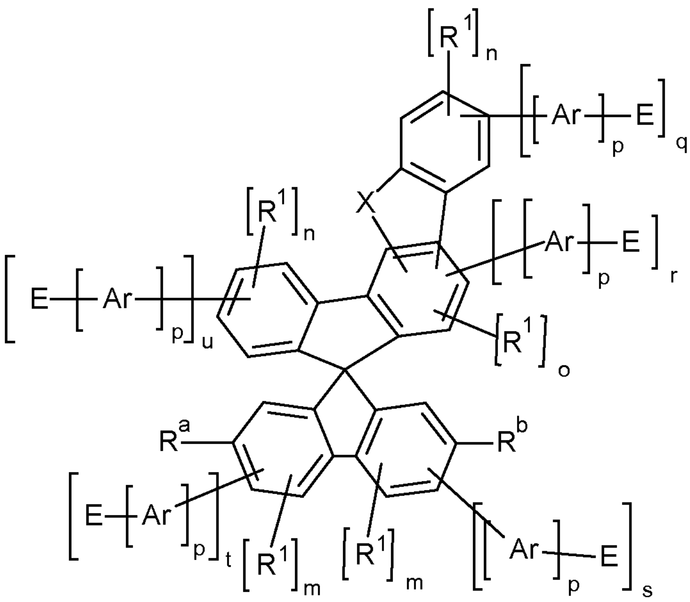

Formel (I)

wobei für die verwendeten Symbole die Definitionen in Anspruch 1 gelten.

Die Gruppe X ist mit dem Spirogrundgerüst und einem Arylrest verbunden, wobei der Arylrest zusätzlich eine Bindung zum Spirogrundgerüst aufweist. Hierbei erfolgt die Bindung zum Arylrest und zur Gruppe X über benachbarte Kohlenstoffatome des Spirogrundgerüsts, so dass eine Dibenzofurangruppe (X = O) gebildet wird.

Gemäß Anspruch 1 handelt es sich bei den Verbindungen um solche der Formel (Ia)

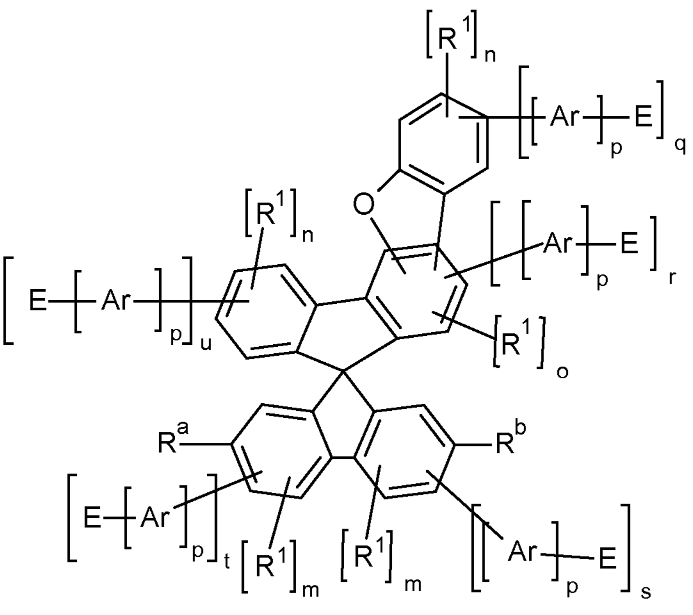

Formel (Ia)

Dabei bedeutet "benachbarte Kohlenstoffatome", dass die Kohlenstoffatome direkt aneinander gebunden sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Zur näheren Erläuterung der nachfolgend dargelegten bevorzugten Ausführungsformen wird im Folgenden die hierin verwendete Zählweise am Spirogrundgerüst dargelegt, wobei die Ringe A und B sowie die Ringe C und D jeweils planar sind. Der Ring E ist direkt mit Ring A über eine Bindung und über die Gruppe X verbunden, wobei die Bindung von X mit dem Ring A an einem C-Atom erfolgt, welches benachbart zu dem C-Atom des Rings A ist, über welches der Ring A mit Ring E verbunden ist.

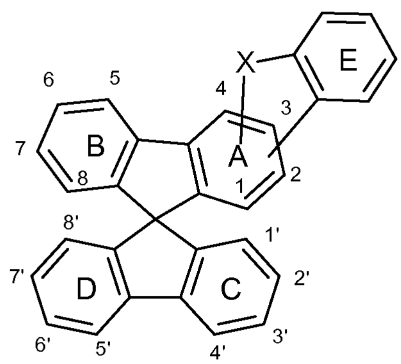

Zählweise am Spirogrundgerüst

Hierbei bilden die Ringe A und E eine Fluoren-, Dibenzothiofuran- oder Dibenzofuran-Gruppe, in bevorzugten Ausführungsformen eine Dibenzothiofuran- oder Dibenzofuran-Gruppe und besonders bevorzugt eine Dibenzofuran-Gruppe.

In den Verbindungen umfassend Strukturen der Formel (I) kann die Summe der Indices q, r, s, t und u kleiner oder gleich 3 sein. Ferner kann die Summe der Indices q, r, s, t und u kleiner oder gleich 3 sein und die Gruppen $R^a$ und $R^b$ enthalten keine elektronentransportierenden Gruppen E. Die Verbindungen können höchstens 3, höchstens 2 oder genau eine elektronentransportierende Gruppe E umfassen.

Für die Verbindungen, bei denen, q gleich 1 ist, kann r = s = 0 sein. Es kann auch q gleich 1 sein und die Summe aus s, t und der in den Gruppen $R^a$ und $R^b$ enthaltenen elektronentransportierenden Gruppen E kleiner oder gleich 1 oder gleich 0 sein. In den Verbindungen kann der Ring E eine elektronentransportierende Gruppe E umfassen und die Ringe C und D keine elektronentransportierende Gruppe E aufweisen.

Ferner kann u gleich 1 sein. Falls u = 1 ist, kann r = s = 0 sein. Wenn u = 1 ist kann die Summe aus s, t und der in den Gruppen $R^a$ und $R^b$ enthaltenen elektronentransportierenden Gruppen E kleiner oder gleich 1 oder gleich 0 sein. Demgemäß können die Verbindungen am Ring B eine elektronentransportierende Gruppe E umfassen und die Ringe C und D keine elektronentransportierende Gruppe E aufweisen.

Darüber hinaus kann o vorzugsweise gleich 1 ist..

In den Verbindungen kannn einer der Reste $R^a$ oder $R^b$ eine Gruppe $-[Ar]_p-E$ oder E sein, wobei Ar, p und E die zuvor genannte Bedeutung haben. Falls einer der Reste $R^a$ oder $R^b$ eine Gruppe $-[Ar]_p-E$ darstellt, kann die Summe aus s und t gleich 0 und die Summe aus q, u und o kleiner gleich 1 oder gleich gleich 0 sein.

Die Verbindungen können in den Position 1, 1', 3, 3', 4, 4', 5, 5', 6, 6', 8, 8' des Spirobifluoren-Grundgerüsts mindestens ein Rest mit einer elektronentransportierenden Gruppe E binden.

Ferner kann vorgesehen sein, dass die Struktur gemäß Formel (I) höchstens 4, höchstens 3, eine oder zwei elektronentransportierende Gruppen E aufweist.

Weiterhin sind Verbindungen gemäß Formel (I) bevorzugt, bei denen die Summe aller n, m und o kleiner oder gleich 4, vorzugsweise kleiner gleich 2 ist. Daher sind Verbindungen bevorzugt, die höchstens 4, besonders bevorzugt höchstens 2 Reste $R^1$ aufweisen.

Darüber hinaus sind Verbindungen bevorzugt, welche sich dadurch auszeichnen, dass p 0 ist, so dass E unmittelbar an die Benzofurangruppes gebunden ist.

Ferner sind Verbindungen bevorzugt, die Strukturen der Formeln (der Formeln (II), (III), (IV), (V), (VI), und/oder (VII)

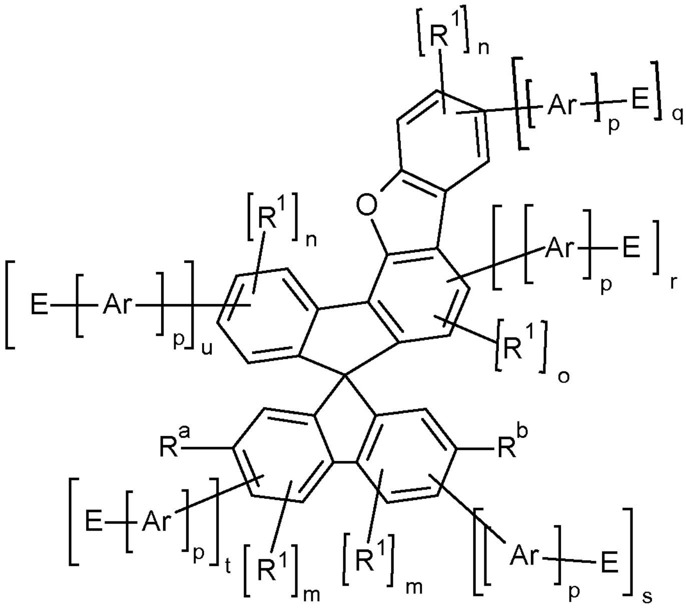

Formel (II)

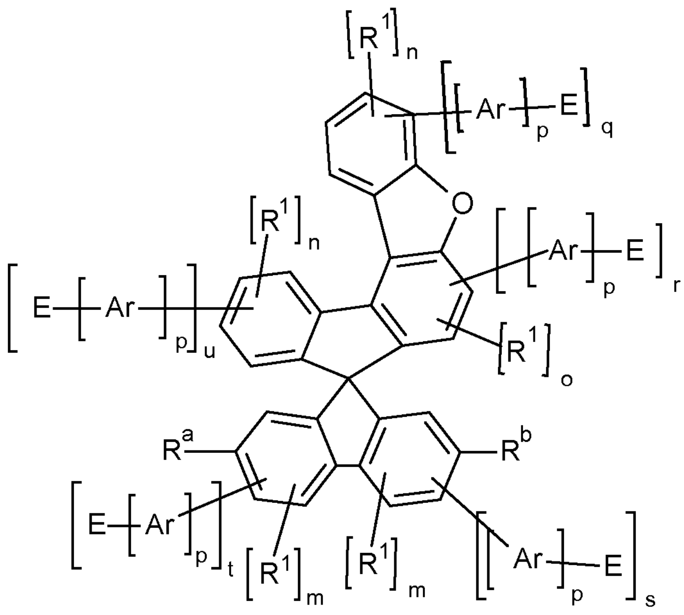

Formel (III)

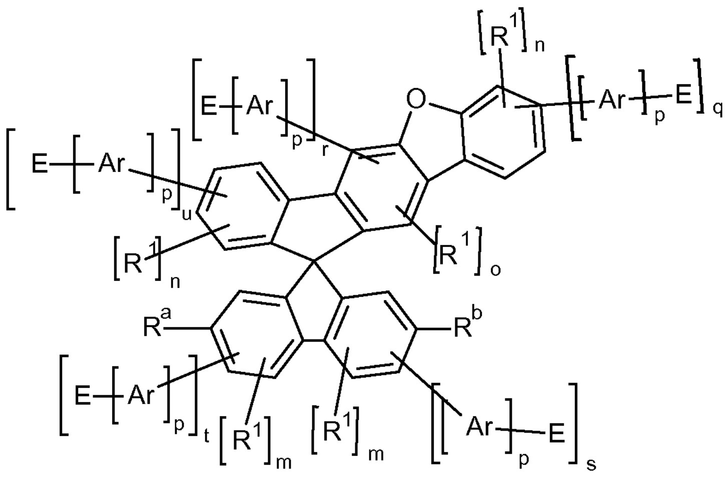

Formel (IV)

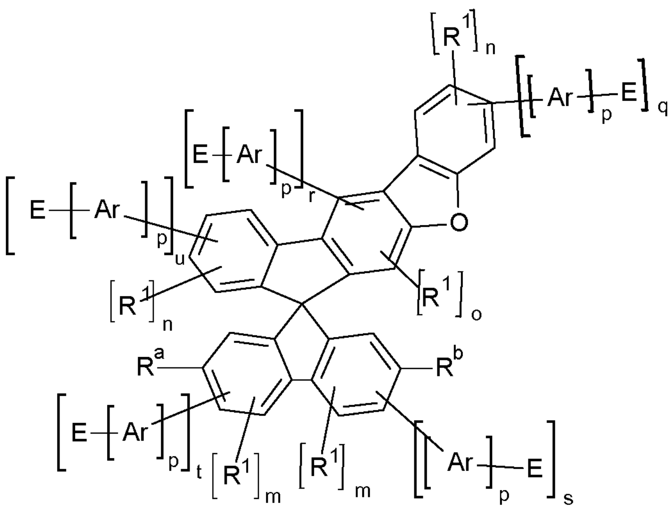

Formel (V)

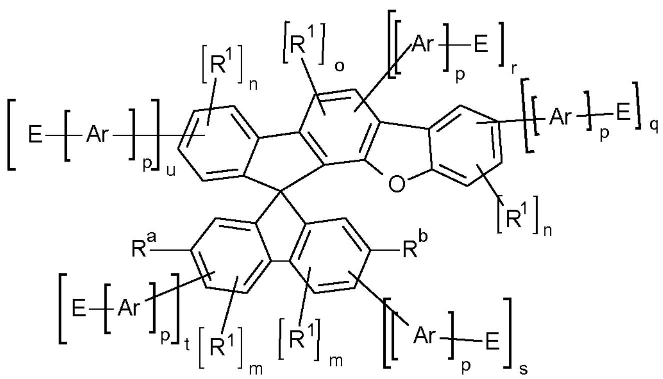

Formel (VI)

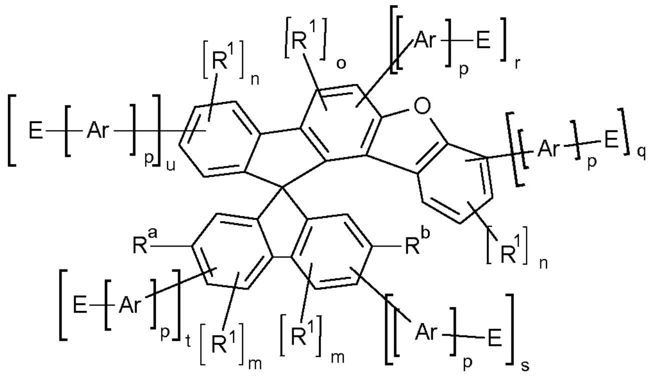

Formel (VII)

umfassen, wobei die dargelegten Symbole die zuvor dargelegte Bedeutung haben.

Von den genannten Verbindungen der Formeln (II) bis (VII) sind Verbindungen gemäß den Formeln (II) und (III) bevorzugt, wobei Verbindungen gemäß den Formel (II) besonders bevorzugt sind.

Gemäß einer bevorzugten Ausführungsform sind die Indices n, m oder o in den Formeln (I), (Ia) und (II) bis (VII) bei jedem Auftreten gleich oder verschieden 0 oder 1.

Vorzugsweise kann die Verbindung mit Strukturen gemäß Formel (I) Reste $R^1$ umfassen, bei denen diese Reste $R^1$ bei jedem Auftreten gleich oder verschieden bevorzugt ausgewählt sind aus der Gruppe bestehend aus H, D, F, Br, I, CN, $Si(R^2)_3$, $B(OR^2)_2$, $C(=O)Ar^1$, einer geradkettigen Alkylgruppe mit 1 bis 10 C-Atomen oder einer geradkettigen Alkoxygruppe mit 1 bis 10 C-Atomen oder einer Alkenylgruppe mit 2 bis 10 C-Atomen oder einer verzweigten oder cyclischen Alkoxygruppe mit 3 bis 10 C-Atomen oder einer verzweigten oder cyclischen Alkylgruppe mit 3 bis 10 C-Atomen, die jeweils mit einem oder mehreren Resten $R^2$ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste $R^2$ substituiert sein kann; dabei können zwei benachbarte Reste $R^1$ oder $R^1$ mit $R^2$ auch miteinander ein mono- oder polycyclisches, aliphatisches oder aromatisches Ringsystem bilden. Besonders bevorzugt sind diese Reste $R^1$ bei jedem Auftreten gleich oder verschieden ausgewählt aus der Gruppe bestehend aus H, D, F, einer geradkettigen Alkoxygruppe mit 1 bis 6 C-Atomen oder einer verzweigten oder cyclischen Alkoxygruppe mit 3 bis 10 C-Atomen, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste $R^2$ substituiert sein kann; dabei können zwei benachbarte Reste $R^1$ oder $R^1$ mit $R^2$ auch miteinander ein mono- oder polycyclisches, aliphatisches oder aromatisches Ringsystem bilden. Besonders bevorzugt kann mindestens einer vorzugsweise beide der Reste $R^1$ in Formel (I) ein Alkylrest mit 1 bis 10 Kohlenstoffatomen oder eine Arylgruppe mit 6 bis 10 Kohlenstoffatomen darstellen, die mit bis zu drei Resten $R^2$ substituiert sein kann.

Die Struktur gemäß Formel (I) umfasst eine elektronentransportierende Gruppe E, die gegebenenfalls mit einem oder mehreren der zuvor näher definierten Resten $R^1$ substituiert sein kann. Eine elektronentransportierende Gruppe E zeichnet sich dadurch aus, dass diese allein oder im Zusammenspiel mit anderen Gruppen die Elektronenleitfähigkeit in einer oder mehreren Schichten einer organischen elektronischen Vorrichtungen verbessert. Im Allgemeinen weisen diese Gruppen eine relativ geringe LUMO (lowest unoccupied molecular orbital) Energie auf.

Bevorzugt kann die elektronentransportierende Gruppe E eine LUMO (lowest unoccupied molecular orbital) Energie aufweisen, die niedriger als -1.3 eV ist, ganz bevorzugt niedriger als -2.5 eV und ganz besonders bevorzugt niedriger als -2.7 eV.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\text{HOMO(eV)} = ((\text{HEh}*27.212)-0.9899)/1.1206$$

$$\text{LUMO(eV)} = ((\text{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Weiter bevorzugt ist die elektronentransportierende Gruppen E dadurch charakterisiert, dass die Elektronenmobilität $\mu_-$ $10^{-6}$ $cm^2/(Vs)$ oder mehr beträgt, ganz bevorzugt beträgt sie $10^{-5}$ $cm^2/(Vs)$ oder mehr und ganz besonders

bevorzugt beträgt sie $10^{-4}$ $cm^2/(Vs)$ oder mehr.

Zu den bevorzugten elektronentransportierenden Gruppen zählen unter anderem elektronenarme heteroaromatische Gruppen sowie Aryl- oder Heteroarylgruppen mit mindestens einer stark elektronenziehenden Gruppe.

Gemäß einer bevorzugten Ausführungsform ist die elektronentransportierende Gruppe E vorzugsweise eine elektronenarme heteroaromatische Gruppe, die mit einem oder mehreren Resten $R^1$ substituiert sein kann. Noch stärker bevorzugt sind heteroaromatische Gruppen mit 6 aromatischen Ringatomen, von denen mindestens eines, bevorzugt 2 und ganz bevorzugt mindestens drei ein N-Atom ist, oder heteroaromatische Gruppen mit 5 aromatischen Ringatomen, von denen mindestens 2 Heteroatome sind, bevorzugt mindestens eines davon ein N-Atom, das mit $R^1$ substituiert sein kann, wobei an diese Gruppen jeweils auch weitere Aryl- oder Heteroarylgruppen ankondensiert sein können.

Gemäß einem besonderen Aspekt der vorliegenden Erfindung kann vorgesehen sein, dass die elektronentransportierende Gruppe E eine Heteroarylgruppe mit 5 bis 60 aromatischen Ringatomen ist, wobei N ganz bevorzugte Heteroatme darstellen, wobei die elektronentransportierende Gruppe E mit einem oder mehreren voneinander unabhängigen Resten $R^1$ substituiert sein kann.

Bevorzugte elektronentransportierende Gruppe E umfassen mindestens eine Struktur, die aus der Gruppe der Pyridine, Pyrazine, Pyrimidine, Pyridazine, 1,2,4-Triazine, 1,3,5-Triazine, Chinoline, Isochinoline, Chinoxaline, Pyrazole, Imidazole, Benzimidazole, Thiazole, Benzothiazole, Oxazole oder Benzooxazole ausgewählt ist, besonders bevorzugt aus der Gruppe der Pyridazine, 1,2,4-Triazine, 1,3,5-Triazine, Pyrimidine, Pyrazine, Imidazole, Benzimidazole und Pyridine. Diese Strukturen können hierbei mit einem oder mehreren voneinander unabhängigen Resten $R^1$ substituiert sein. Noch mehr bevorzugt ist die elektronentransportierende Gruppe ein mit einem oder mehreren Resten $R^1$ substituiertes Pyridin, Pyrazin, Pyrimidin, Pyridazin und 1,3,5-Triazin.

Ferner kann vorgesehen sein, dass dass die elektronentransportierende Gruppe E mindestens eine Struktur gemäß den Formeln (E-1) bis (E-10)

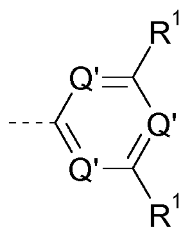
Formel (E-1)

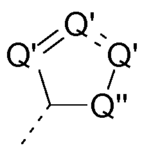
Formel (E-2)

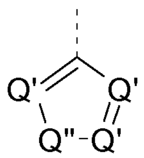
Formel (E-3)

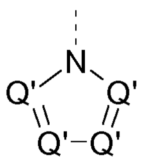
Formel (E-4)

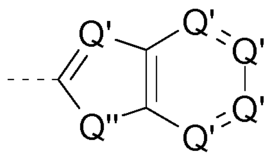
Formel (E-5)

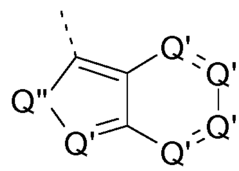
Formel (E-6)

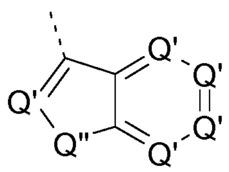
Formel (E-7)

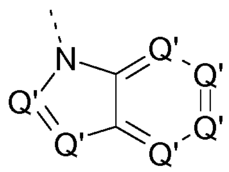
Formel (E-8)

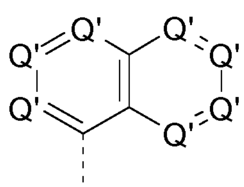
Formel (E-9)

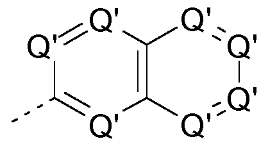

Formel (E-10),

aufweist, wobei die gestrichelte Bindung die Anbindungsposition markiert,

Q' bei jedem Auftreten gleich oder verschieden $CR^1$ oder N darstellt, und

Q" $NR^1$, O oder S darstellt;

wobei wenigstens ein Q' gleich N und/oder wenigstens ein Q" gleich $NR^1$ ist und

$R^1$ wie in zuvor definiert ist.

Besonders bevorzugt kann vorgesehen sein, dass die elektronentransportierende Gruppe E mindestens eine Struktur gemäß den Formeln (E-11) bis (E-19)

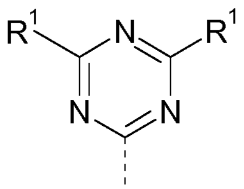

Formel (E-11)

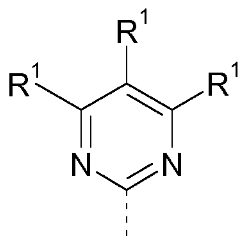

Formel (E-12)

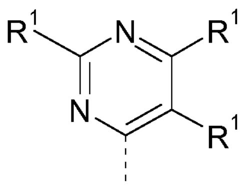

Formel (E-13)

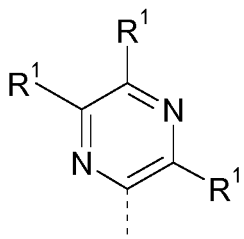

Formel (E-14)

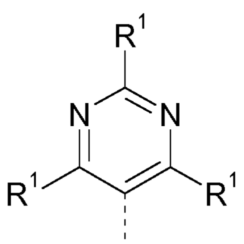

Formel (E-15)

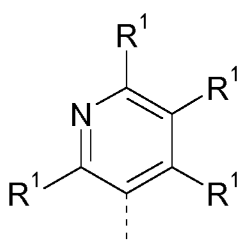

Formel (E-16)

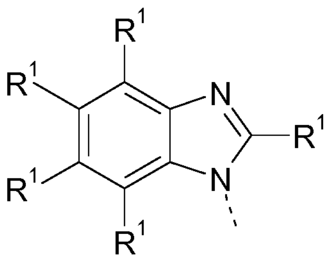

Formel (E-17)

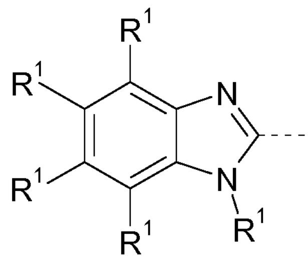

Formel (E-18)

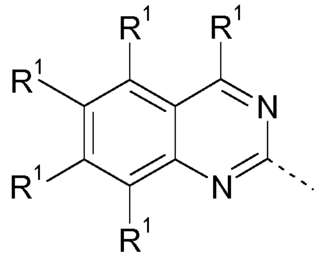

Formel (E-19)

aufweist, wobei die gestrichelte Bindung die Anbindungsposition markiert und $R^1$ die zuvor genannte Bedeutung aufweist.

Im Fall der Formel (E-18) ist bevorzugt, wenn $R^1$ nicht für H und D steht. Vorzugsweise kann für Verbindungen mit Strukturen gemäß Formel (E-1) bis Formel (E-19) mindestens einer vorzugsweise mindestens zwei der Reste $R^1$ für Ar stehen, wobei Ar die zuvor genannte Bedeutung hat.

Die Substituenten $R^1$ in der elektronentransportierende Gruppe E sind vorzugsweise ausgewählt aus der Gruppe bestehend aus H oder ein aromatisches oder heteroaromatisches Ringsystem mit 5 bis 60 aromatischen Ringatomen, das jeweils durch einen oder mehrere Reste $R^2$ substituiert sein kann, wobei die Gruppen der Formel (E-11), (E-17) und (E-18) noch bevorzugter sind und die Gruppe der Formel (E-11) am meisten bevorzugt ist.

Beispiele ganz besonders bevorzugter elektronentransportierender Gruppen E sind die folgenden Gruppen, die mit einem oder mehreren voneinander unabhängigen Resten $R^2$ substituiert sein können, wobei die gestrichelten Bindungen die Anbindungsposition kennzeichnen.

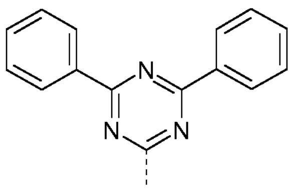

Formel (E-20)

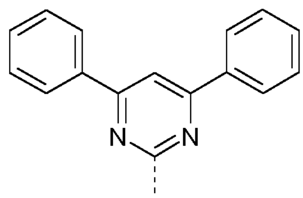

Formel (E-21)

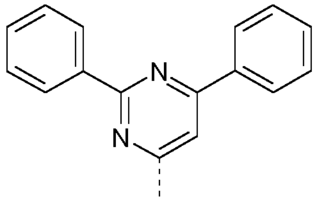

Formel (E-22)

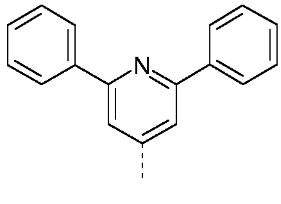

Formel (E-23)

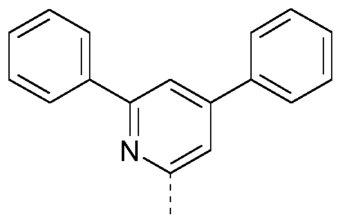

Formel (E-24)

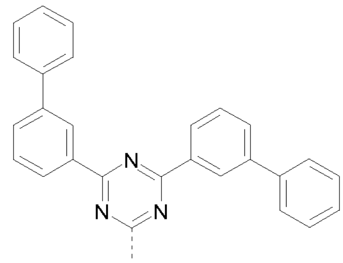

Formel (E-25)

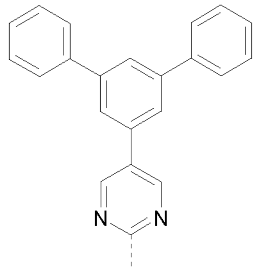

Formel (E-26)

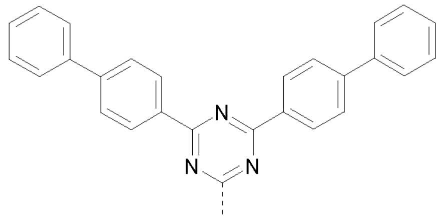

Formel (E-27)

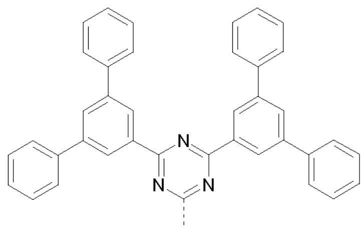

Formel (E-28)

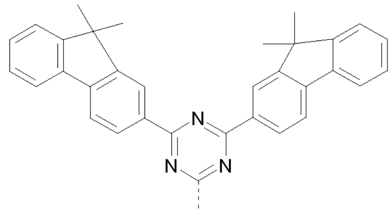

Formel (E-29)

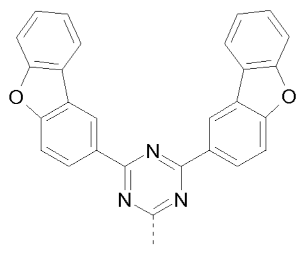

Formel (E-30)

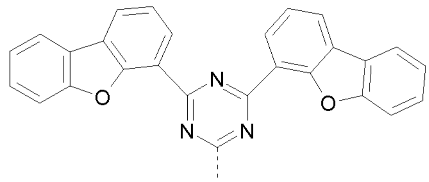

Formel (E-31)

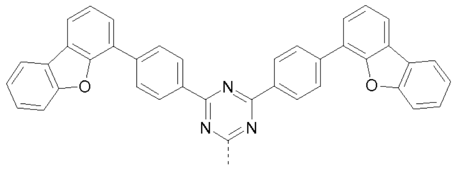

Formel (E-32)

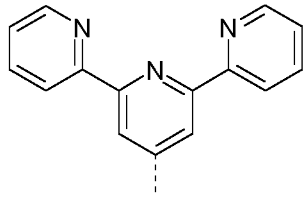

Formel (E-33)

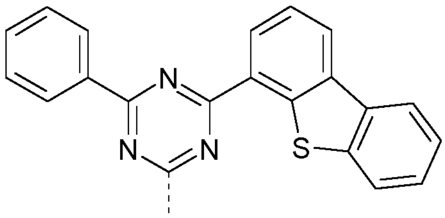

Formel (E-34)

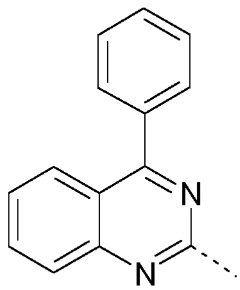

Formel (E-35)

Gemäß einer weiteren Ausführungsform kann die elektronentransportierende Gruppe E mindestens eine stark elektronenziehende Gruppe umfassen, die bevorzugt an einer Aryl- oder Heteroarylgruppen gebunden ist.

Hierbei kann die stark elektronenziehende Gruppe bevorzugt aus den Strukturen (E-36), (E-37) und (E-38)

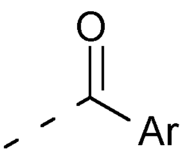

(E-36)

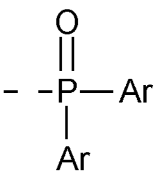

(E-37)

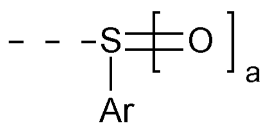

(E-38)

ausgewählt sein, worin

Ar jeweils gleich oder verschieden ist und die zuvor genannte Bedeutung hat;

a 1 oder 2, bevorzugt 2 ist; und

die gestrichelte Linie die Bindung an das Spirobifluoren-Grundgerüst oder, falls p = 1 ist, gegebenenfalls an die Gruppe Ar darstellt, wobei diese Gruppe Ar an das Spirobifluoren-Grundgerüst gebunden ist.

Hierbei kann eine erfindungsgemäße Verbindung lediglich elektronenarme heteroaromatische Gruppen oder Aryl- oder Heteroarylgruppen mit mindestens einer stark elektronenziehenden Gruppe als elektronentransportierende Gruppe umfassen. Falls die erfindungsgemäße Verbindung zwei oder mehr elektronentransportierende Gruppen aufweist, kann die Verbindung sowohl mindestens eine elektronenarme heteroaromatische Gruppe als auch mindestens eine Aryl- oder Heteroarylgruppen mit mindestens einer stark elektronenziehenden Gruppe aufweisen.

Insbesondere weisen die erfindungemäßen Verbindungen ein im Vergleich zu Verbindungen des Standes der Technik deutlich höheres Triplett T1-Niveau auf, wobei dies für den Bau von grünen und insbesondere von blauen phosphoreszierenden OLEDs von Vorteil ist. Dieser Vorteil ist insbesondere für den Einsatz der Materialien als Triplettmatrixmaterial (TMM), Lochblockiermaterial (HBM) und Elektronentransportmaterial (ETM) wesentlich. In der emittierenden Schicht und den angrenzenden Schichten (EBM / ETM) sollte das T1-Niveau von TMM, EBH, ETM größer oder gleich sein, als die des emittierenden Materials, um ein Quenschen der Emission zu vermeiden.

Die erfindungsgemäßen Spiroverbindungen, umfassend Strukturen der Formel (I), können je nach Struktur auch chiral sein. Dies ist insbesondere dann der Fall, wenn sie Substituenten enthalten, beispielsweise Alkyl-, Alkoxy oder Aralkylgruppen, welche ein oder mehrere Stereozentren aufweisen. Da es sich bei der Grundstruktur der borenthaltenden Verbindung auch um eine chirale Struktur handeln kann, ist die Bildung von Diastereomeren und mehreren Enantiomerenpaaren möglich. Die erfindungsgemäßen Verbindungen umfassen dann sowohl die Mischungen der verschiedenen Diastereomere bzw. die entsprechenden Racemate wie auch die einzelnen isolierten Diastereomere bzw. Enantiomere.

Vorzugsweise kann die Verbindung in Form einer Enantiomerenmischung, besonders bevorzugt einer Diastereomerenmischung vorliegen. Hierdurch können die Eigenschaften von elektronischen Vorrichtungen, die unter Verwendung der erfindungsgemäßen Verbindungen erhältlich sind, unerwartet gesteigert werden. Zu diesen Eigenschaften gehören insbesondere die Lebensdauer der Vorrichtungen.

Beispiele erfindungsgemäßer und nicht-erfindungsgemäßer Verbindungen umfassen Strukuren gemäß den folgenden Formeln 1 bis 141:

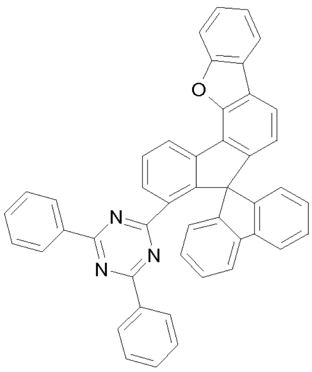

1

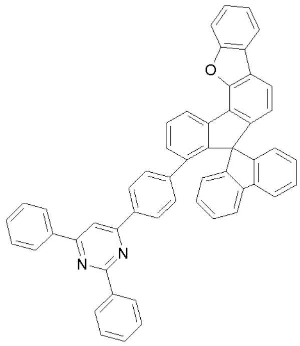

2

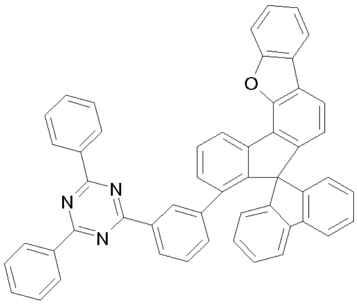

3

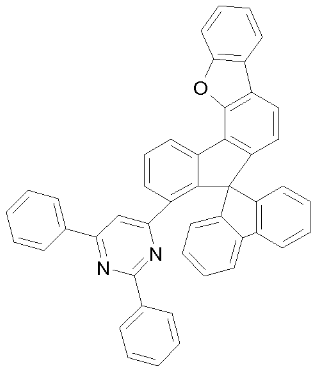

4

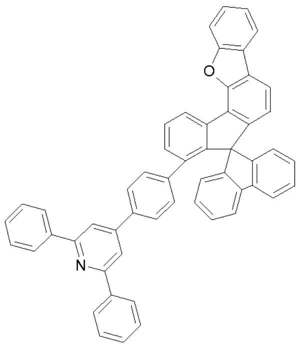

5

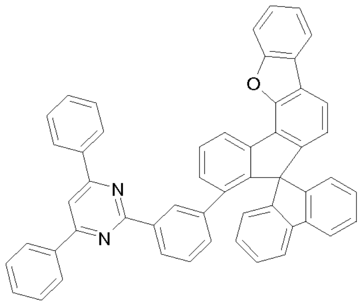

6

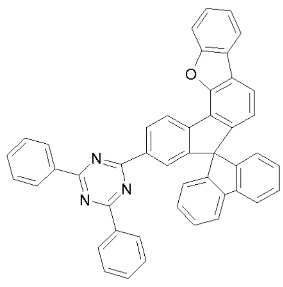

7

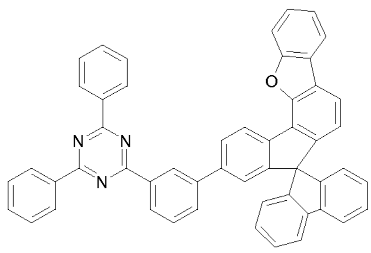

8

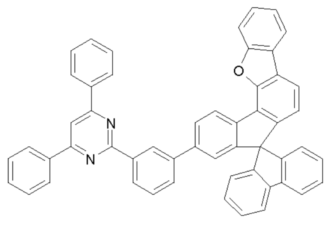

9

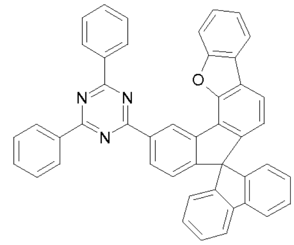

10

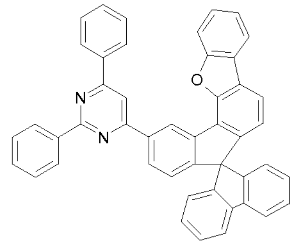

11

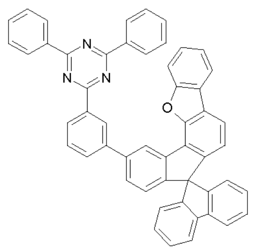

12

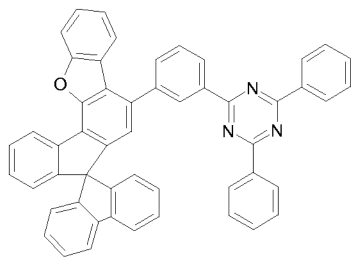

13

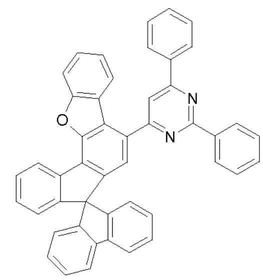

14

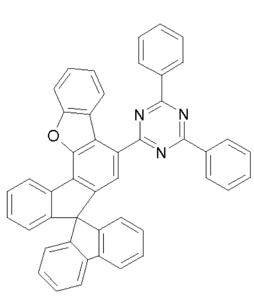

15

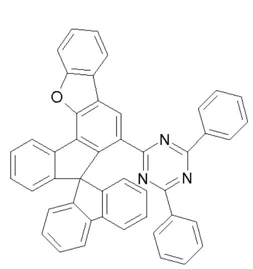

16

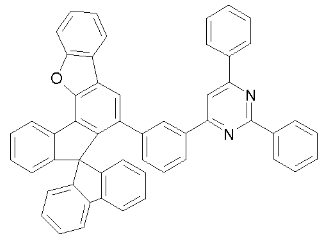

17

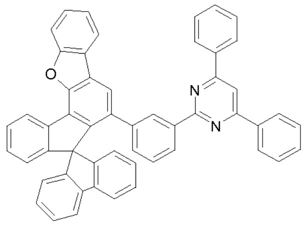

18

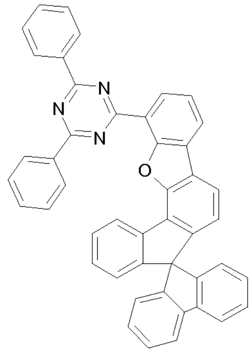

19

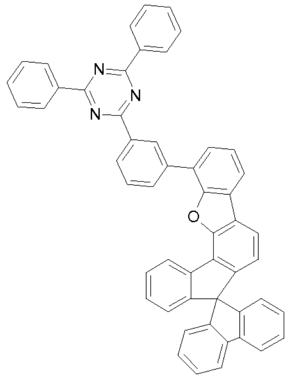

20

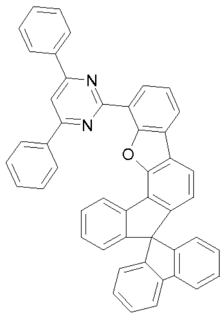

21

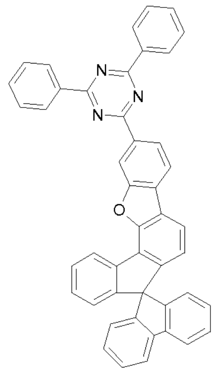

22

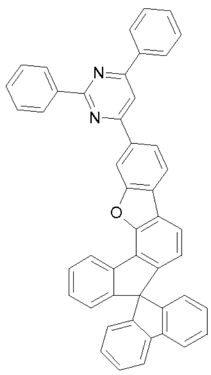

23

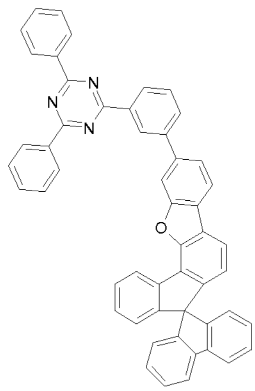

24

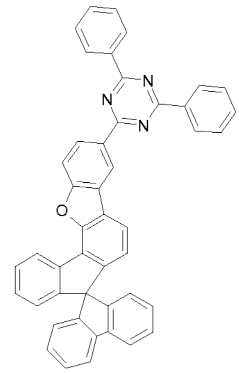

25

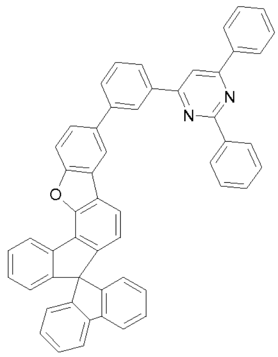

26

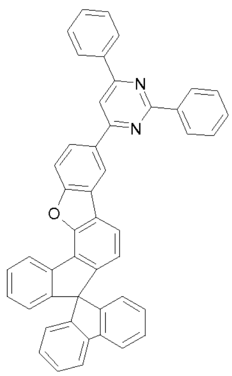

27

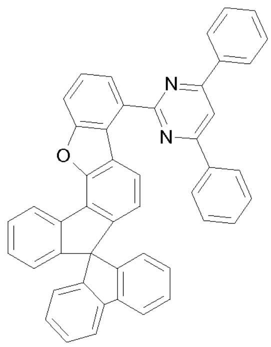

28

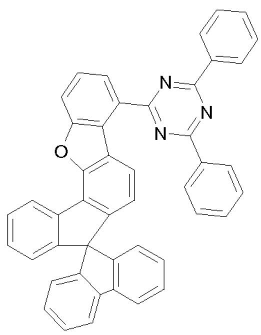

29

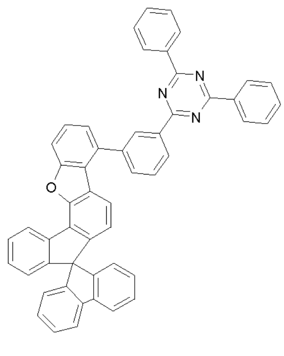

30

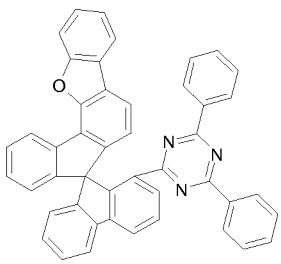

31

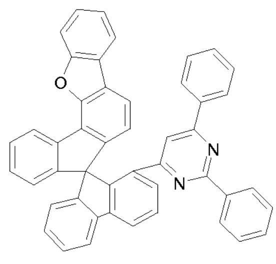

32

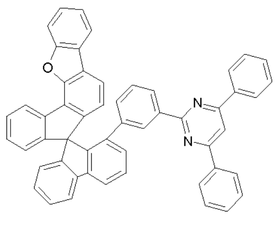

33

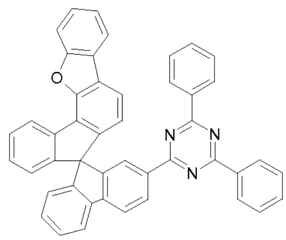

34

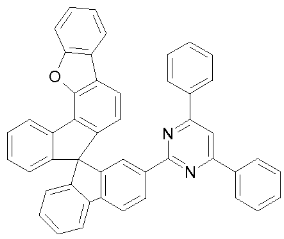

35

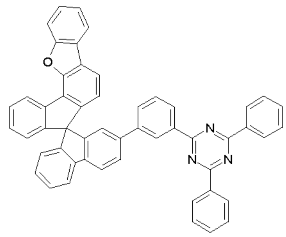

36

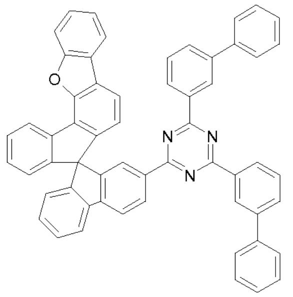

37

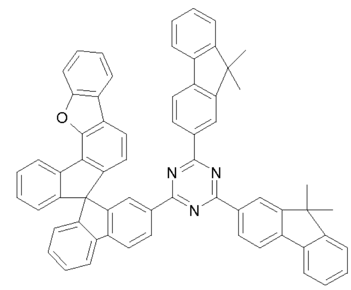

38

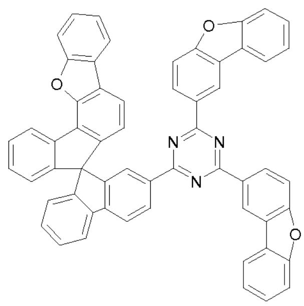

39

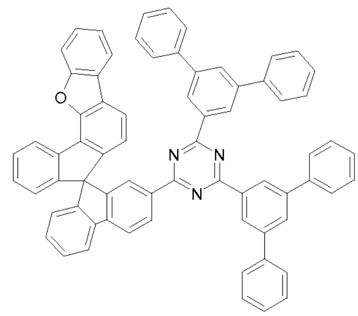

40

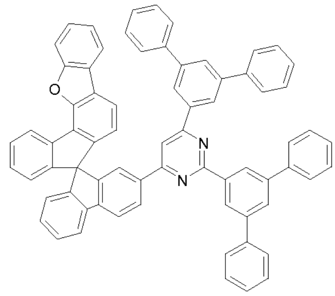

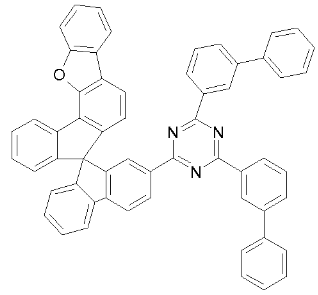

41

42

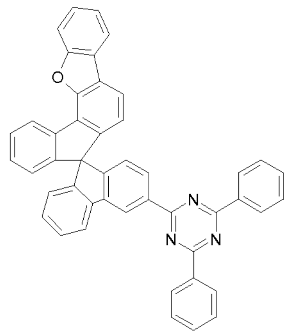

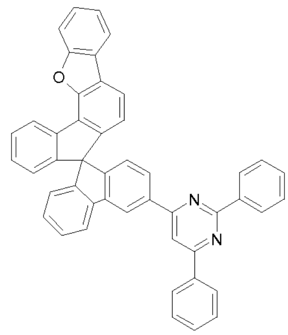

43

44

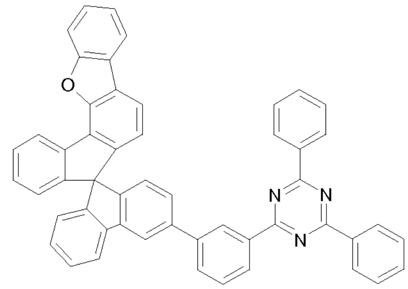

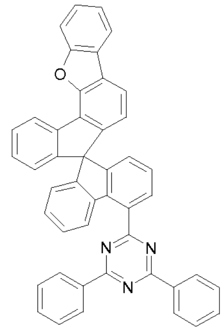

45

46

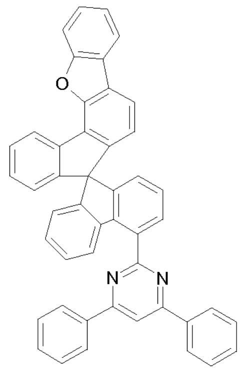

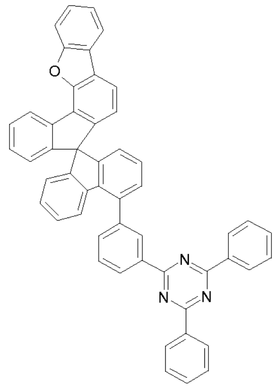

47

48

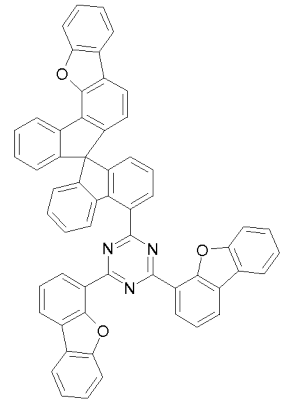

49

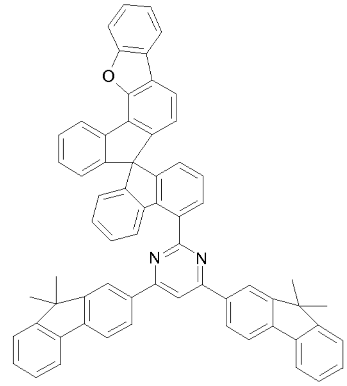

50

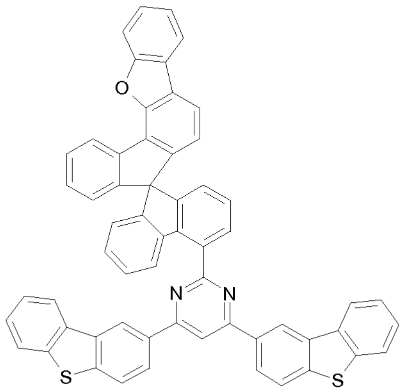

51

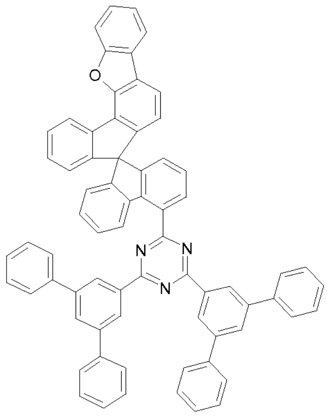

52

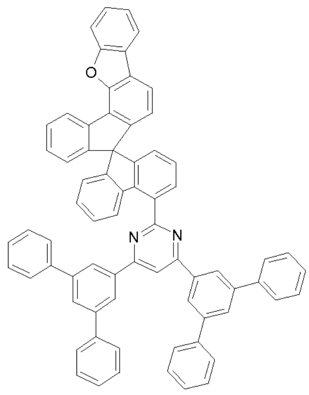

53

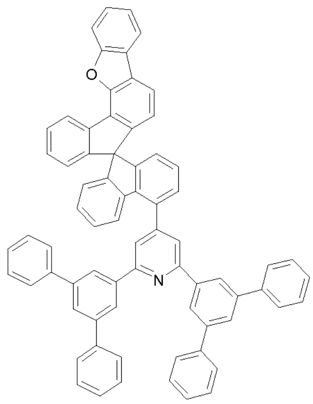

54

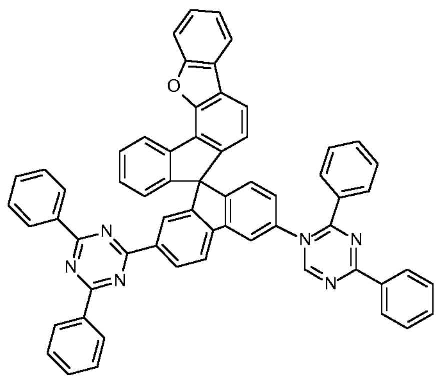

55

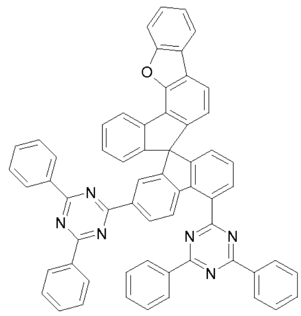

56

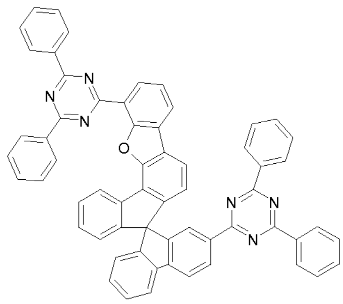

57

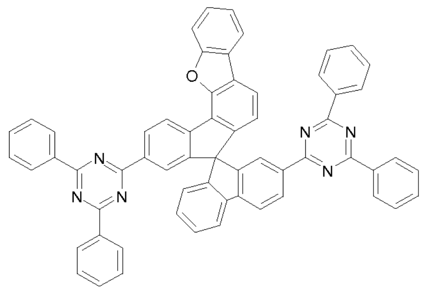

58

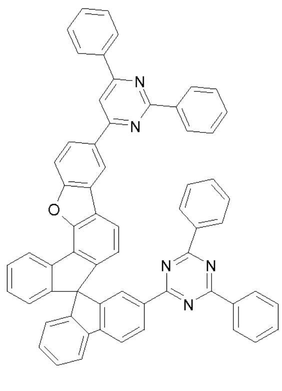

59

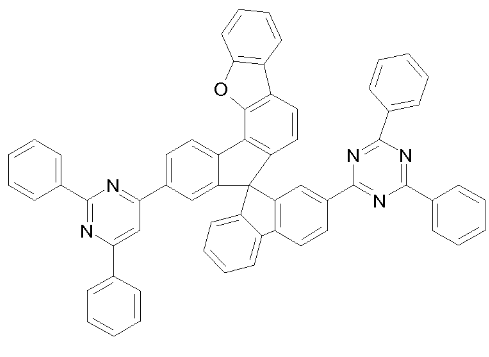

60

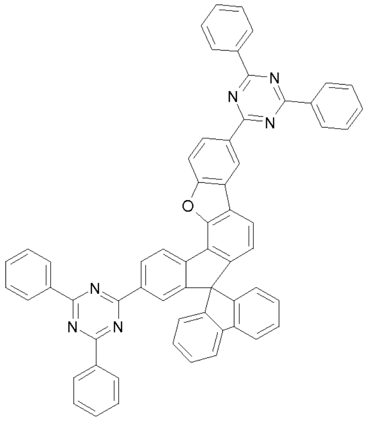

61

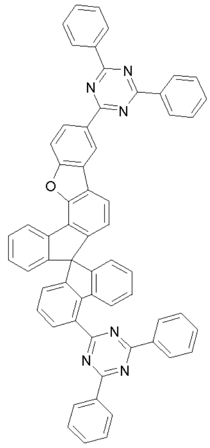

62

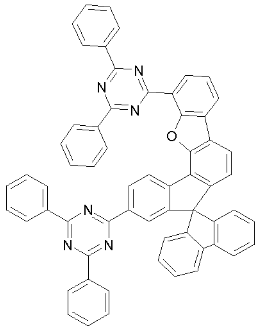

63

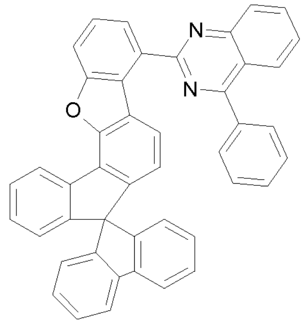

64

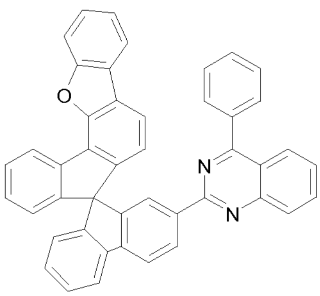

65

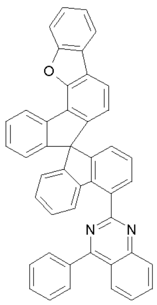

66

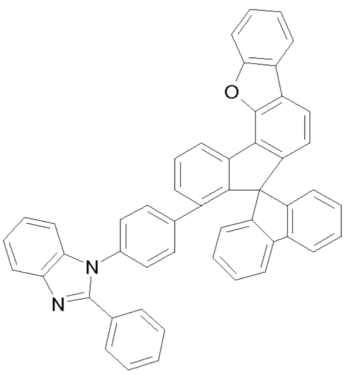

67

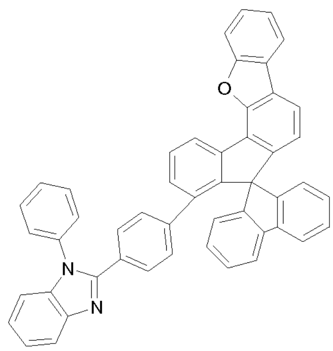

68

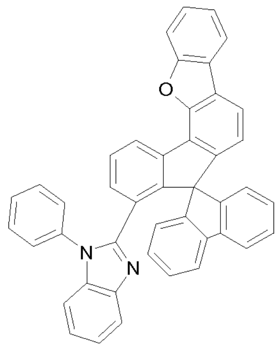

69

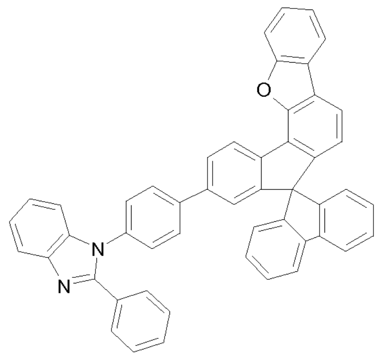

70

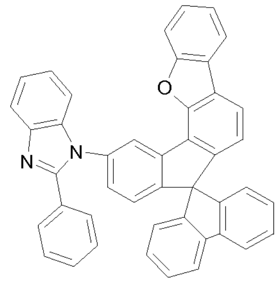

71

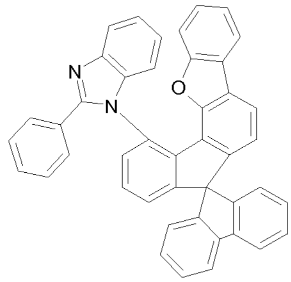

72

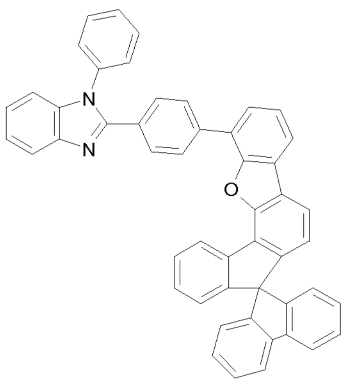

73

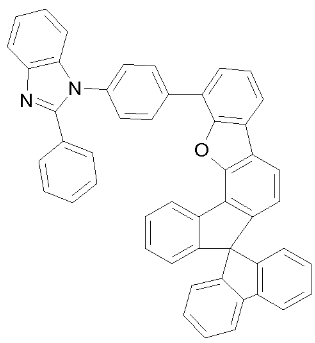

74

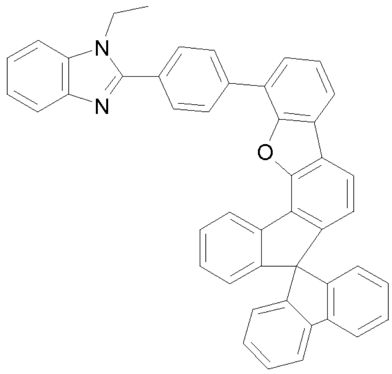

75

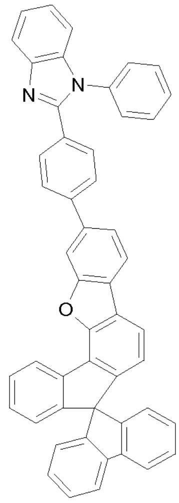

76

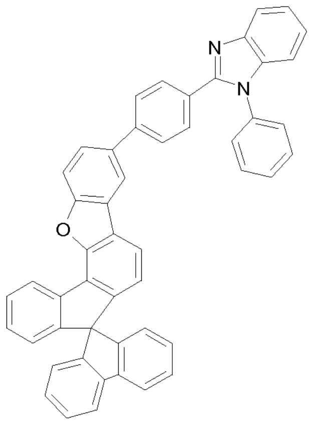

77

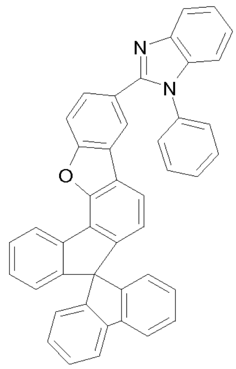

78

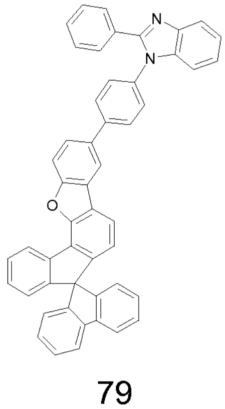

79

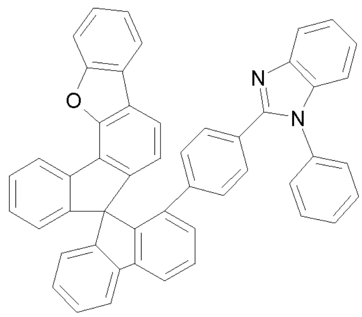

80

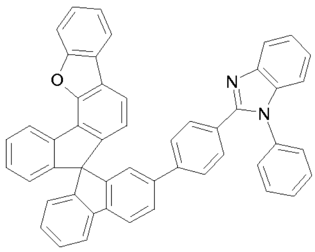

81

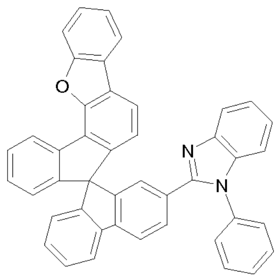

82

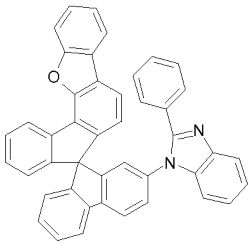

83

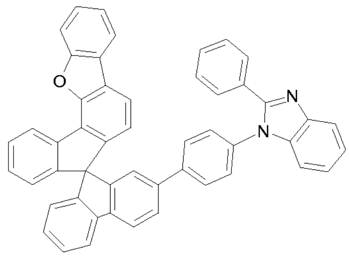

84

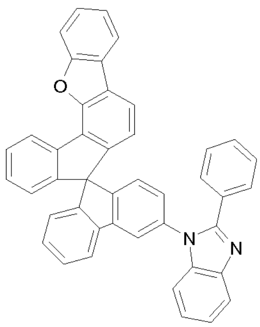

85

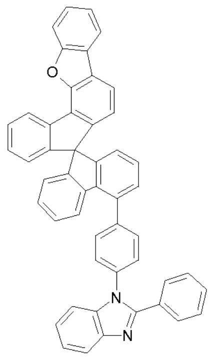

86

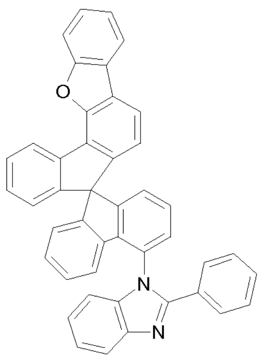

87

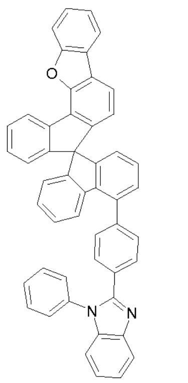

88

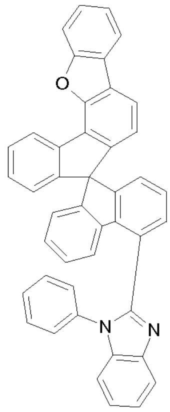

89

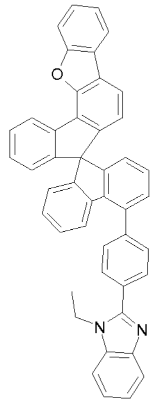

90

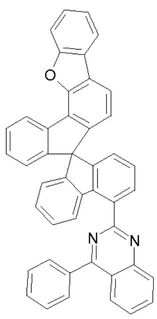

91

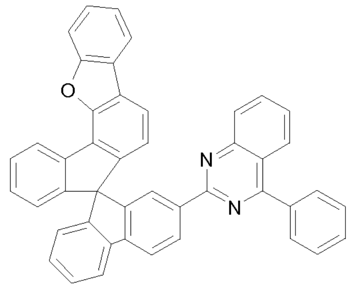

92

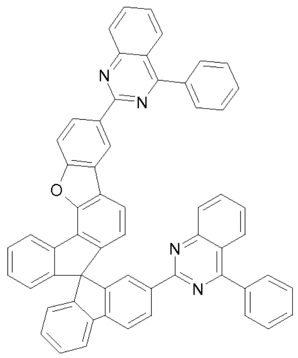

93

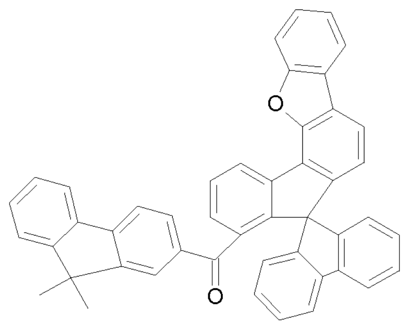

94

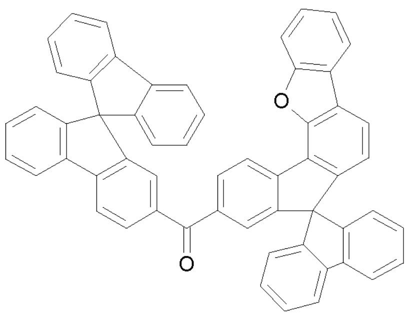

95

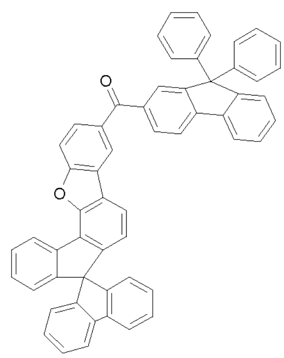

96

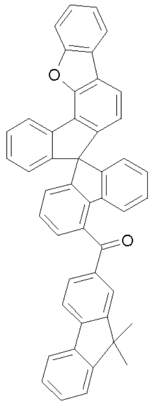

97

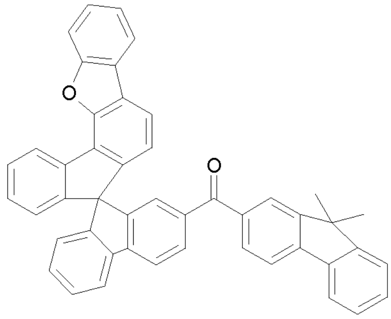

98

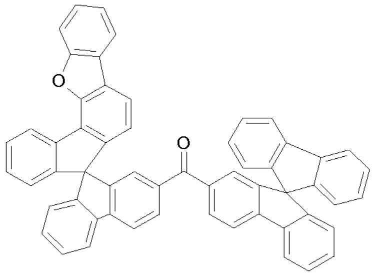

99

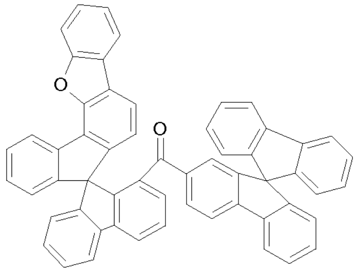

100

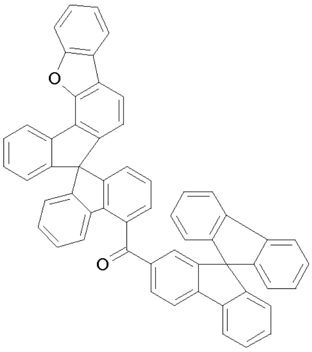

101

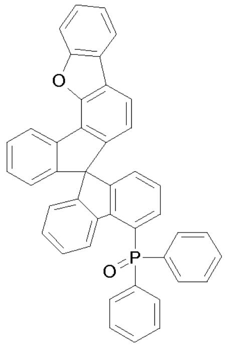

102

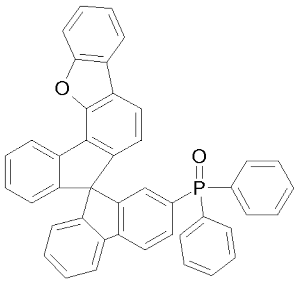

103

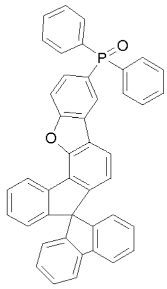

104

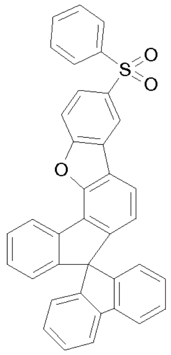

105

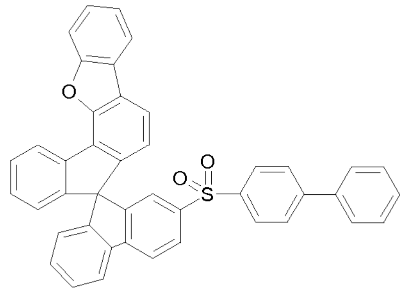

106

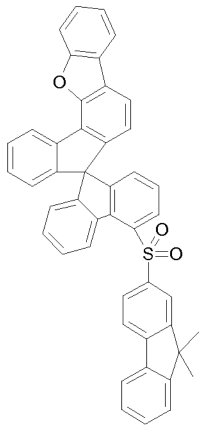

107

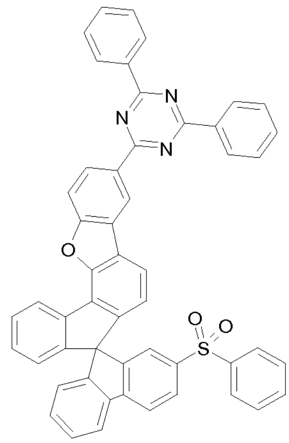

108

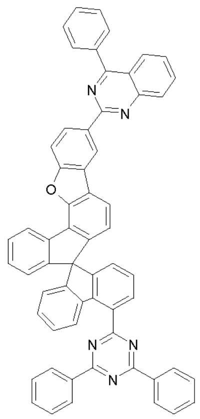

109

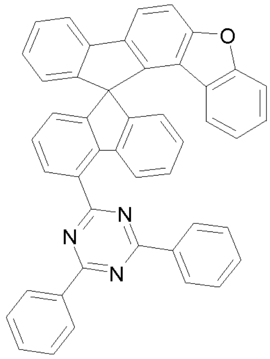

110

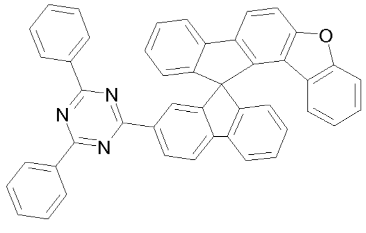

111

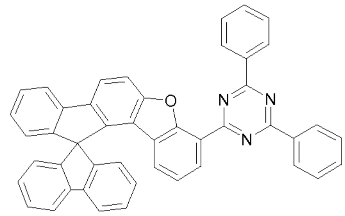

112

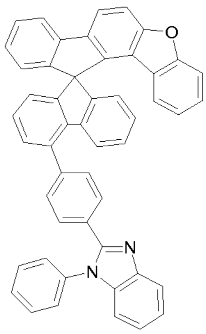

113

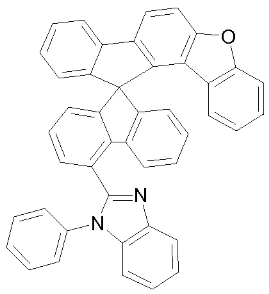

114

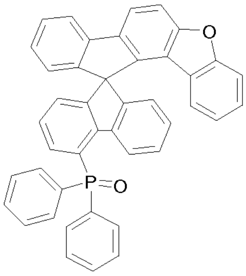

115

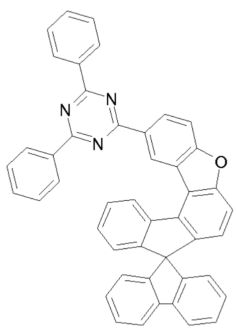

116

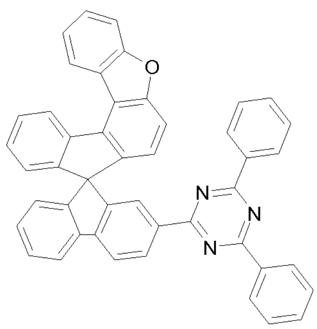

117

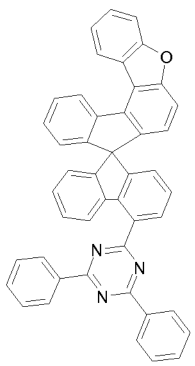

118

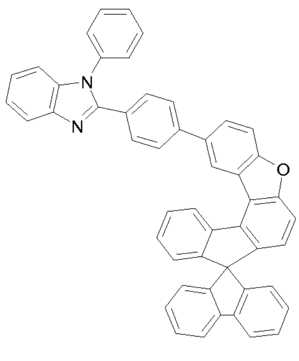

119

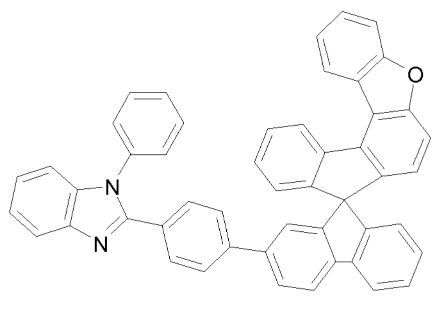

120

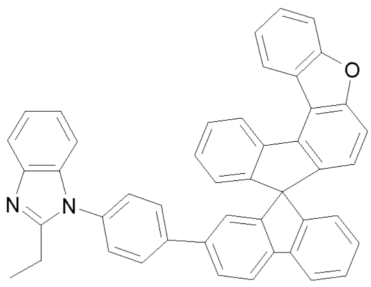

121

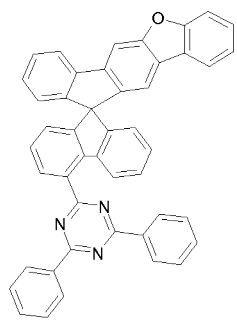

122

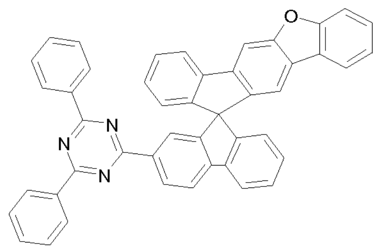

123

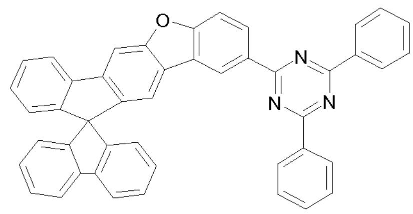

124

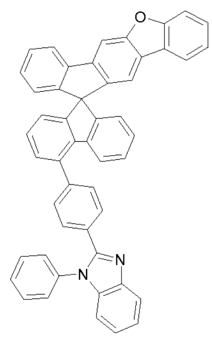

125

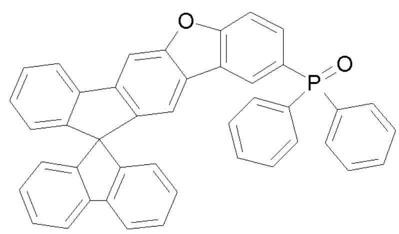

126

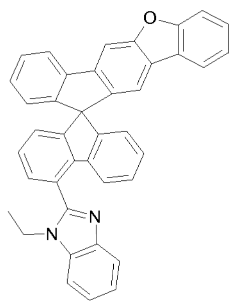

127

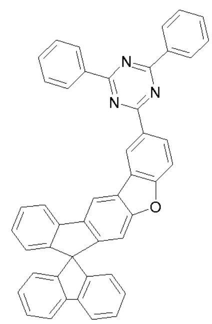

128

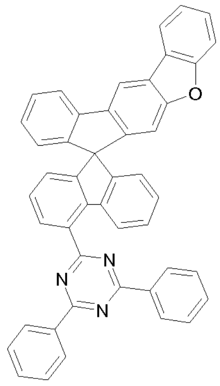

129

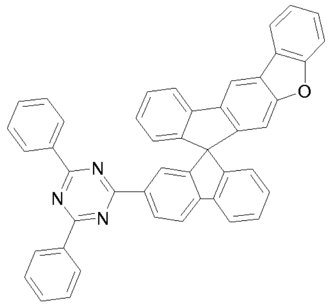

130

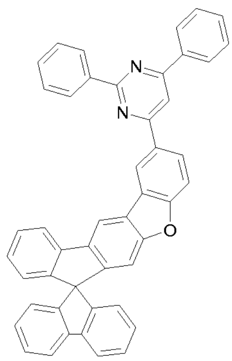

131

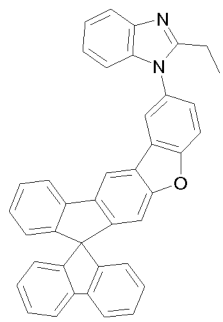

132

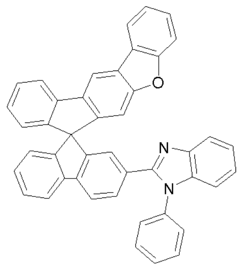

133

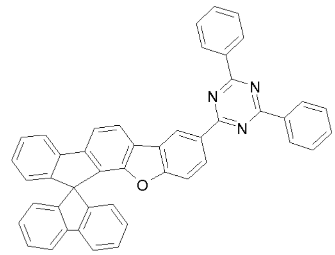

134

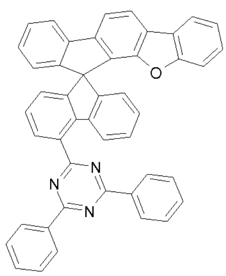

135

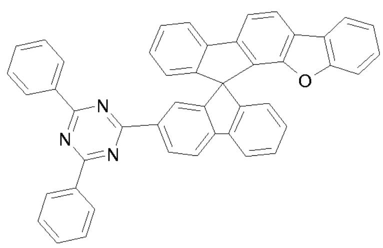

136

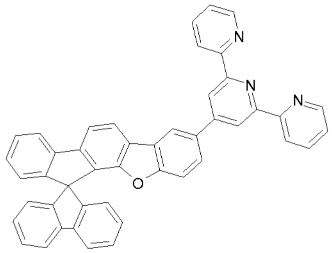

137

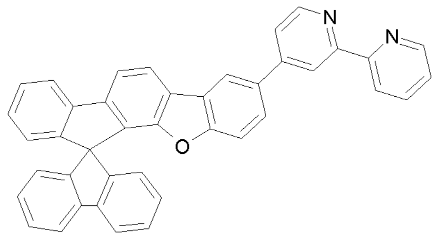

138

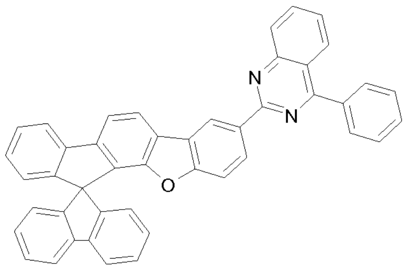

139

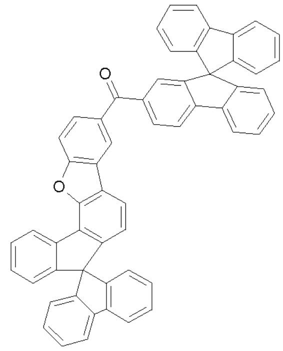

140

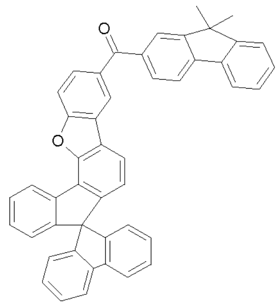

141

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I), bei dem zunächst das Spirobifluoren-Grundgerüst hergestellt wird, und in einem späteren Schritt über eine Kupplungsreaktion ein Rest mit einer elektronentransportierenden Gruppe eingeführt wird.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA.

In allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Die sechs Isomere des Spirogrundgerüsts A bis F, die nachfolgend für die bevorzugten Spiroverbindungen mit einer Dibenzofurangruppe gezeigt sind, können gemäß den Schema 1-8 erhalten werden.

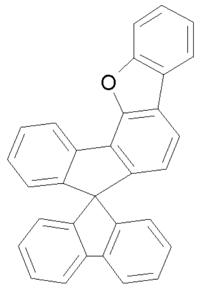

A

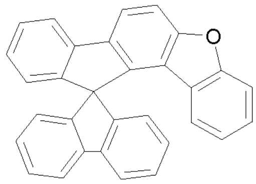

B

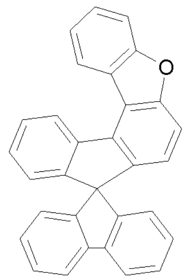

C

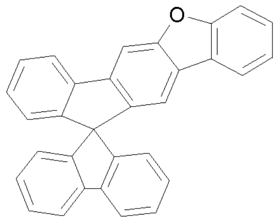

D

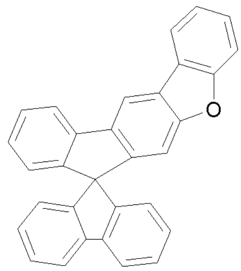

E

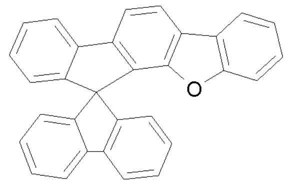

F

Die entsprechenden halogenierten Spirobenzofluoren-Derivate von Typ A, vorzugsweise die Spirobenzofluorenfuran oder -thiophen- Derivate, können ausgehend von Dibenzothiophen- oder Dibenzofuranboronsäure-Derivaten durch Suzuki-Kupplung und anschließende Lithiierung und Umsetzung mit Fluorenon-Derivaten hergestellt werden, wie dies in Schema 1 beispielhaft dargestellt ist.

## Schema 1

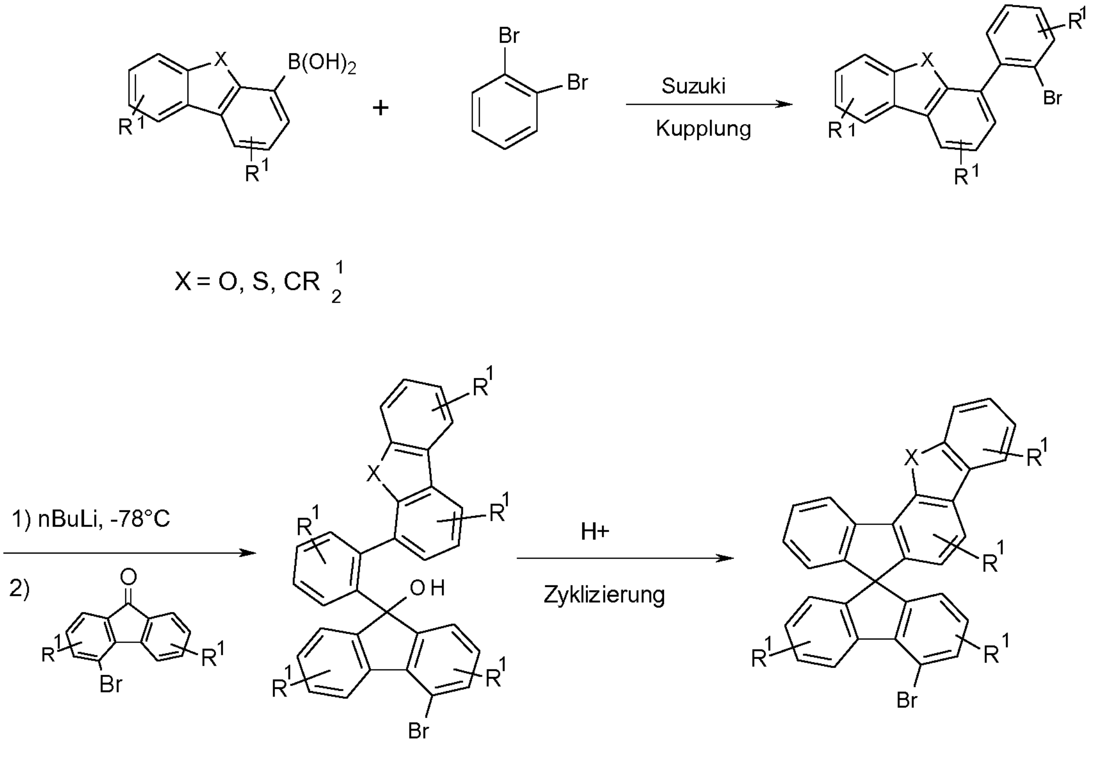


Die halogenierte Spirobenzofluorenfuran oder -Thiophen von Typ B können ausgehend von halogenierte Mercaptoflurenon-Derivat oder Hydroxyfluorenon-Derivate durch Suzuki-Kupplung und anschließende Cyclisierung und weitere Umsetzung mit monolithiierten 2,2'-Dibrombiphenyl-Derivaten und weitere Cyclisierung hergestellt werden (Schema 2).

## Schema 2

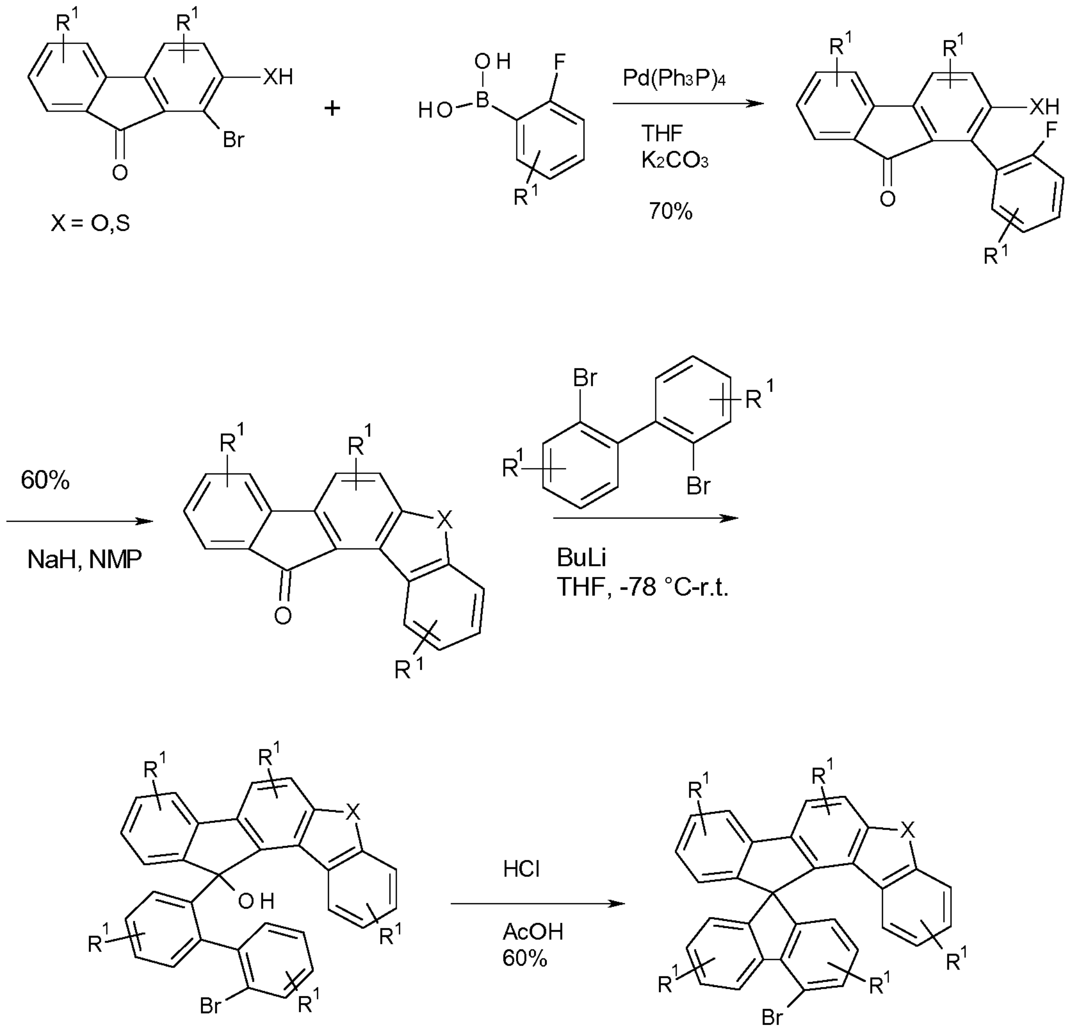


Weiter können die entsprechenden halogenierten Spirobenzofluorenfuran oder -thiophen- Derivate von Typ C ausgehend von den entsprechenden Boronsäure-Derivaten durch Suzuki-Kupplung und anschließender Lithiierung und weiterer Umsetzung mit einem 4-Bromfluorenon-Derivat und anschließender Cyclisierung hergestellt werden (Schema 3).

## Schema 3

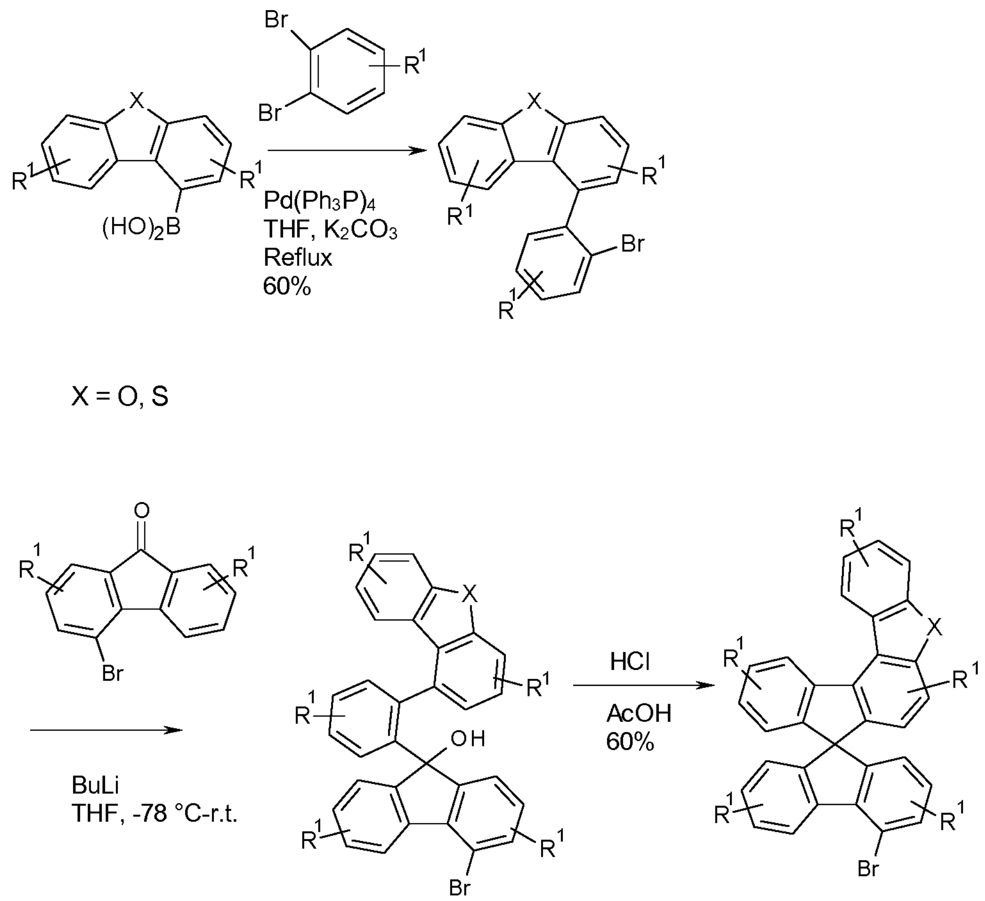


Die entsprechenden halogenierten Spirobenzofluoren-Derivate von Typ **D,** vorzugsweise die Spirobenzofluorenfuran oder -thiophen- Derivate, können ausgehend vom Benzofluorenofuranon-Derivat oder Benzofluorenothiphenon Derivat durch Umsetzung mit monolithiierten 2,2'-Dibrombiphenyl-Derivaten und anschließender Cyclisierung hergestellt werden (Schema 4).

## Schema 4

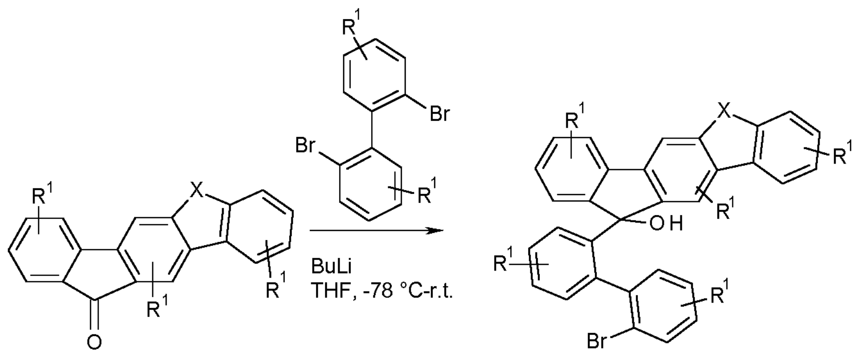


X = S,O, $C(R^1)_2$

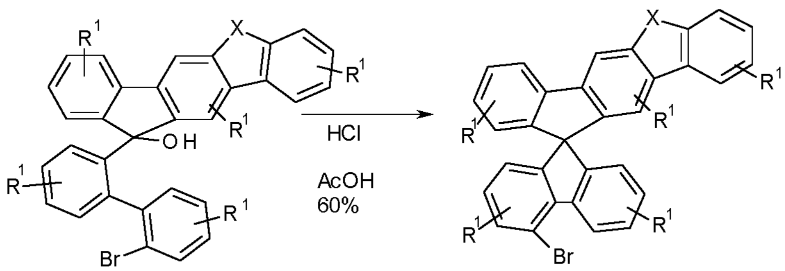


Die entsprechenden halogenierten Spirobenzofluorenfuran oder -thiophen-Derivate von Typ **E** können ausgehend vom halogenierten Mercaptoflurenon-Derivat oder Hydroxyfluorenon-Derivaten durch Suzuki-Kupplung mit anschließender Cyclisierung und weiterer Umsetzung mit monolithiierten 2,2'-Dibrombiphenyl-Derivaten und weitere Cyclisierung hergestellt werden (Schema 5).

**Schema 5**

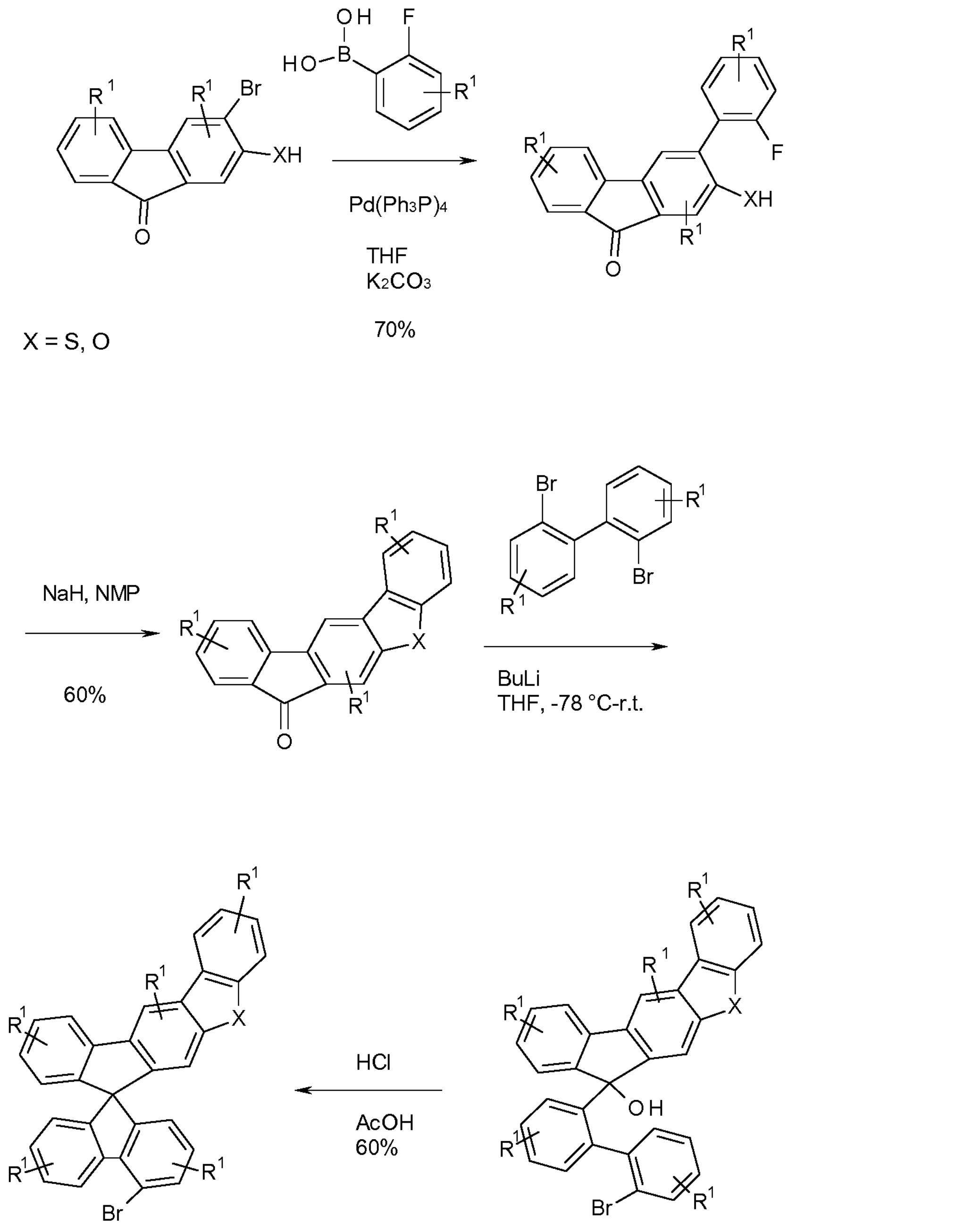


Die entsprechenden halogenierten Spirobenzofluorenfuran oder -thiophen-Derivate von Typ F können ausgehend vom Benzofluorenobfuran- oder - thiophen Derivat oder Benzofluorenothiphen durch Umsetzung mit monolithiierten 2,2'-Dibrombiphenyl-Derivaten und weiterer Cyclisierung hergestellt werden (Schema 6).

## Schema 6

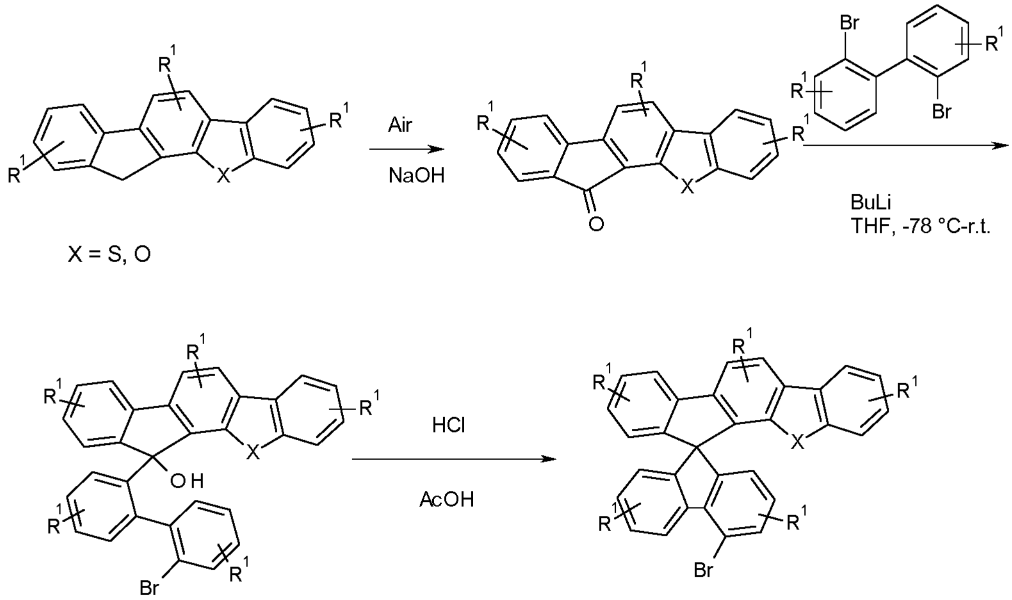


Eine weitere Möglichkeit Spirobenzofluoren-Derivate vom Typ **F** oder **D,** vorzugsweise Spirobenzofluorenfuran oder -thiophen- Derivate, darzustellen ist im Schema 7 beschrieben, wobei sich der Syntheseablauf ähnlichen Schritten wie in Schema 6 gezeigt bedient. Die Isolierung der Isomere erfolgt chromatographisch.

## Schema 7

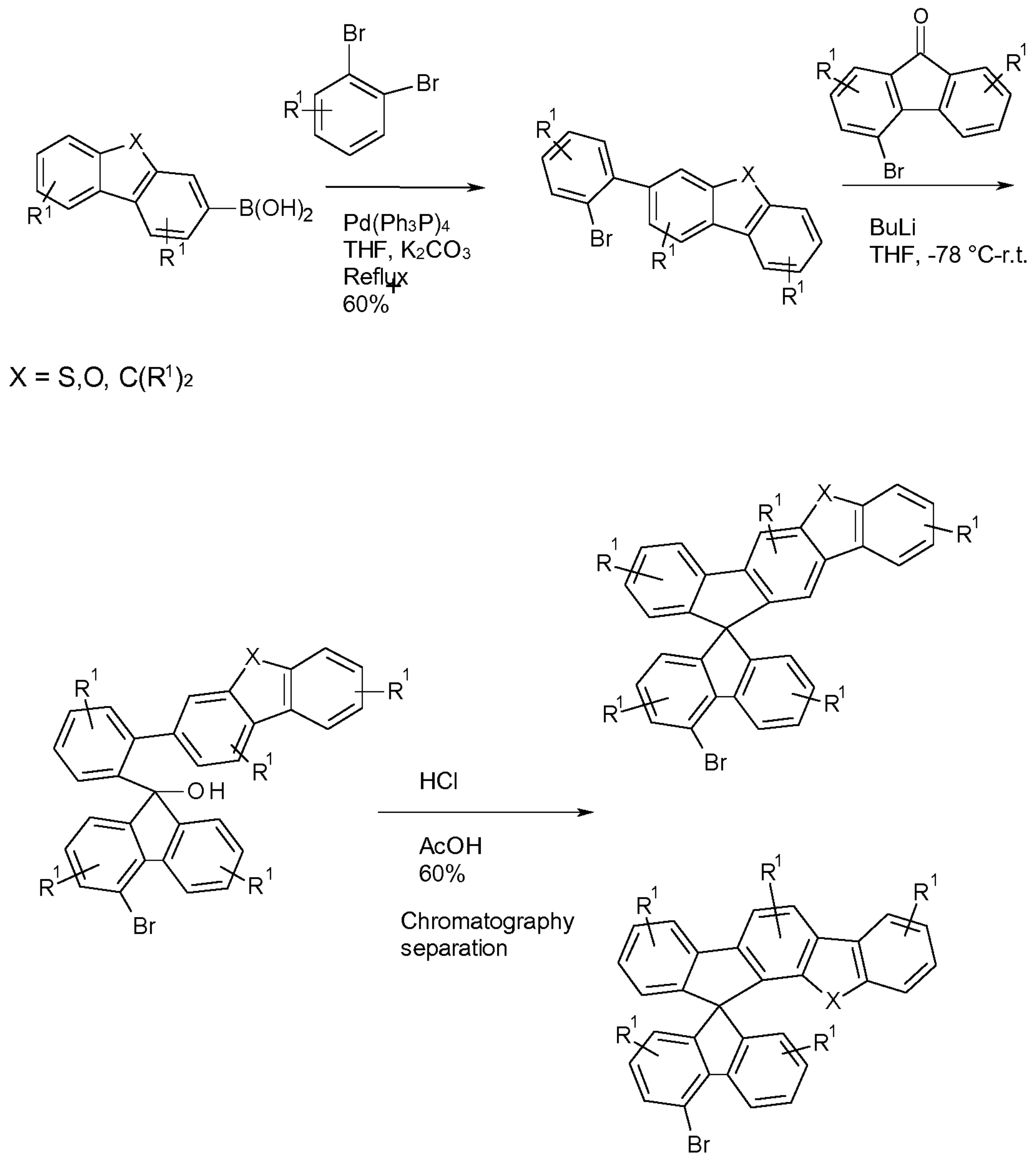


Eine weitere Möglichkeit Spirofluoren- Derivate, vorzugsweise Dibenzofuran- oder -thiophen- Spirofluorenderivate vom Typ **B** oder **E** darzustellen ist im Schema 8 beschrieben, wobei sich der Syntheseablauf ähnlichen Schritten wie in Schema 1 gezeigt bedient. Die Isolierung der Isomere erfolgt chromatographisch.

## Schema 8

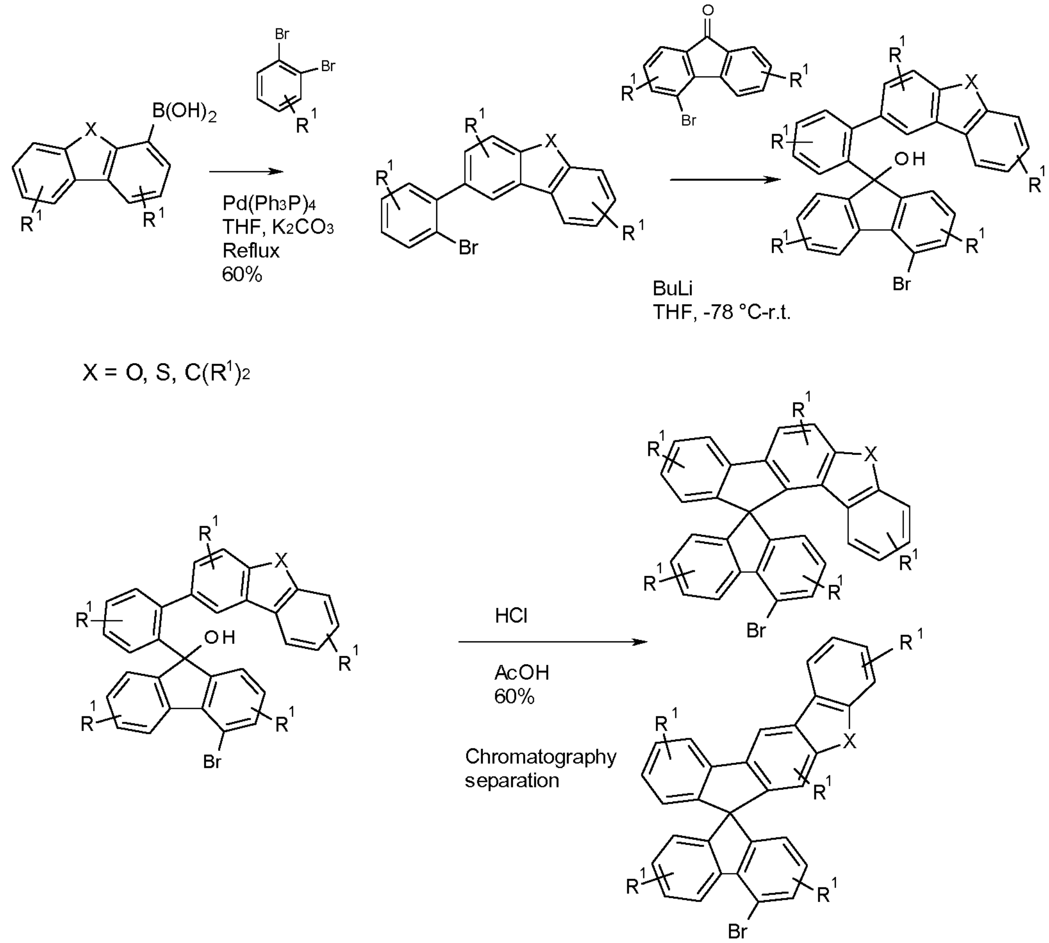


Für die Synthese von halogenfreien Spirofluoren-Derivaten, vorzugsweise Spirofluorenbenzofuranen oder -thiophenen wird von halogenfreien Zwischenstufen ausgegangen. Durch weitere Litiierung oder Bromierung können Dibenzofuran- bzw. Dibenzothiopheneinheiten funktionalisiert werden (Schema 9).

## Schema 9

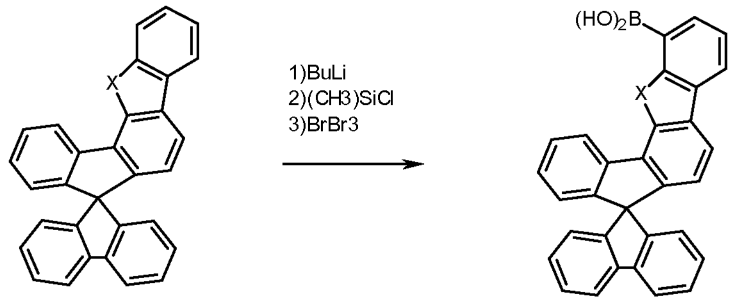


X = S, O, $C(R^1)_2$

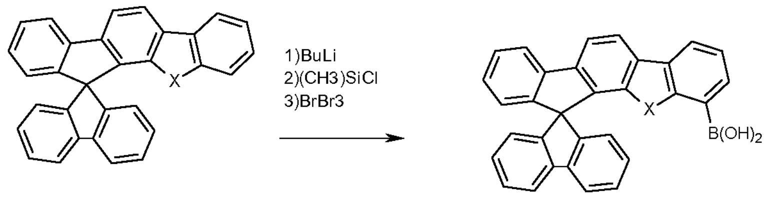


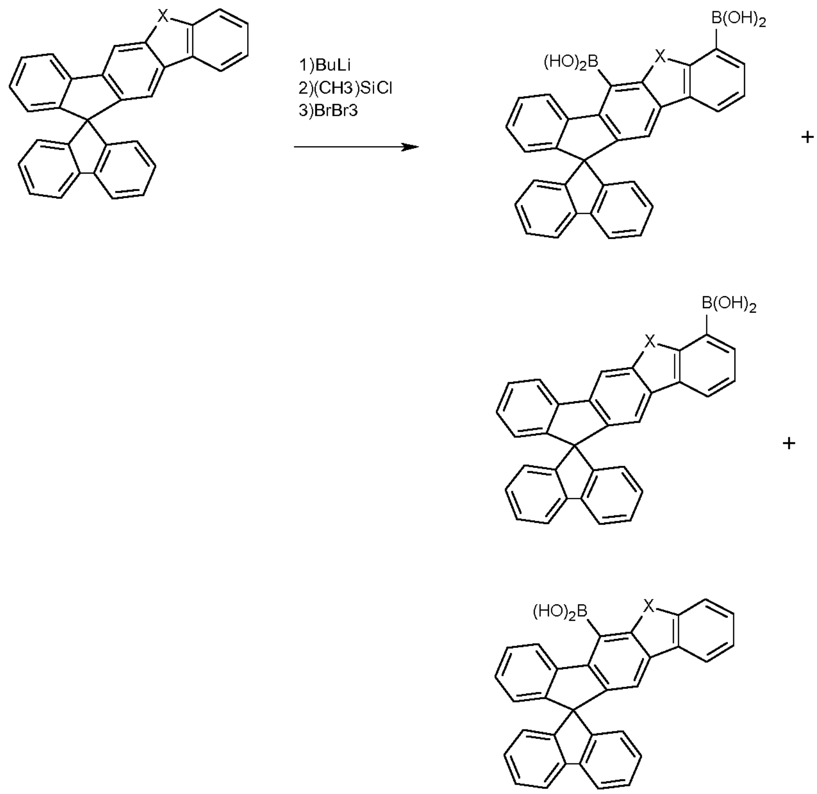

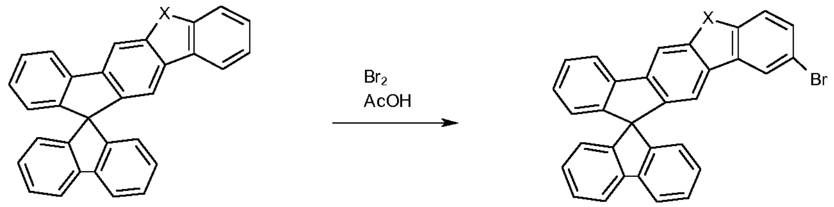


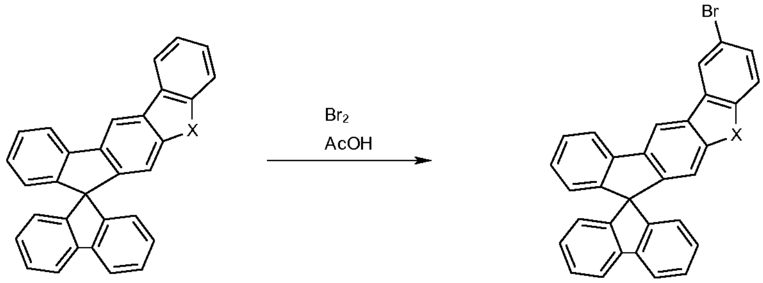


Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen können als vernetzte oder unvernetzte Schicht eingesetzt werden.

Weiterer Gegenstand der Offenbarung sind daher Oligomere, Polymere oder Dendrimere enthaltend eine oder mehrere der oben aufgeführten Strukturen der Formel (I) oder Verbindungen, wobei ein oder mehrere Bindungen der Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder

auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Ferner können die vorliegenden Verbindungen ein relativ geringes Molekulargewicht aufweisen. Ein weiterer Gegenstand der vorliegenden Erfindung ist demgemäß eine Verbindung, die ein Molekulargewicht von vorzugsweise höchstens 10000 g/mol, besonders bevorzugt höchstens 5000 g/mol und speziell bevorzugt höchstens 3000 g/mol aufweist.

Weiterhin zeichnen sich bevorzugte Verbindungen dadurch aus, dass diese sublimierbar sind. Diese Verbindungen weisen im Allgemeinen eine Molmasse von weniger als ca. 1200 g/mol auf.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005.

Nochmals ein weiterer Gegenstand der vorliegenden Erfindung ist eine Formulierung, enthaltend eine erfindungsgemäße Verbindung und mindestens ein Lösungsmittel.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Nochmals ein weiterer Gegenstand der vorliegenden Erfindung ist eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, n-Dotanden, Host-Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

Daher ist ein weiterer Gegenstand der vorliegenden Erfindung insbesondere eine Zusammensetzung, die dadurch gekennzeichnet ist, das organisch funktionelle Material ein Elektronentransportmaterial ist.

Zu den bevorzugten Elektronentransportmaterialien zählen unter anderem elektronenarme heteroaromatische Verbindungen. Zu den elektronenarme heteroaromatische Verbindungen gehören unter anderem Pyridine, Pyrazine, Pyrimidine, Pyridazine, 1,2,4-Triazine, 1,3,5-Triazine, Chinoline, Isochinoline, Chinoxaline, Pyrazole, Imidazole, Benzimidazole, Thiazole, Benzothiazole, Oxazole oder Benzooxazole.

Ferner kann erfindungsgemäß vorgesehen sein, dass die Zusammensetzung neben Verbindungen, enthaltend Strukturen gemäß Formel (I), als organisch funktionelles Material vorzugsweise mindestens einen n-Dotanden umfasst.

Unter n-Dotanden werden hierin Reduktionsmittel, d.h. Elektronendonatoren verstanden. Bevorzugte Beispiele für n-Dotanden sind $W(hpp)_4$ und weitere elektronenreiche Metallkomplexe gemäß WO 2005/086251 A2, P=N-Verbindungen (z.B. WO 2012/175535 A1, WO 2012/175219 A1), Naphthylencarbodiimide (z.B. WO 2012/168358 A1), Fluorene (z.B. WO 2012/031735 A1), Radikale und Diradikale (z.B. EP 1837926 A1, WO 2007/107306 A1), Pyridine (z.B. EP 2452946 A1, EP 2463927 A1), N-heterocyclische Verbindungen (z.B. WO 2009/000237 A1) und Acridine sowie Phenazine (z.B. US 2007/145355 A1).

Die vorliegenden Erfindung betrifft daher auch eine Zusammensetzung enthaltend wenigstens eine Verbindung umfassend Strukuren gemäß Formel (I) sowie wenigstens ein weiteres Matrix- oder Hostmaterial. Gemäß einem besonderen Aspekt der vorliegenden Erfindung weist das weitere Matrix- oder Hostmaterial lochtransportierende Eigenschaften auf.

Zu den bevorzugten Matrix- oder Hostmaterialien, die lochtransportierende Eigenschaften aufweisen, zählen Arylamine, Triaryl-amine, verbrückte Amine; bevorzugte verbrückte Amine sind dabei Dihydroacridine, Dihydrophenazine, Phenoxazione und Phenothiazine, Carbazole, verbrückten Carbazole, Biscarbazole, Indenocarbazole und Indolocarbazole.

Ein weiterer Gegenstand der vorliegenden stellt eine Zusammensetzung dar, enthaltend wenigstens eine Verbindung, umfassend mindestens eine Struktur gemäß Formel (I) sowie wenigstens ein wide band gap Material, wobei unter wide band gap Material ein Material im Sinne der Offenbarung von US 7,294,849 verstanden wird. Diese Systeme

zeigen besondere vorteilhafte Leistungsdaten in elektrolumineszierenden Vorrichtungen.

Vorzugsweise kann die zusätzliche Verbindung eine Bandlücke (band gap) von 2.5 eV oder mehr, bevorzugt 3.0 eV oder mehr, ganz bevorzugt von 3.5 eV oder mehr aufweisen. Die Bandlücke kann unter anderem durch die Energieniveaus des highest occupied molecular orbital (HOMO) und des lowest unoccupied molecular orbital (LUMO) berechnet werden, wie dies zuvor beispielhaft dargelegt wurde.

Die vorliegende Erfindung betrifft auch eine Zusammensetzung umfassend wenigstens eine Verbindung umfassend Strukuren gemäß Formel (I) sowie wenigstens einen phosphoreszierende Emitter, wobei unter dem Begriff phosphoreszierende Emitter auch phosphoreszierende Dotanden verstanden werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

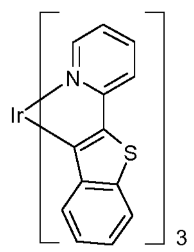

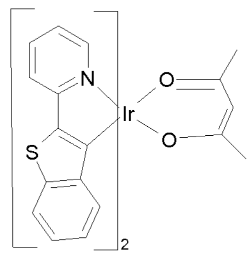

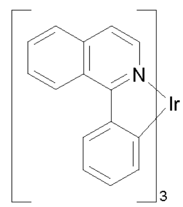

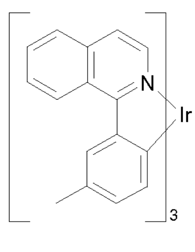

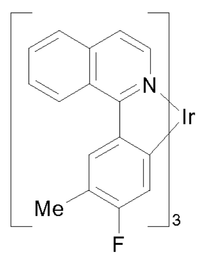

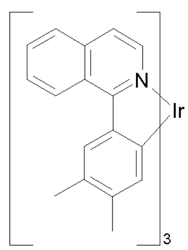

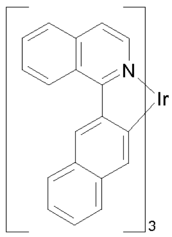

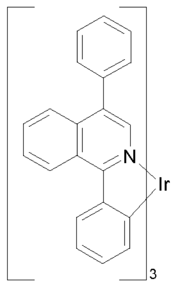

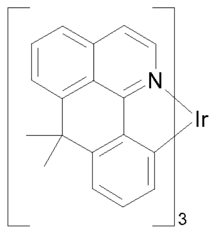

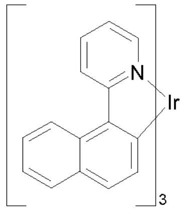

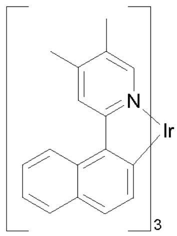

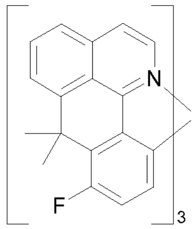

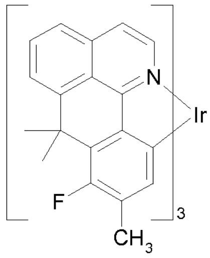

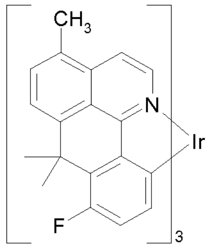

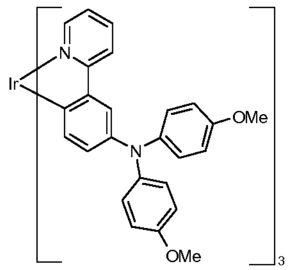

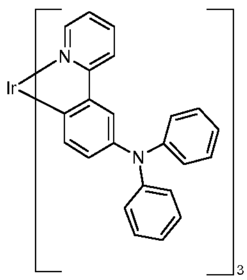

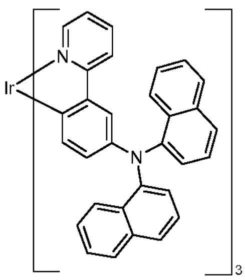

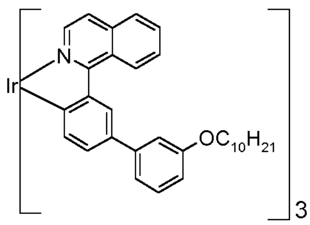

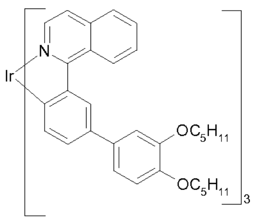

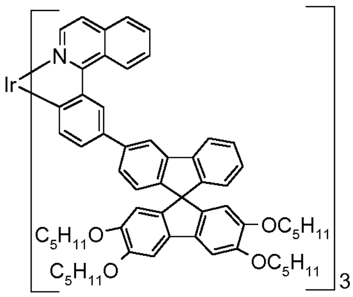

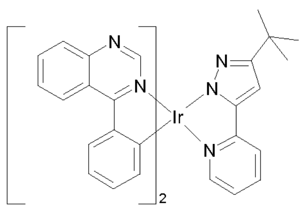

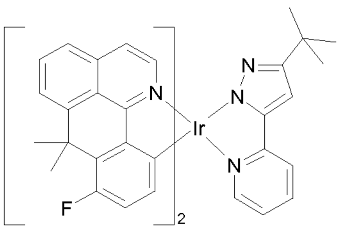
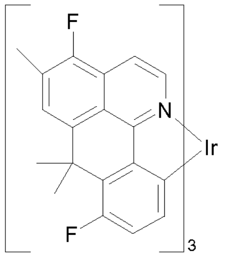
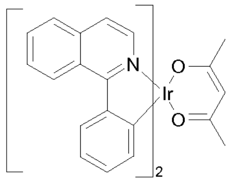

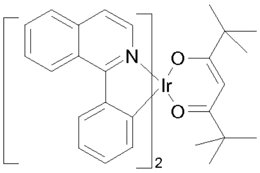
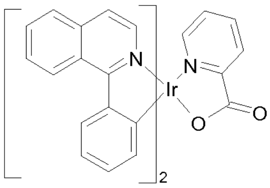
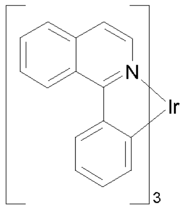

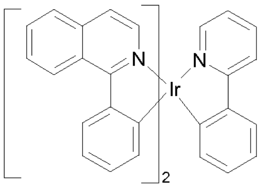
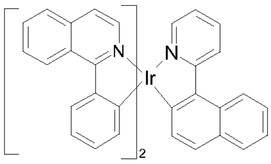
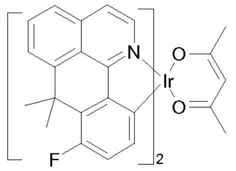

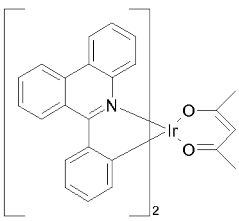
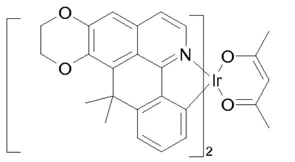
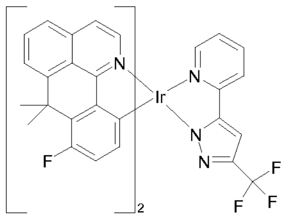

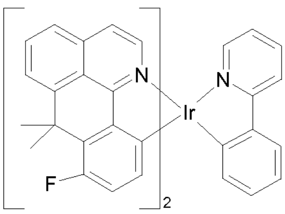
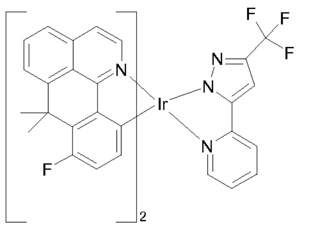
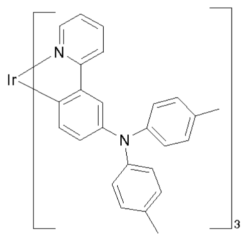

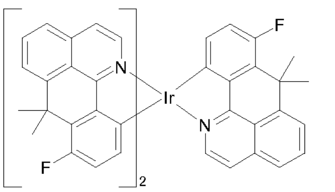
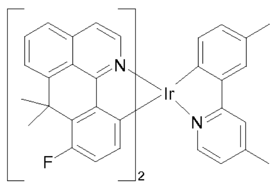
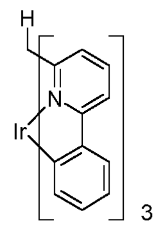

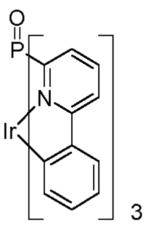

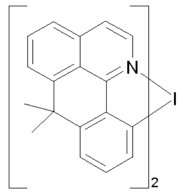

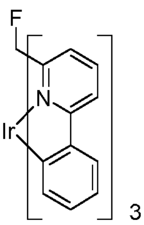

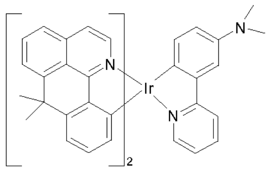

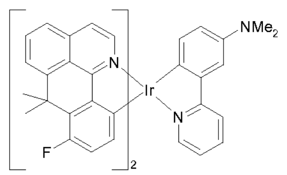

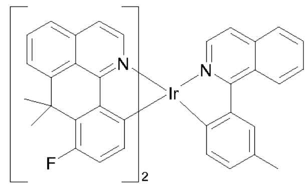

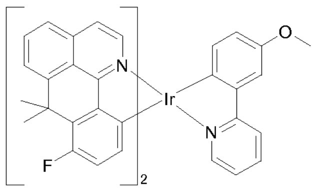

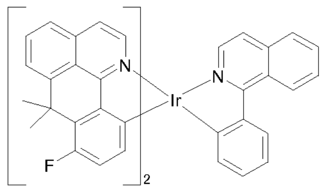

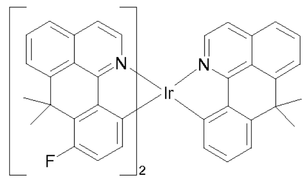

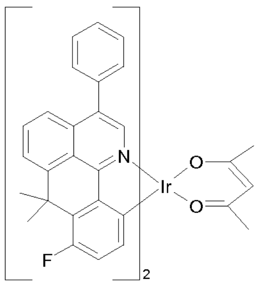

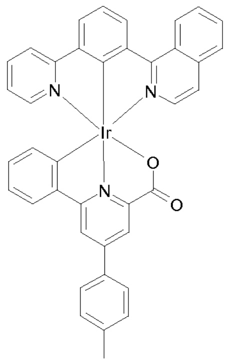

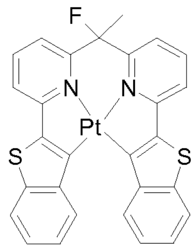

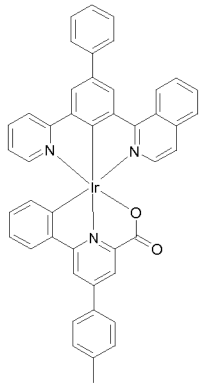

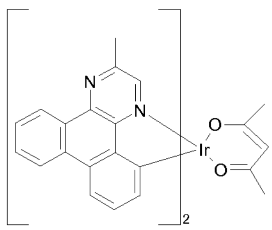

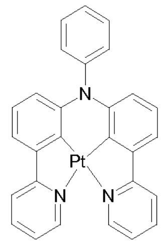

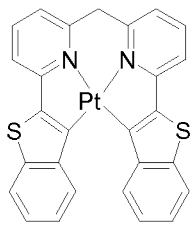
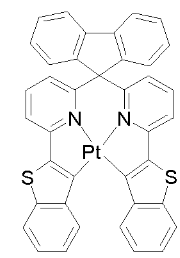
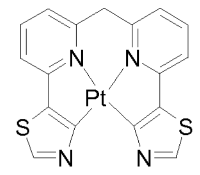

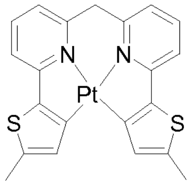
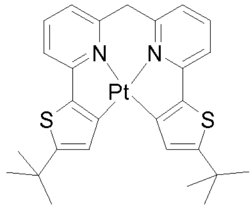
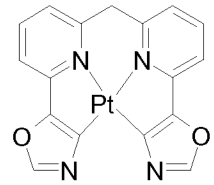

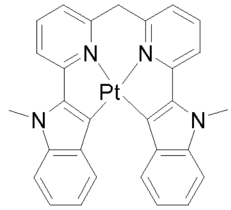
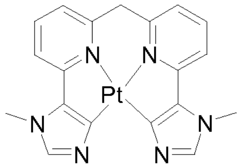
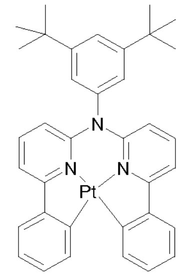

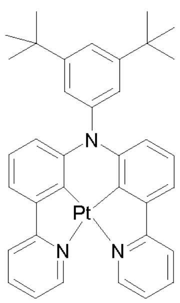
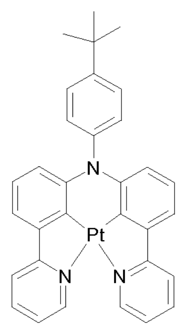
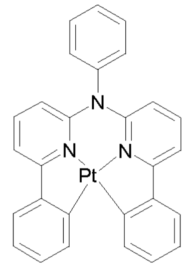

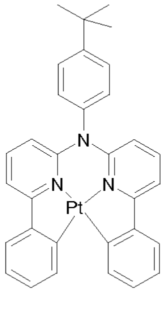
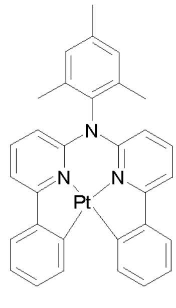
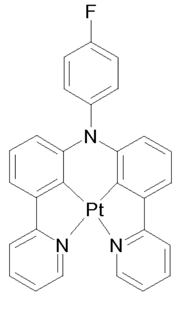

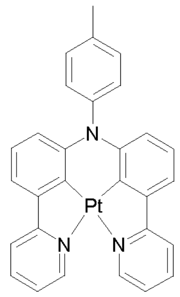
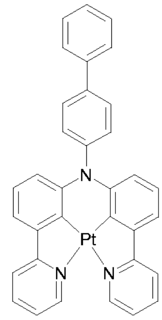
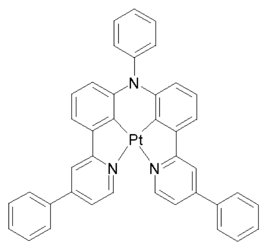

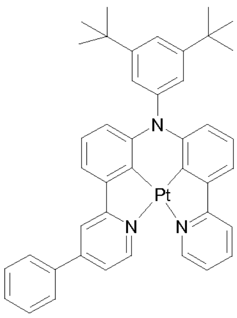
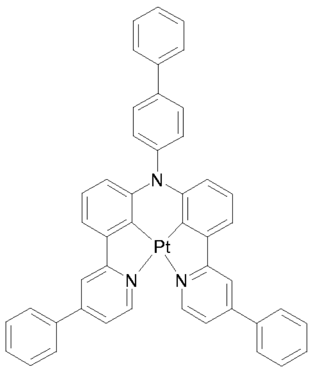
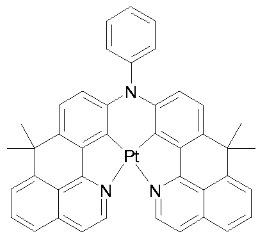

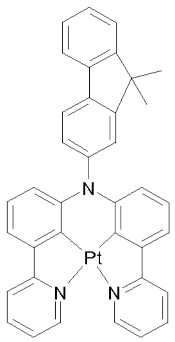
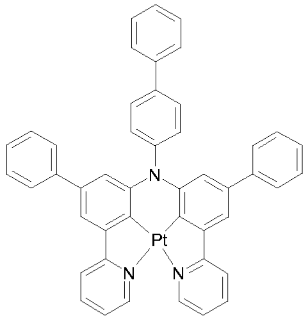
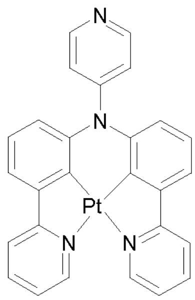

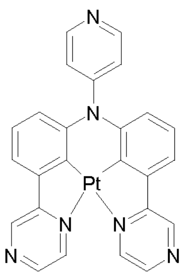
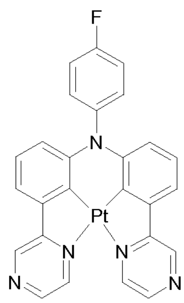
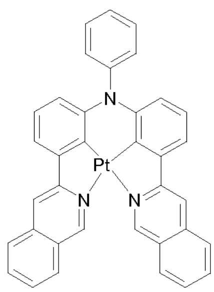

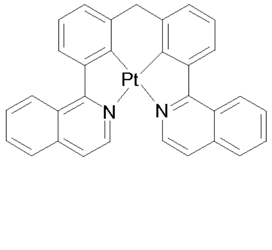
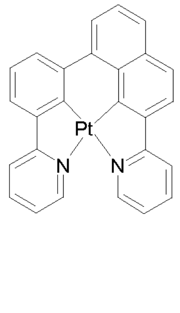
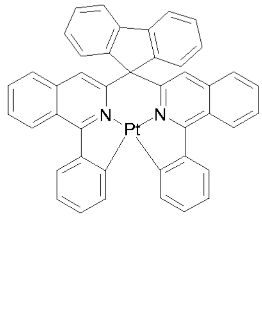

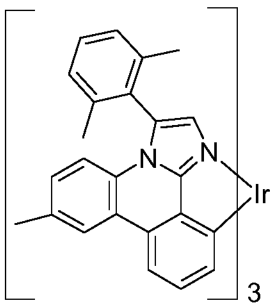
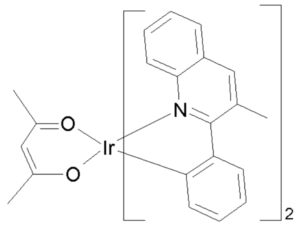
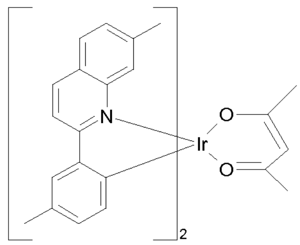

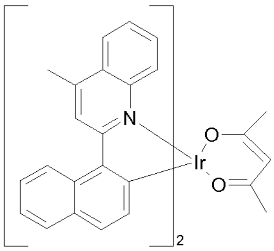
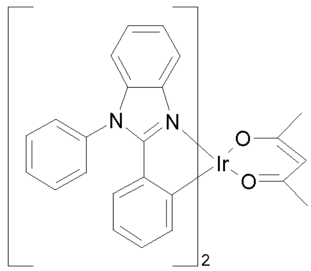
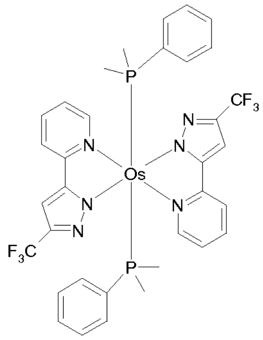

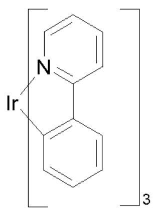
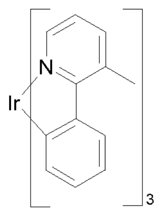
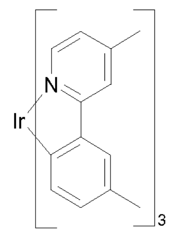

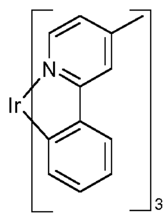
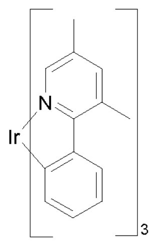
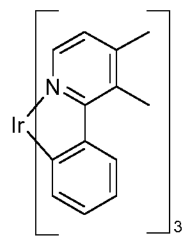

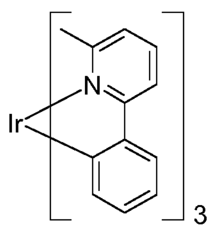
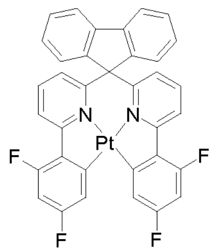
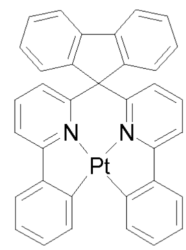

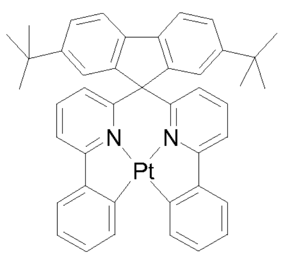 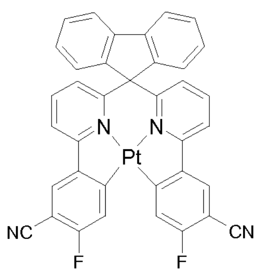 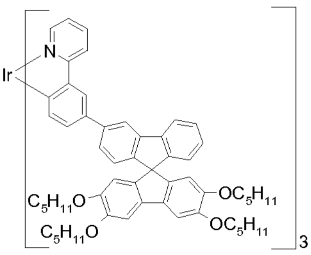

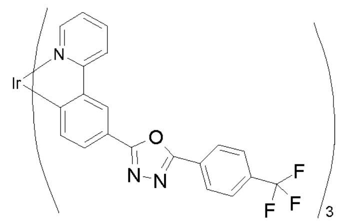 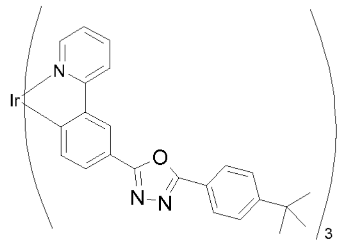 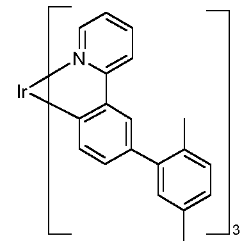

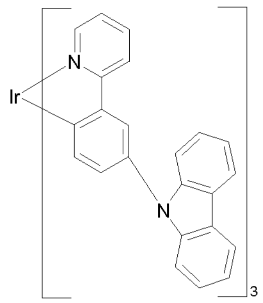 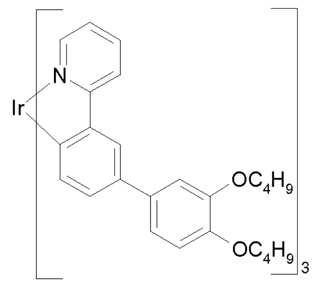 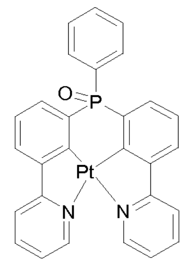

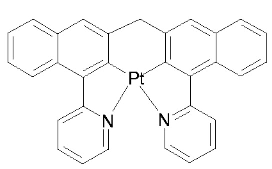 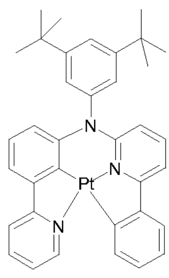 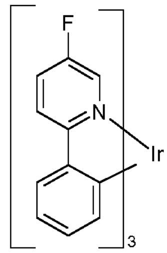

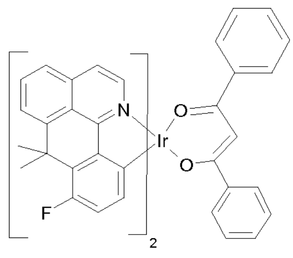 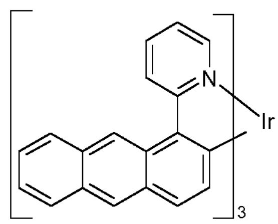 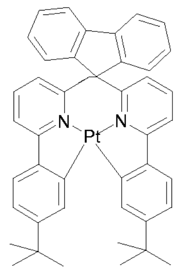

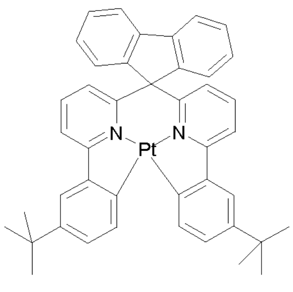
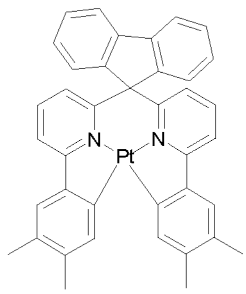
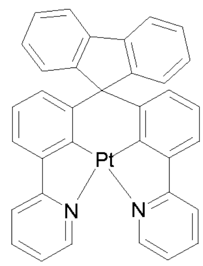
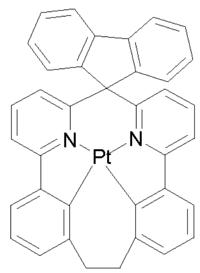
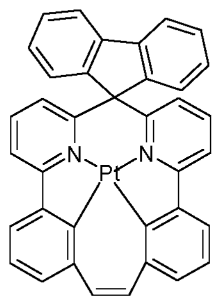
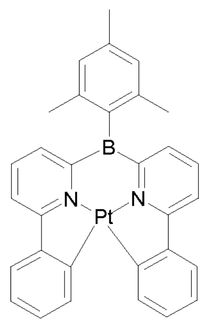
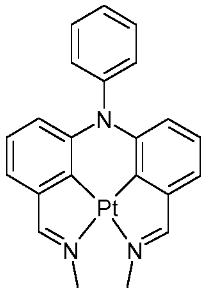
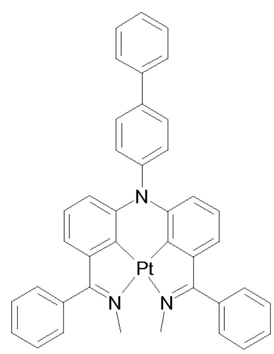
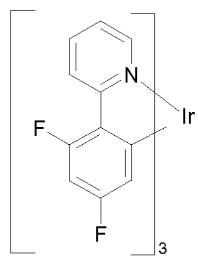
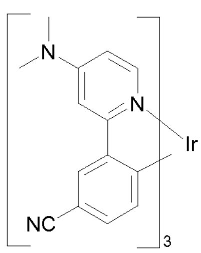
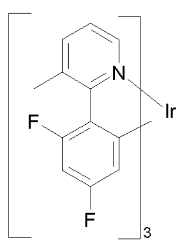
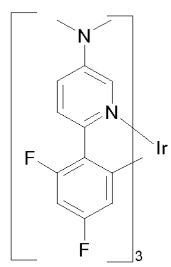
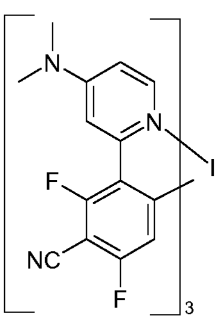
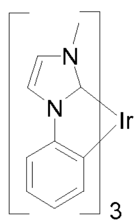
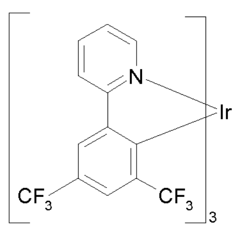

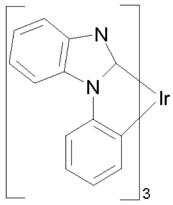
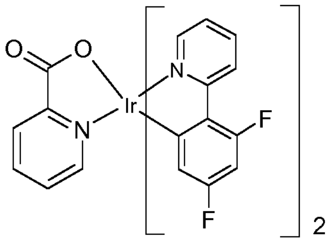
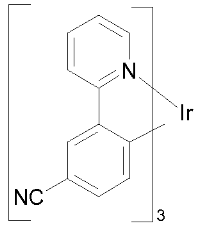

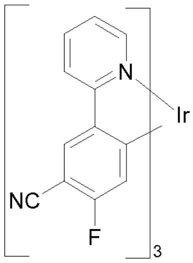
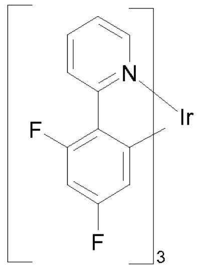
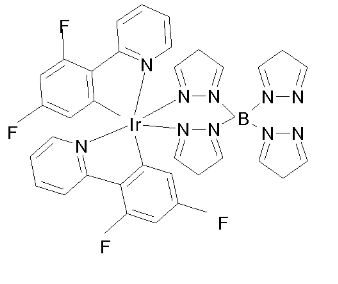

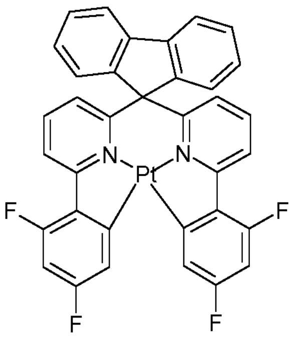
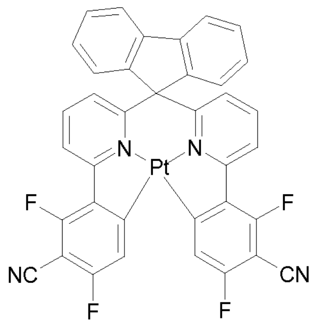
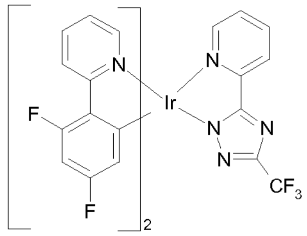

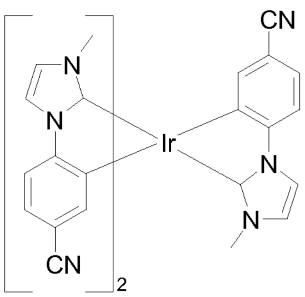
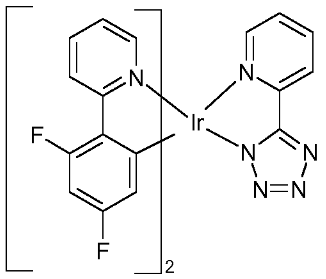
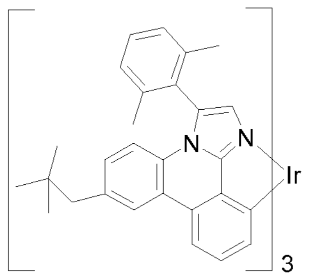

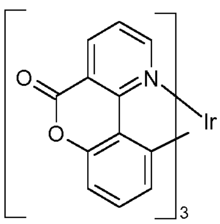
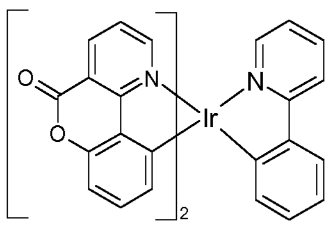
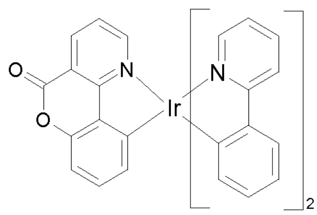

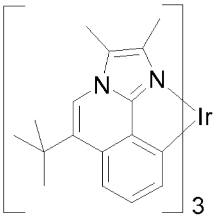

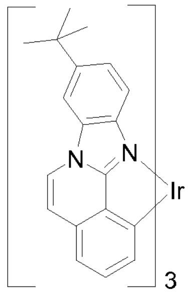

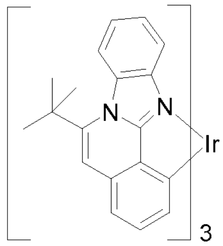

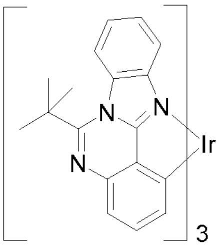

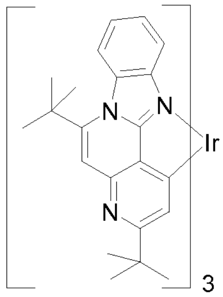

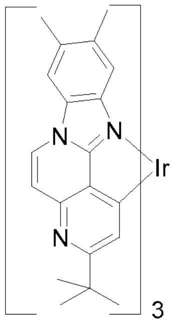

Die oben beschriebenen Verbindung, umfassend Strukturen der Formel (I) bzw. die oben aufgeführten bevorzugten Ausführungsformen können in einer organischen Elektrolumineszenzvorrichtung bevorzugt als aktive Komponente verwendet werden. Die erfindungsgemäße organischen Elektrolumineszenzvorrichtung enthält also Anode, Kathode und mindestens eine Schicht, welche mindestens eine Verbindung, umfassend Strukturen der Formel (I), enthält.

Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Elektronentransportmaterial in organischen Elektrolumineszenzvorrichtungen. Eine bevorzugte Ausführungsform der Erfindung sind daher organische Elektrolumineszenzvorrichtungen, in denen mindestens eine Schicht mit mindestens einer erfindungsgemäßen Verbindung enthalten ist.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. Eine emittierende Schicht umfasst mindestens eine emittierende Verbindung.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B.

Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend efindungsgemäße Verbindungen umfassend Strukturen gemäß Formel (I) bzw. die oben aufgeführten bevorzugten Ausführungsformen angewandt werden.

Die erfindungsgemäßen organische Elektrolumineszenzvorrichtungen, zeichnen sich durch einen oder mehrere der folgenden überraschenden Vorteile gegenüber dem Stand der Technik aus:

1. Organische Elektrolumineszenzvorrichtungen enthaltend Verbindungen, mit Strukturen gemäß Formel (I), insbesondere als elektronenleitende Materialien, weisen eine sehr gute Lebensdauer auf.

2. Verbindungen, mit Strukturen gemäß Formel (I) zeichnen sich durch eine ausgezeichnete thermische Stabilität aus, wobei Verbindungen mit einer Molmasse von weniger als ca. 1200 g/mol gut sublimierbar sind.

3. Verbindungen, mit Strukturen gemäß Formel (I) weisen eine sehr hohe Löslichkeit auf und bilden aus Lösungen

sehr gute Filme.

4. Verbindungen, mit Strukturen gemäß Formel (I) zeigen eine sehr hohe Redoxstabilität in Lösung. Hierdurch wird die Handhabung sehr erleichtert, so dass die Verbindungen sehr und gut gereinigt und gelagert werden können.

5. Organische Elektrolumineszenzvorrichtungen enthaltend Verbindungen mit Strukturen gemäß Formel (I) als elektronenleitende Materialien weisen eine hervorragende Effizienz auf. Insbesondere ist die Effizienz deutlich höher gegenüber analogen Verbindungen, die keine Struktureinheit gemäß Formel (I) enthalten.

6. Die erfindungsgemäßen Verbindungen, mit Strukturen gemäß Formel (I) zeigen eine sehr hohe Stabilität und führen zu Verbindungen mit einer sehr hohen Lebensdauer.

7. Mit Verbindungen, mit Strukturen gemäß Formel (I) kann in organischen Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

8. Die Verwendung von Verbindungen, mit Strukturen gemäß Formel (I) in Schichten organischer Elektrolumineszenzvorrichtungen führt zu einer hohen Mobilität der Elektron-Leiterstrukturen.

9. Verbindungen mit Strukturen gemäß Formel (I) weisen eine ausgezeichnete Glastemperatur auf.

10. Die Verbindungen, umfassend Strukturen gemäß Formel (I) weisen ein überraschend hohes Triplett-Niveau $T_1$ auf.

Diese oben genannten Vorteile gehen nicht mit einer Verschlechterung der weiteren elektronischen Eigenschaften einher.

Ein weiterer Gegenstand der vorliegenden Erfindung ist die Verwendung einer erfindungsgemäßen Verbindung in einer organischen Elektrolumineszenzvorrichtung als Lochblockiermaterial, Elektroneninjektionsmaterial, und/oder Elektronentransportmaterial.

Es sei darauf hingewiesen, dass Variationen der in der vorliegenden Erfindung beschriebenen Ausführungsformen unter den Umfang dieser Erfindung fallen.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße organische Elektrolumineszenzvorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

### A-1) Beispiel 1: Synthese der Verbindungen (1-1) bis (1-19)

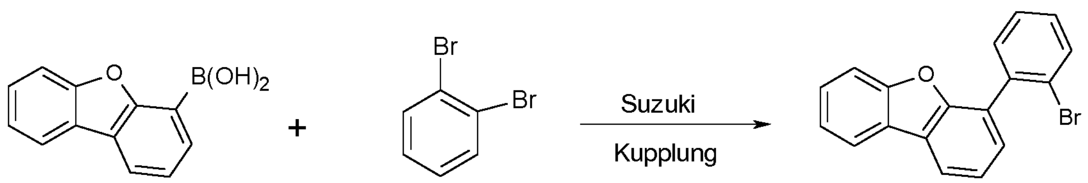


Int-1

**Synthese von 4-(2-Bromo-phenyl)-dibenzofuran Int-1**

100 g (462 mmol) Dibenzofuran-4-Boronsäure, 106 g (439 mmol) 1,2-Dibrombenzol und 10,7 g (9,2 mmol) $Pd(Ph_3P)_4$ werden in 980 mL Dioxan suspendiert. Zu dieser Suspension werden 979 mL Kaliumcarbonat Lösung 2 M langsam addiert, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockne eingeengt. Der Rückstand wird durch Chromatographie an Kieselgel gereinigt. Ausbeute: 87 g (270 mmol), 58% d. Th., Reinheit nach HPLC >98%.

Analog zu der beschriebenen Synthese von Verbindung **Int-1** werden die folgenden Verbindungen hergestellt:

| | Edukt 1 | Edukt 2 | Produkt | Ausbeute |
|---|---|---|---|---|
| **Int-2** | 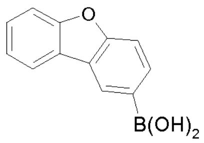 [402936-15-6] | 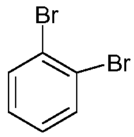 | 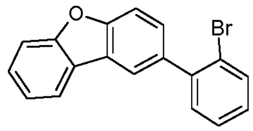 | 62% |
| **Int-3** | 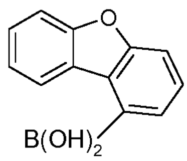 [162607-19-4] | 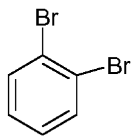 | 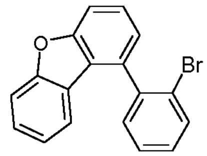 | 52% |
| **Int-4** | 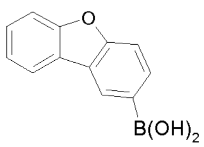 [402936-15-6] | 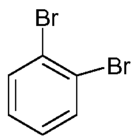 | 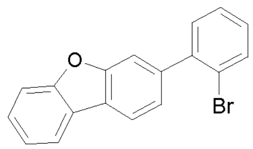 | 55% |
| **Int-5** | 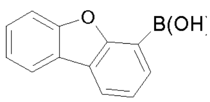 [100124-06-9] | 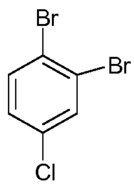 | 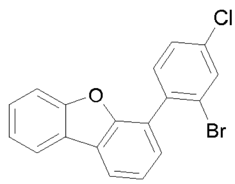 | 35% |
| **Int-6** | 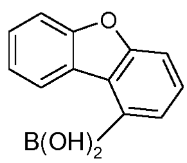 [162607-19-4] | 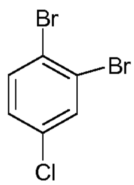 | 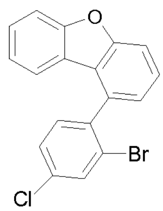 | 40% |
| **Int-7** | 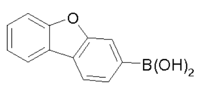 [395087-98-5] | 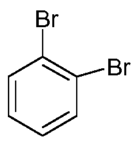 | 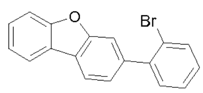 | 43% |

(fortgesetzt)

| | Edukt 1 | Edukt 2 | Produkt | Ausbeute |
|---|---|---|---|---|
| **Int-8** | 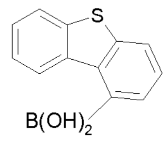 [1245943-60-5] | 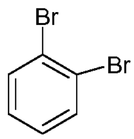 | 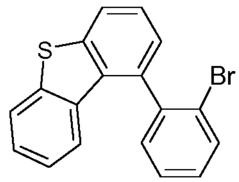 | 42% |
| **Int-9** | 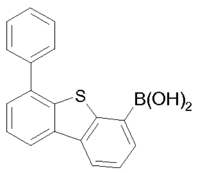 [1115640-18-0] | 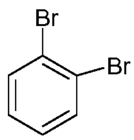 | 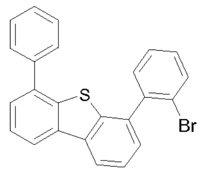 | 40% |

Analog zu der beschriebenen Synthese von Verbindung **Int-29** werden die folgenden Verbindungen hergestellt:

| | Edukt 1 | Edukt 2 | Produkt | Ausbeute |
|---|---|---|---|---|
| **Int-10** | 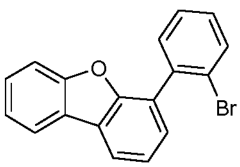 | 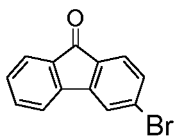 [2041-19-2] | 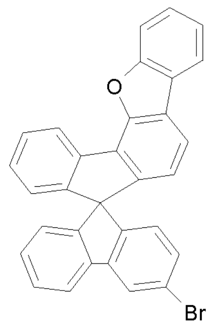 | 80% |
| **Int-11** | 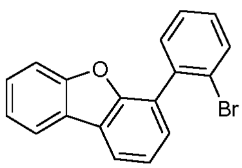 | 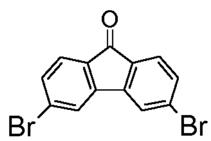 [216312-73-1] | 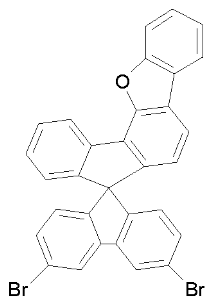 | 70% |
| **Int-12** | 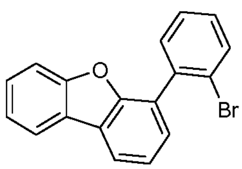 | 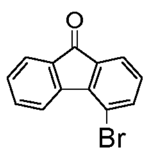 [4269-17-4] | 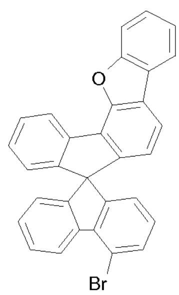 | 70% |

(fortgesetzt)

| | Edukt 1 | Edukt 2 | Produkt | Ausbeute |
|---|---|---|---|---|
| **Int-13** | 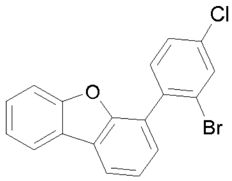 | 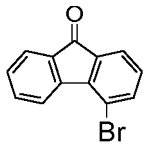 [4269-17-4] | 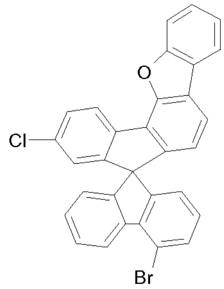 | 72% |
| **Int-14** | 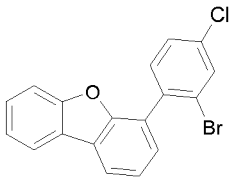 | 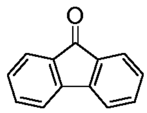 [486-25-9] | 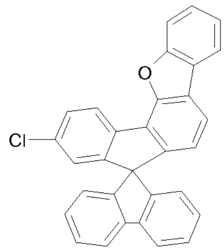 | 75% |
| **Int-15** | 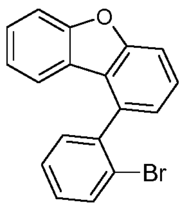 | 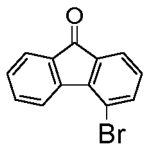 [4269-17-4] | 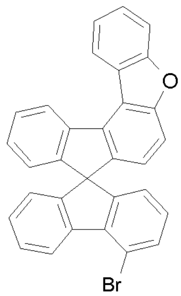 | 80% |
| **Int-16** | 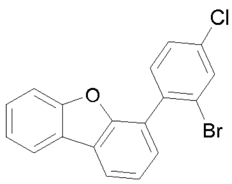 | 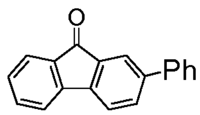 [3096-49-9] | 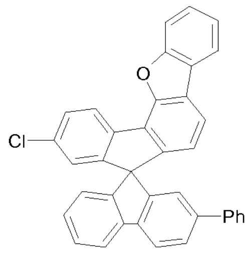 | 75% |
| **Int-17** | 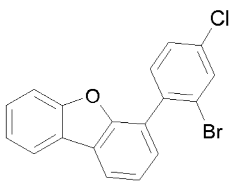 | 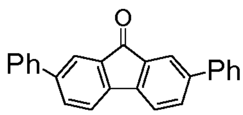 [115033-91-5] | 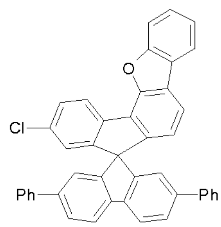 | 73% |

(fortgesetzt)

| | Edukt 1 | Edukt 2 | Produkt | Ausbeute |
|---|---|---|---|---|
| **Int-18** | 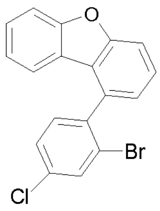 | 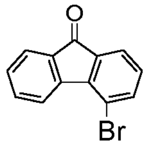 [4269-17-4] | 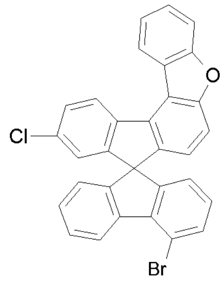 | 70% |
| **Int-19** | 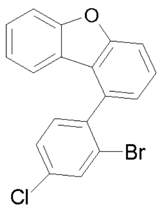 | 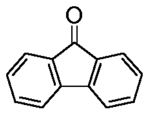 [486-25-9] | 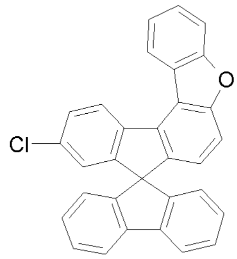 | 75% |
| **Int-20** | 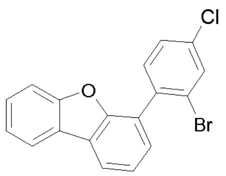 | 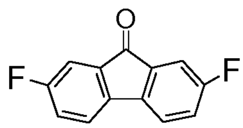 [24313-53-9] | 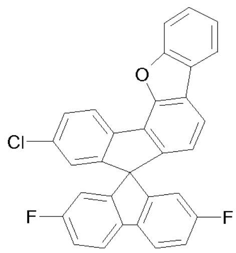 | 65% |
| **Int-21** | 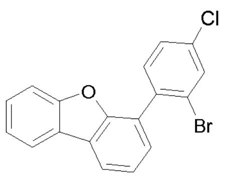 | 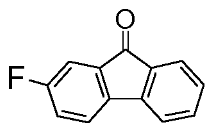 [343-01-1] | 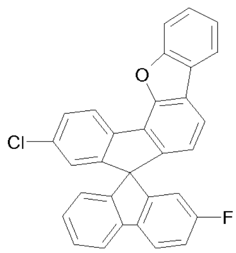 | 58% |
| **Int-22** | 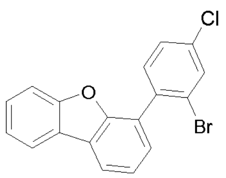 | 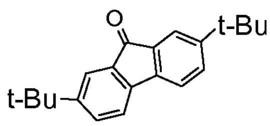 [58775-13-6] | 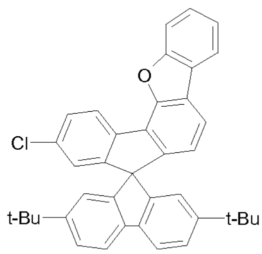 | 80% |

(fortgesetzt)

| | Edukt 1 | Edukt 2 | Produkt | Ausbeute |
|---|---|---|---|---|
| **Int-23** | 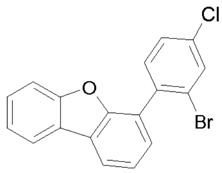 | 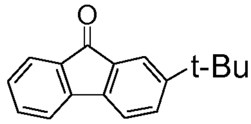 [58775-11-4] | 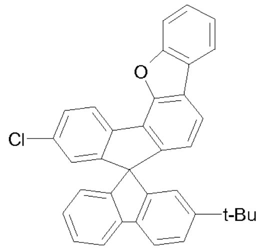 | 72% |
| **Int-24** | 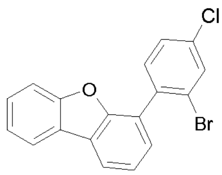 | 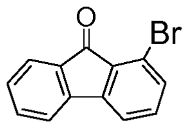 [36804-63-4] | 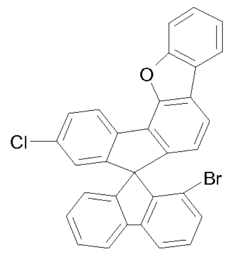 | 75% |
| **Int-25** | 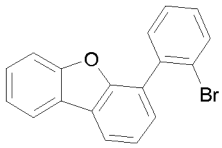 | 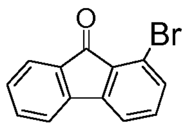 [36804-63-4] | 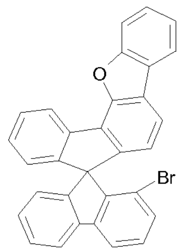 | 67% |
| **Int-26** | 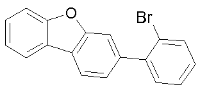 | 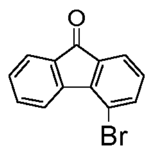 [4269-17-4] | 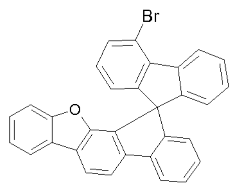 | 59% |
| **Int-27** | 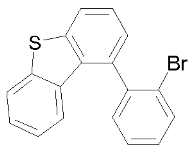 | 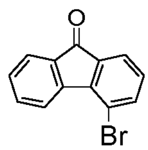 [4269-17-4] | 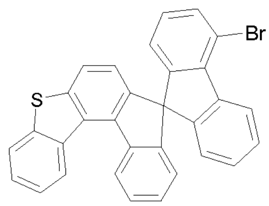 | 47% |
| **Int-28** | 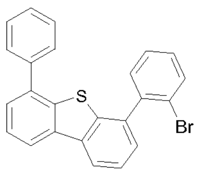 | 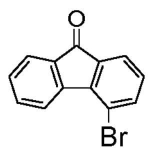 [4269-17-4] | 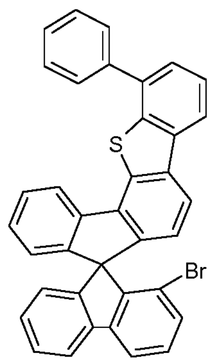 | 51% |

**Synthese der Verbindung (1-1)**

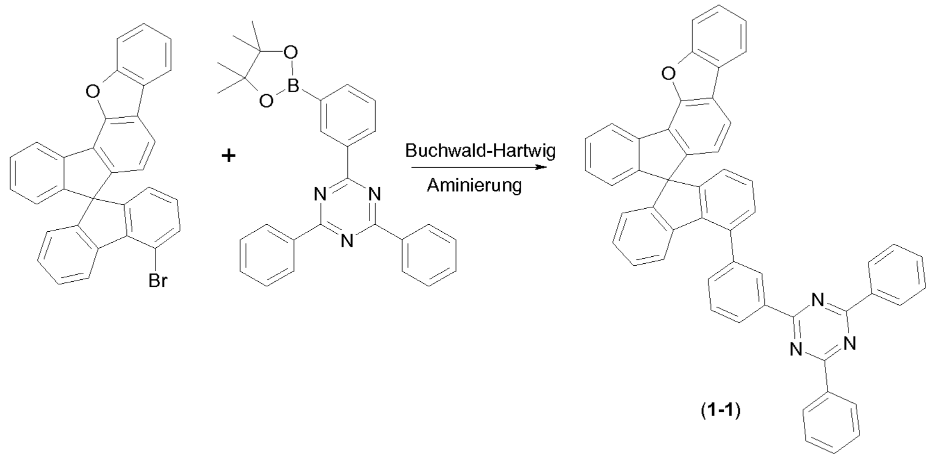


(1-1)

#

# nicht erfindungsgemäß

15,5 g (32 mmol) Bromo-Spiroderivats, 15,2 g (35 mmol) 2,4-dophenyl-6-[3-(4,4,5,5-tetramethyl-1,3,2-dioxaborolan-yl]phenyl]1,3,5-Triazin-Dibromdibenzofuran, 31 ml (63 mmol) $Na_2CO_3$ (2M-Lösung) werden in 120 mL Toulol, 120 mL Ethanol suspendiert. Zu dieser Suspension werden 0,73 g (0,63 mmol) $Pd(PPh_3)_4$ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt.Der Rückstand wird aus Toluol und aus Dichlormethan / iso-Propanol umkristallisiert und abschließend im Hochvakuum (p = 5 x $10^{-5}$ mbar) sublimiert. Die Reinheit beträgt 99.9%(HPLC). Die Ausbeute von Verbindung **(1-1)** beträgt 16,6 g (23 mmol), entsprechend 73 % der Theorie.

**Synthese der Verbindungen (1-2) bis (1-19)**

Analog zu der in Beispiel 1 beschriebenen Synthese von Verbindung **(1-1)** werden auch die folgenden Verbindungen **(1-2)** bis **(1-19)** hergestellt.

| | Edukt 1 | Edukt 2 | Produkt | Ausbeute |
|---|---|---|---|---|
| **1-2** | 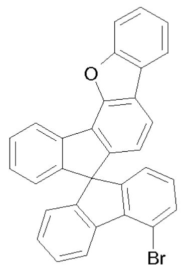 | 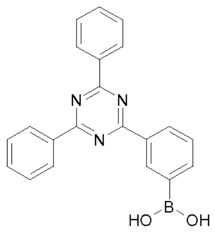 [1269508-31-7 ] | 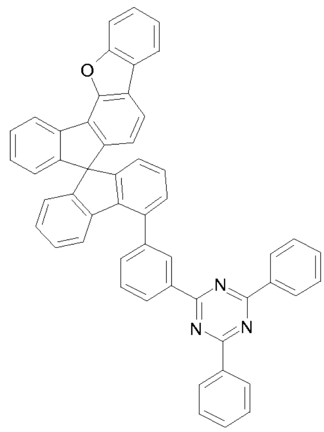 # | 74 % |

(fortgesetzt)

| | Edukt 1 | Edukt 2 | Produkt | Ausbeute |
|---|---|---|---|---|
| **1-3** | 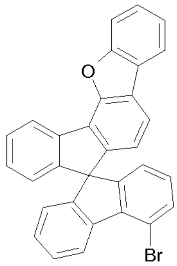 | 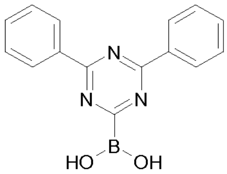 [1251825-65-6 ] | 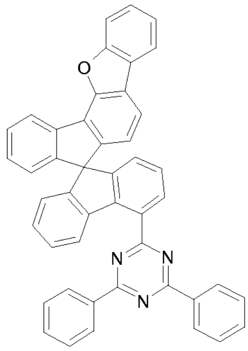 # | 81% |
| **1-4** | 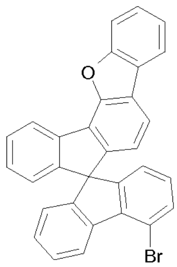 | 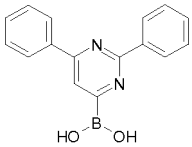 [1251825-66-7 ] – | 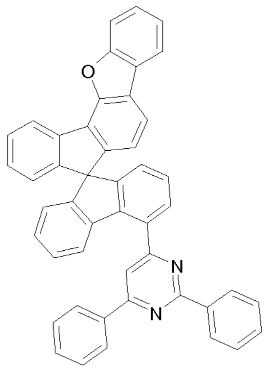 # | 84% |
| **1-5** | 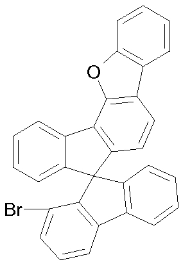 | 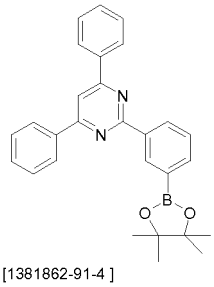 [1381862-91-4 ] | 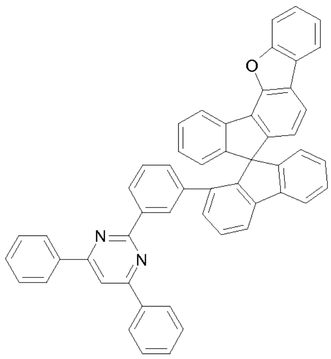 # | 67% |
| **1-6** | 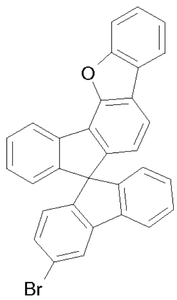 | 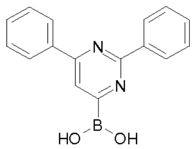 [1251825-66-7 ] | 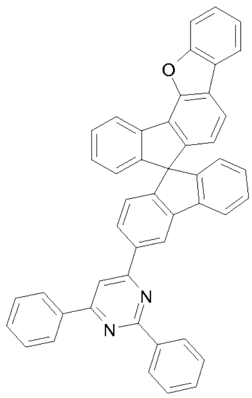 # | 72% |

(fortgesetzt)

| | Edukt 1 | Edukt 2 | Produkt | Ausbeute |
|---|---|---|---|---|
| **1-7** | 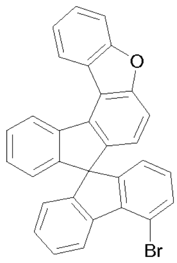 | 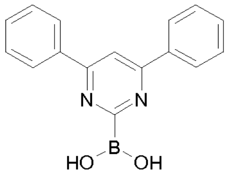 [1314221-56-1 ] | 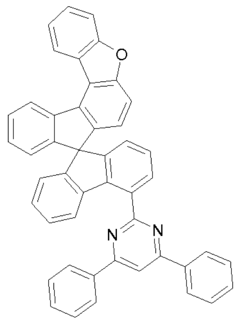 # | 82% |
| **1-8** | 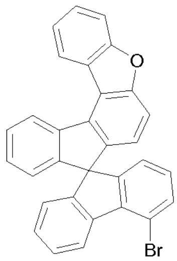 | 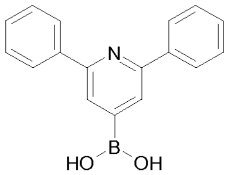 [1246022-33-2 ] | 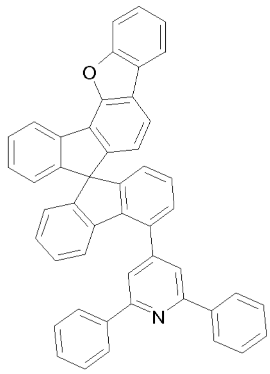 # | 77% |
| **1-9** | 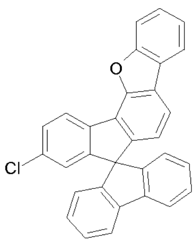 | 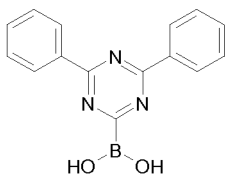 [1251825-65-6 ] [ | 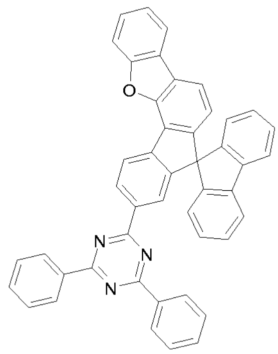 # | 71 % |
| **1-10** | 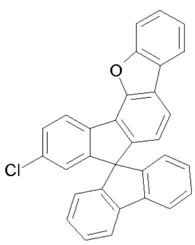 | 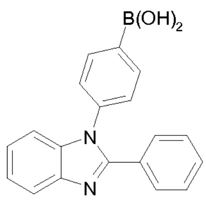 [867044-33-5] | 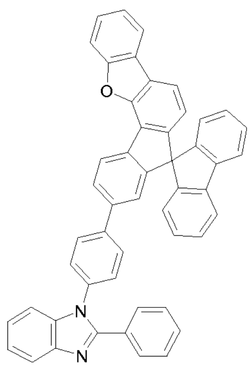 # | 81% |

(fortgesetzt)

| | Edukt 1 | Edukt 2 | Produkt | Ausbeute |
|---|---|---|---|---|
| **1-11** | 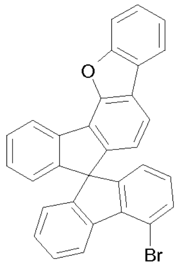 | 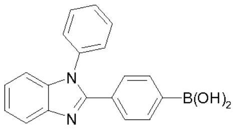 [952514-79-3] 2.eq. | 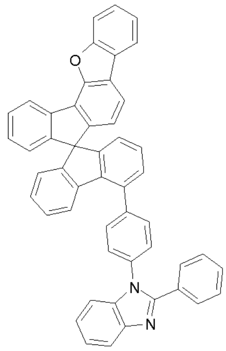 # | 72% |
| **1-12** | 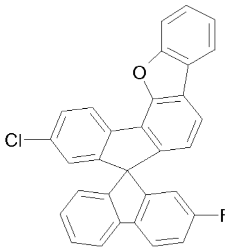 | 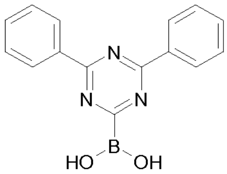 [1251825-65-6 ] | 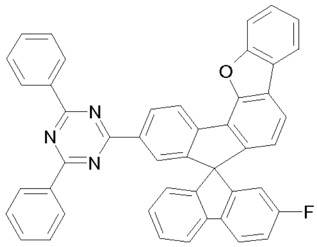 # | 70% |
| **1-13** | 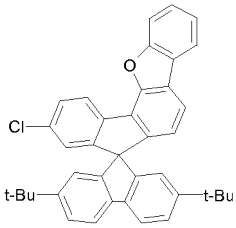 | 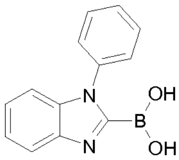 [1214723-25-7] | 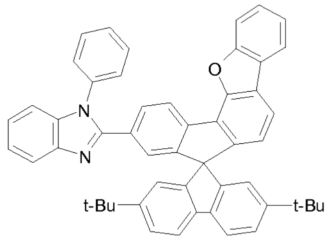 # | 80% |
| **1-14** | 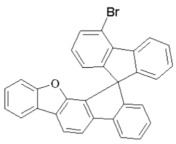 | 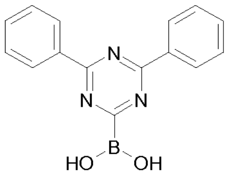 [1251825-65-6 ] | 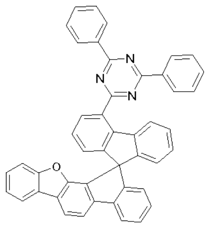 # | 76% |
| **1-15** | 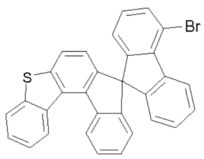 | 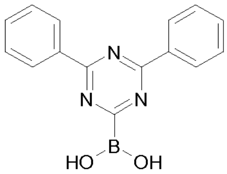 [1251825-65-6 ] | # 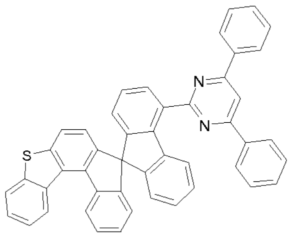 | 70% |

(fortgesetzt)

| | Edukt 1 | Edukt 2 | Produkt | Ausbeute |
|---|---|---|---|---|
| **1-16** | 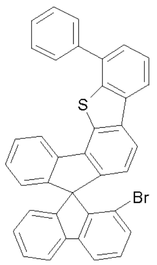 | 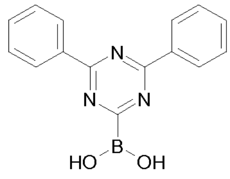 [1251825-65-6 ] | 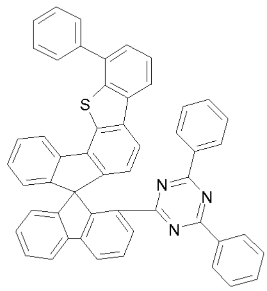 # | 66% |
| **1-17** | 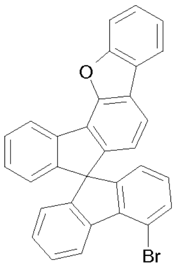 | 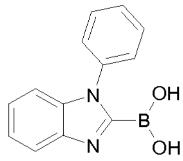 [1214723-25-7] | 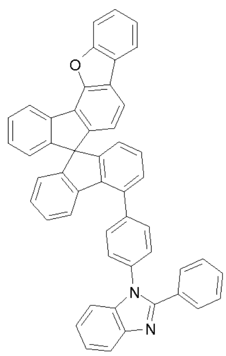 # | 72% |
| **1-18** | 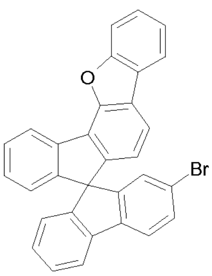 [1376933-75-3] | 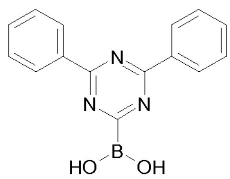 [1251825-65-6 ] | 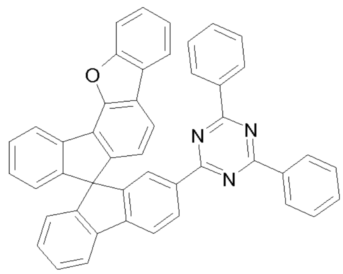 [1376933-75-3] # | 76% |
| **1-19** | 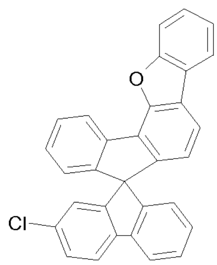 | 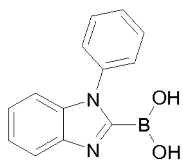 [1214723-25-7] | # 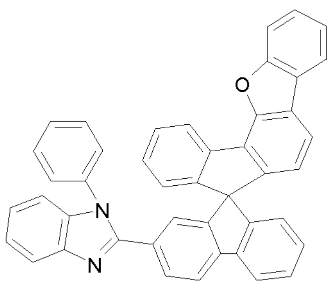 | 77% |
| # nicht erfindungsgemäß | | | | |

Die Verbindung 1-19 kann alternativ auch gemäß dem folgenden Schema hergestellt werden. Dabei wird die unsubstituierte Benzimidazolboronsäure eingesetzt, um die Verbindung 1-19a zu erhalten. Schritt (I) erfolgt dabei analog zu denen der Synthesen der Verbindungen 1-1 bis 1-19.

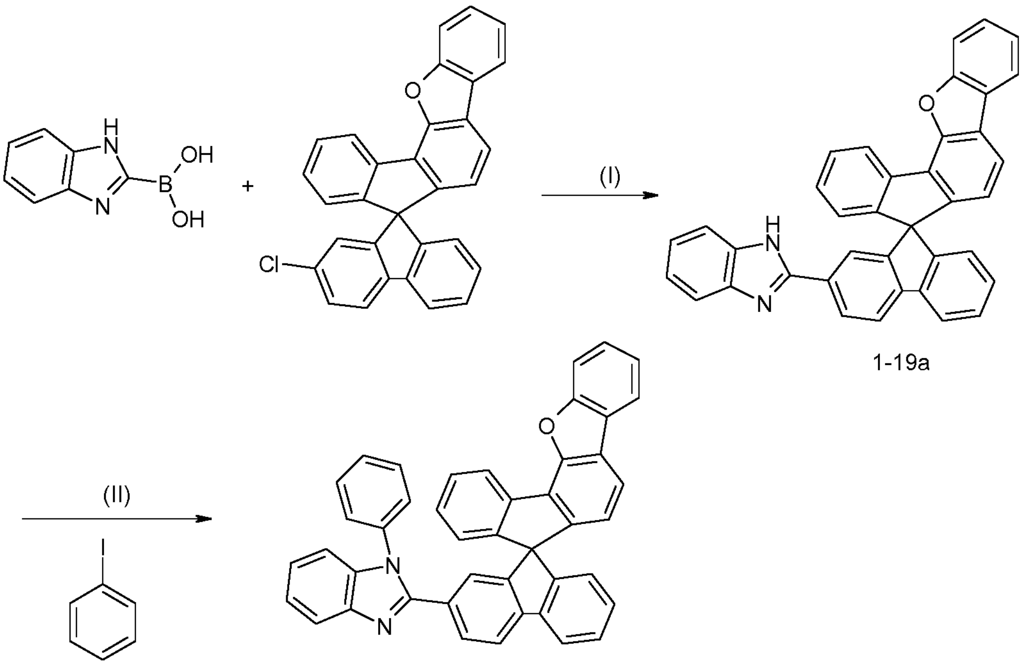


1-19a

1-19

#

# nicht erfindungsgemäß

In Schritt (II) wird das Produkt aus Schritt (I) mit Iodbenzol umgesetzt. Dazu werden unter Schutzgas 26 g (50 mmol) der Verbindung 1-19a, 560 mg (25 mmol) $Pd(OAc)_2$, 19,3 g (118 mmol) CuI, 20,8 g (100 mmol) Iodbenzol in 300 ml entgastes DMF suspendiert und die Reaktionsmischung wird 24 h unter Rückfluss bei 140°C erhitzt. Nach dem Erkalten der Mischung wird das Lösungsmittel im Vakuum entfernt, der Rückstand wird in Dichloromethan gelöst und mit Wasser versetzt. Danach wird die organische Phase abgetrennt und über Kieselgel filtriert. Das Produkt wird mittels Säulenchromatographie an Kieselgel mit Toluol/Heptan (1:2) gereinigt und abschließend im Hochvakuum ($p = 5 \times 10^{-7}$ mbar) sublimiert (Reinheit 99,9%). Die Ausbeute beträgt 18,5 g (30 mmol), entsprechend 62% der Theorie.

**A-2) Beispiel 2: Synthese der Verbindungen (2-1) bis (2-4)**

**Synthese der Zwischenstufe Int-29**

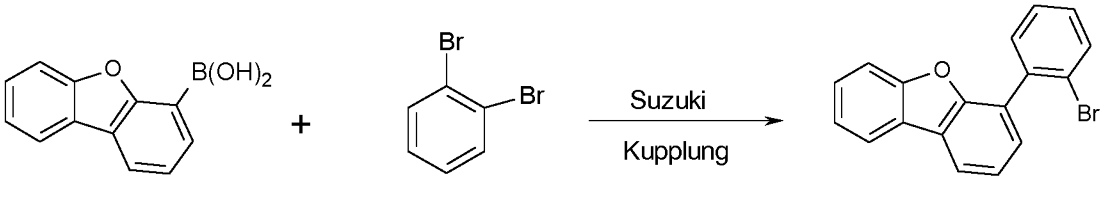


Int-1

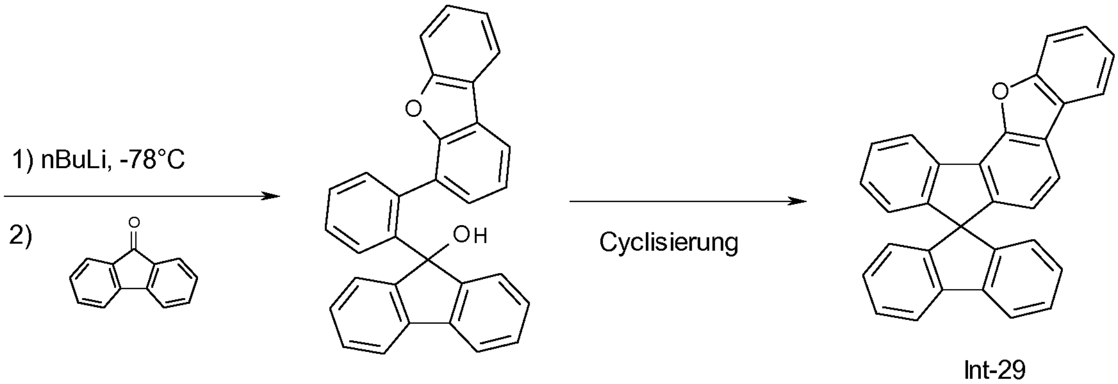


Int-29

31 g (90 mmol) 4-(2-Bromo-phenyl)-dibenzofuran wird in 300 mL THF bei -78°C vorgelegt. Bei dieser Temperatur werden 40 mL BuLi (2M in Hexan) zugetropft. Nach 1 Stunde wird 16,9 g (94 mmol) Fluoren-9-on in 200 mL THF zugetropft. Der Ansatz wird über Nacht bei Raumtemperatur rühren gelassen, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand wird ohne weitere Aufreinigung mit 94 mL HCl und 1074 mL AcOH bei 100°C über Nacht unter Rückfluß erhitzt. Nach Erkalten saugt man vom ausgefallenen Feststoff ab, wäscht diesen einmal mit 100 ml Wasser, dreimal mit je 100 ml Ethanol und kristallisiert abschließend aus Heptan um. Ausbeute: 23.1 g (57 mmol), 58 %; Reinheit ca. 98 % nach $^1$H-NMR.

**Synthese der Verbindungen (Int-30) bis (Int-32)**

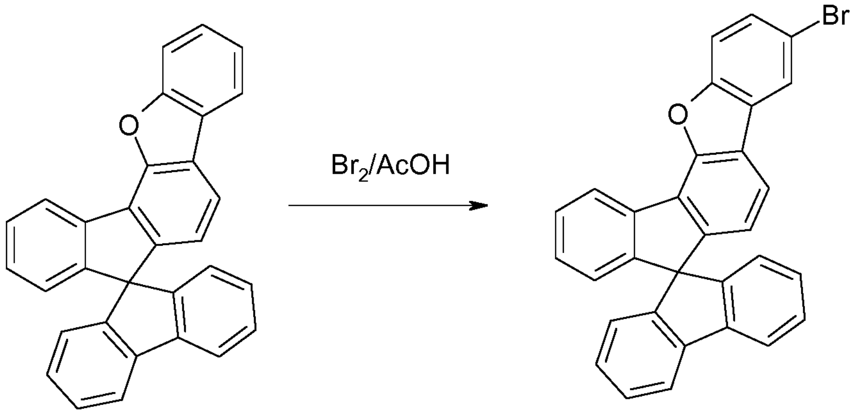


Int-30

15,0g (36,9 mmol) eines Spiroderivates werden in 150 mL AcOH gelöst und bei Raumtemperatur portionsweise mit 5,7 g (29 mmol) Brom gekost in 20 ml AcOH versetzt. Nach vollständiger Zugabe wird die mischung auf 50-60°C erhitzt und nach vollständiger Umsetzung werden Wasser und Essigsäureethylester dazu gegeben, die organische Phase abgetrennt, getrocknet und eingeengt. Das Rohprodukt wird anschließend mit MeOH/Heptan (1:1) mehrfach heiß

ausgerührt. Ausbeute: 14,3 g (80%) des Brom-Spiroderivats Int-30.

Analog dazu werden die folgenden bromierten Verbindungen hergestellt:

| | Edukt 1 | Bromierung Reagenz | Produkt | Ausbeute |
|---|---|---|---|---|
| **Int-31** | 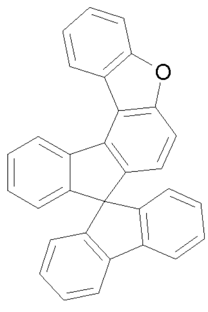 | Br/AcOH | 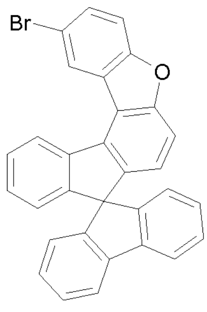 | 58 % |
| **Int-32** | 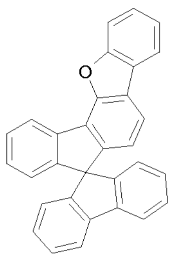 | Br/AcOH | 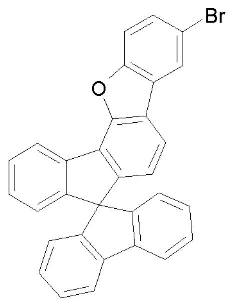 | 55% |

**Synthese der Verbindungen (Int-33) bis (Int-40)**

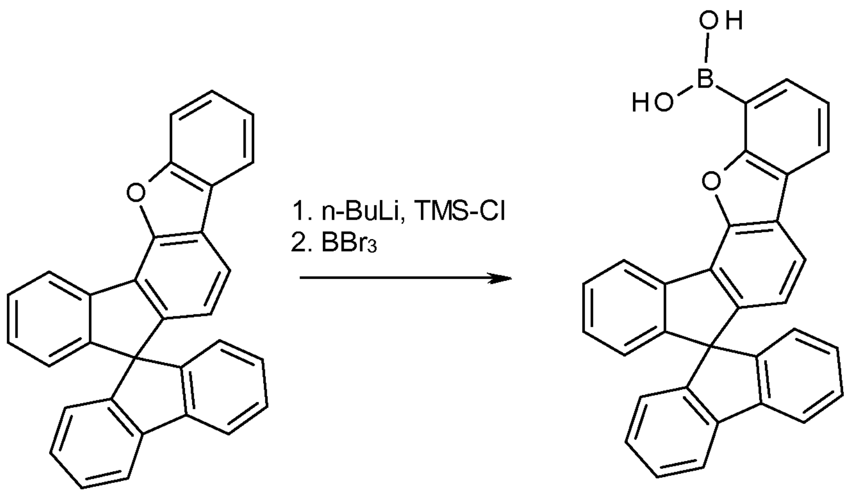


In-31

Zu einer Lösung von 8,0 g (19,7 mmol) eines Spiro-Derivates und 2,93 mL (19,7 mmol) TMEDA in 100 mL getrocknetem Tetrahydrofuran wird bei 0 °C *n*-Butyllithium (2,5 M in Hexan, 19,7 ml, 49,2 mmol) so zugegeben, dass die Temperatur nicht über 10 °C steigt und anschließend 4 h bei Raumtemperatur gerührt. Dann wird die Reaktionsmischung auf -78 °C abgekühlt, Chlortrimethylsilan (7,51 mL, 58,0 mmol) zugegeben und über Nacht gerührt, wobei man die Reaktionsmischung auf Raumtemperatur erwärmen lässt. Die Reaktionsmischung wird über wenig Kieselgel filtriert und am Rotationsverdampfer eingeengt. Der ölige Rückstand wird in 100 ml getrocknetem Dichlormethan gelöst, mit Bortribromid (2,24 ml, 23,6 mmol) versetzt und über Nacht gerührt. Nach vollständiger Umsetzung wird die Reaktionsmischung auf Eis gegeben. Die organische Phase wird abgetrennt und die Wässrige mit Essigsäureethylester extrahiert.

Die vereinigten organischen Phasen werden getrocknet und eingeengt. Das trübe leicht bräunliche Rohprodukt wird ohne weitere Aufreinigung weiter umgesetzt. Ausbeute: 9,3 g (105 %).

Analog dazu werden die folgenden borylierten Verbindungen hergestellt. Isomere wurden als entsprechende Pinacolester chromatographisch getrennt:

| | Edukt 1 | Reagenz | Produkt 1 | Produkt 2 |
|---|---|---|---|---|
| **Int-32-33** | 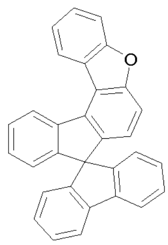 | BuLi/BBr3 | 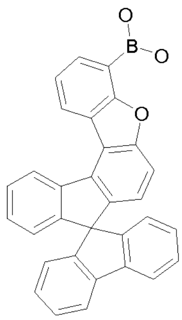 31% | 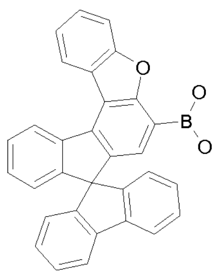 67% |
| **Int-34-35** | 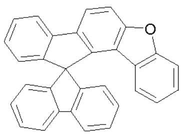 | BuLi/BBr3 | 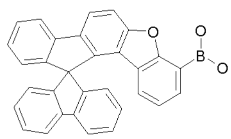 45% | 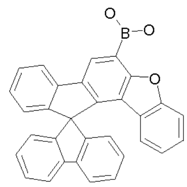 52% |
| **Int-36-37** | 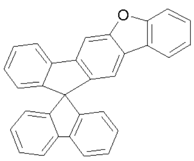 | BuLi/BBr3 | 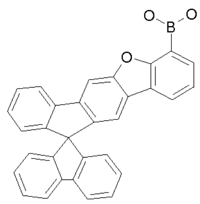 38% | 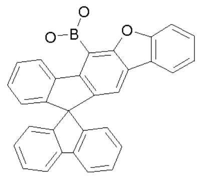 58% |
| **Int-38-39** | 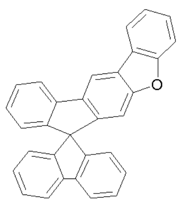 | BuLi/BBr3 | 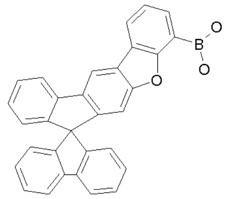 77% | 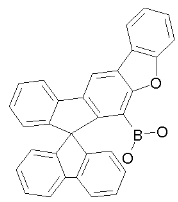 13% |
| **Int-40** | 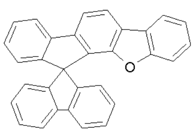 | BuLi/BBr3 | 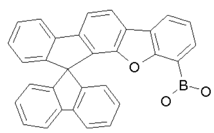 94% | |

**Synthese der Verbindungen (2-1) bis (2-4)**

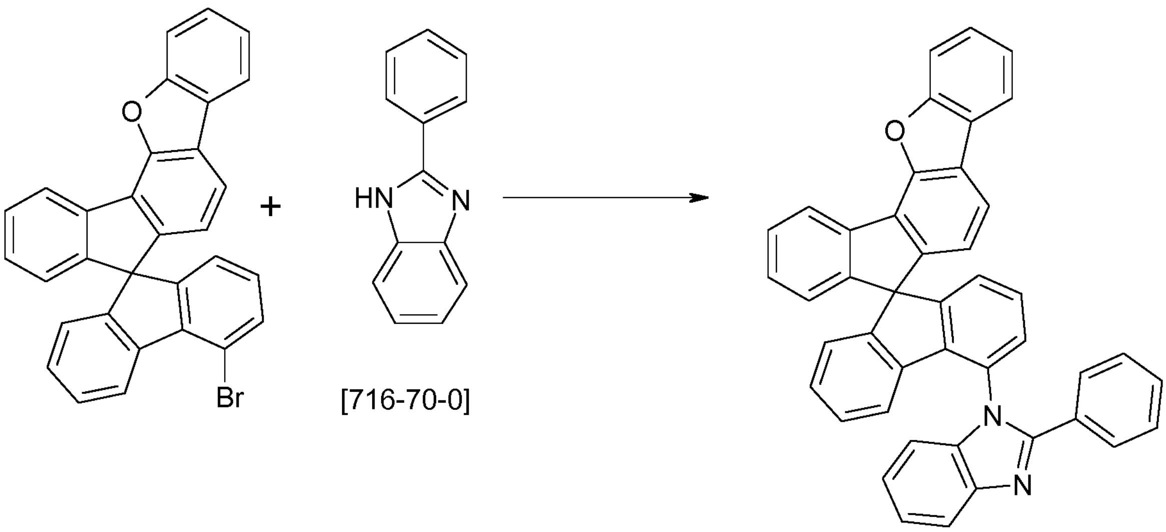


2-1

nicht erfindungsgemäß

39 g (81 mmol) eines Bromo-Spiroderivats, 115 g (406 mmol) 2-Phnyl-1-benzimidazol, 22.4 g (162 mmol) Kaliumcarbonat, 1.84 g (8.1 mmol) 1,3-Di(2-pyridyl)-1,3-propandion, 1.55 g (8.1 mmol) Kupferiodid und 1000 mL DMF werden 30 h unter Rückfluss erhitzt. Anschließend wird die Reaktionsmischung am Rotationsverdampfer bis zur Trockene eingeengt. Der Rückstand wird in THF gelöst und über ein kurzes Kieselgel-Bett filtriert, dann das Lösungsmittel im Vakuum entfernt. Anschließend wird der Feststoff aus Heptan/THF umkristallisiert und über Aluminiumoxid mit Heptan/Toluol heiß extrahiert. Der beim Erkalten ausgefallene Feststoff wird filtriert und getrocknet. Die Ausbeute von Verbindung **(2-1)** beträgt 36g (60 mmol), 75 %.

Analog zu der in Beispiel 2 beschriebene Synthesen von Verbindung **(2-1)** werden auch die folgenden Verbindungen **(2-2)** bis **(2-4)** hergestellt.

| | Edukt 1 | Edukt 2 | Produkt | Ausbeute |
|---|---|---|---|---|
| **2-2** | 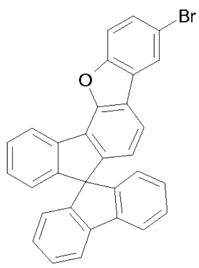 | 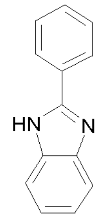 [716-70-0] | 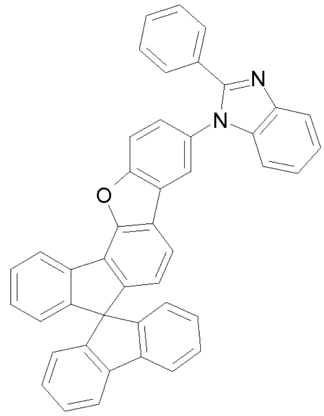 | 82% |

(fortgesetzt)

| | Edukt 1 | Edukt 2 | Produkt | Ausbeute |
|---|---|---|---|---|
| **2-3** | 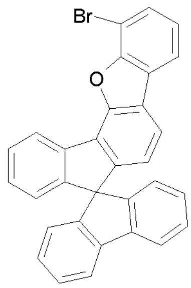 | 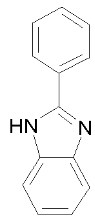 [716-70-0] | 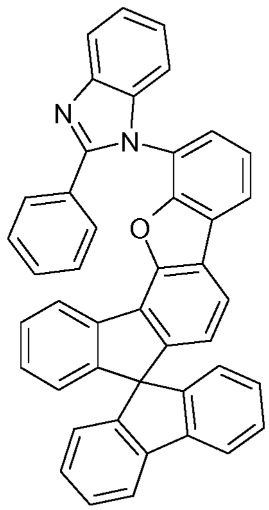 | 64% |
| **2-4** | 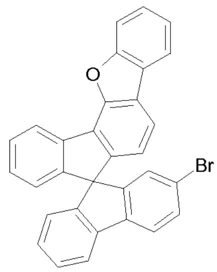 [1376933-75-3] | 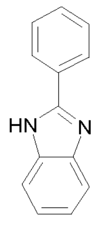 [716-70-0] | 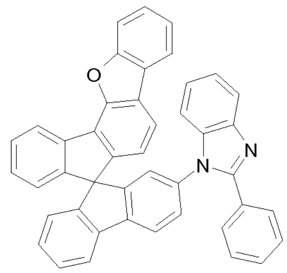 # | 68% |
| # nicht erfindungsgemäß | | | | |

**A-3) Beispiel 3: Synthese der Verbindungen 3-1 bis 3-5**

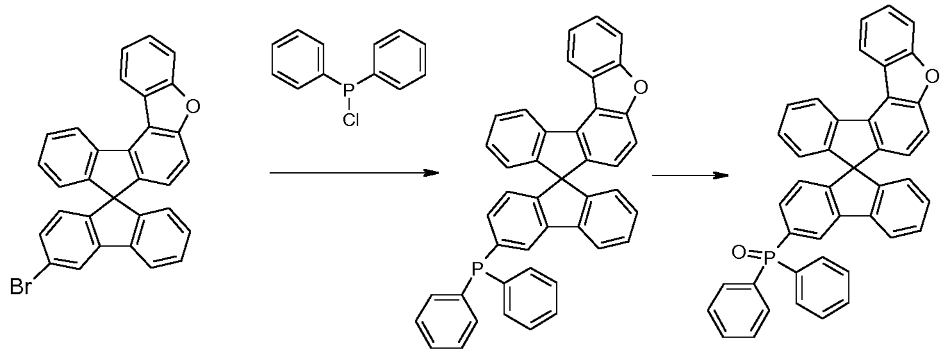

3-1 #

# nicht erfindungsgemäß

Eine auf -78 °C gekühlte Lösung von 137 g (270 mmol) Bromo-Spiroderivats in 1500 ml THF wird tropfenweise mit 110 ml (276 mmol) n-Buthyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 50

ml (270 mmol) Chlordiphenylphosphin und rührt weiter 3 Stunden bei -70°C. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml $CH_2Cl_2$ gelöst und mit 270 ml 30% Hydroxyperoxid Lösung 12 Stunden bei Raumtemperatur gerührt. Die organische Phase wird getrennt und eingeengt, der farblose Feststoff wird abgesaugt, mit n-Heptan gewaschen und im Vakuum getrocknet und aus Dichlormethan / *Toluol* umkristallisiert und abschließend im Hochvakuum (p = 5 x $10^{-5}$ mbar) sublimiert. Die Reinheit beträgt 99.9%(HPLC). Die Ausbeute von Verbindung **(3-1)** beträgt 119 g (191 mmol), entsprechend 70 % der Theorie

Analog werden die folgenden Verbindungen erhalten:

| | Edukt 1 | Edukt 2 | Produkt | Ausbeute |
|---|---|---|---|---|
| **3-2** | 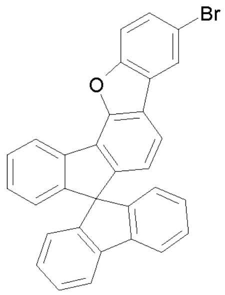 | 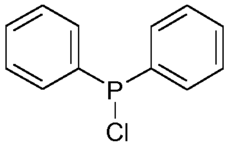 [1079-66-9] | 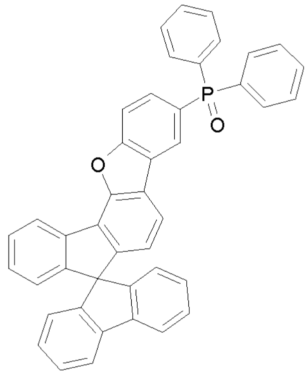 | 72% |
| **3-3** | 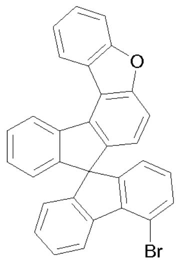 | 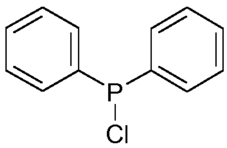 [1079-66-9] | 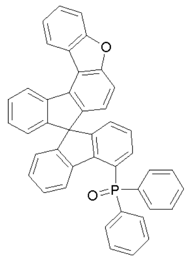 # | 69% |
| **3-4** | 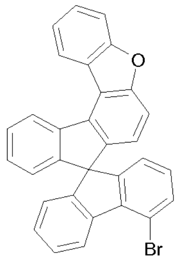 | 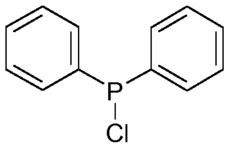 [1079-66-9] | 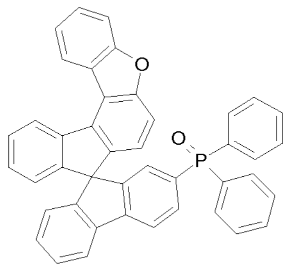 # | 72% |
| **3-5** | 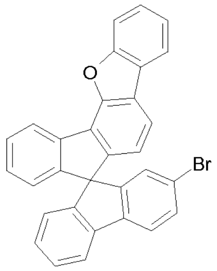 [1376933-75-3] | 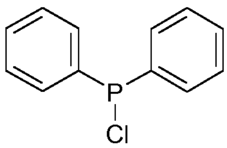 [1079-66-9] | 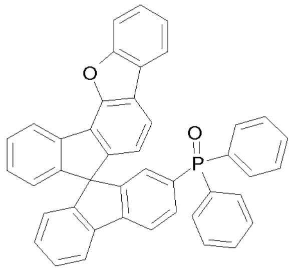 # | 73% |
| # nicht erfindungsgemäß | | | | |

**A-4) Beispiel 4: Synthese der Verbindungen 4-1 bis 4-18**

**Synthese der Zwischenstufe Int-41**

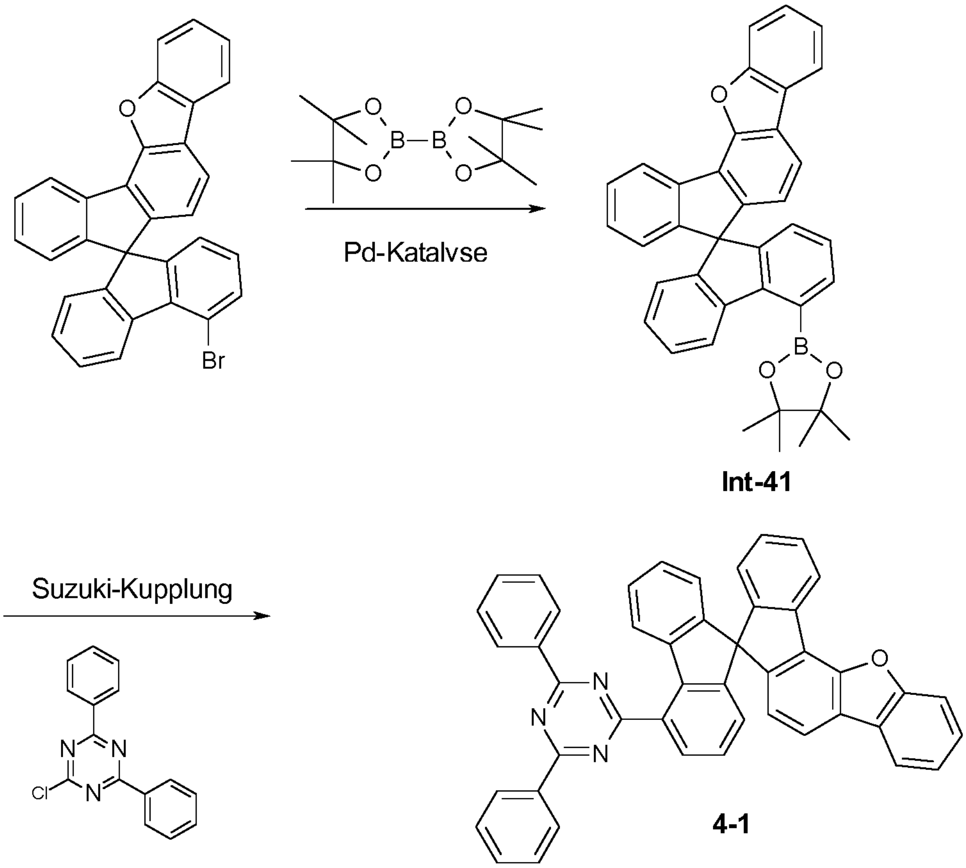


**Int-41**

**4-1**

#

# nicht erfindungsgemäß

50 g (103 mmol) des Spirofluoren-bromderivats, 32 g (123 mmol) Bis(pinacolato)diboran und 30 g (309 mmol) Kalliumacetat werden in 800 ml Dioxan suspendiert. Zu dieser Suspension werden 2.5 g (3.09 mmol) 1,1-Bis(diphenylphosphino)ferrocen-dichloropalladium(II) Komplex mit DCM gegeben. Die Reaktionsmischung wird 16 h unter Rückfluss erhitzt. Nach Erkalten wird die organische Phase abgetrennt, dreimal mit 400 mL Wasser gewaschen und anschließend zur Trockne eingeengt. Der Rückstand wird aus Toluol umkristallisiert (52 g, 95% Ausbeute).

Analog dazu werden folgende Verbindungen hergestellt:

| | Edukt 1 | Produkt | Ausbeute |
|---|---|---|---|
| **Int-42** | 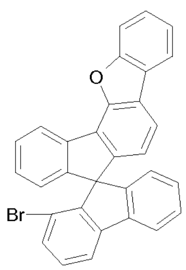 | 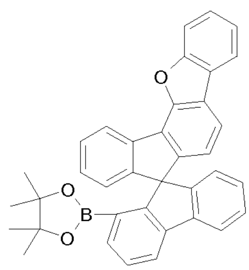 | 90% |

(fortgesetzt)

| | Edukt 1 | Produkt | Ausbeute |
|---|---|---|---|
| **Int-43** | 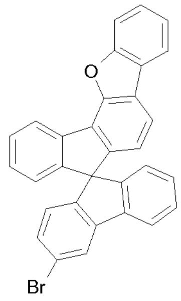 | 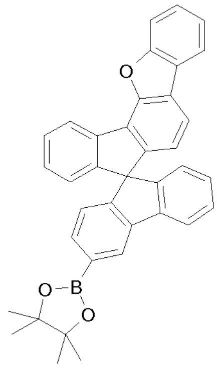 | 80% |
| **Int-44** | 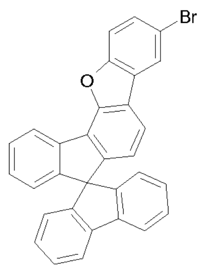 | 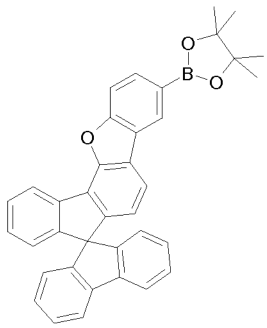 | 88% |
| **Int-45** | 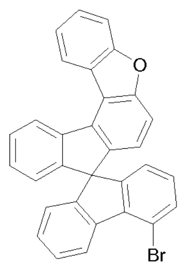 | 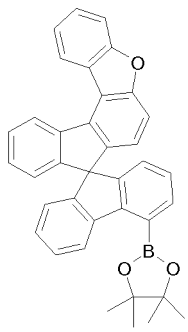 | 88% |
| **Int-46** | 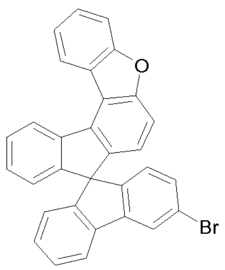 | 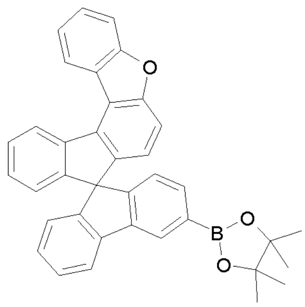 | 91% |
| **Int-47** | 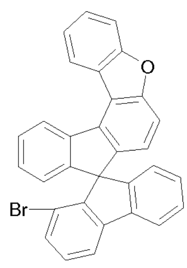 | 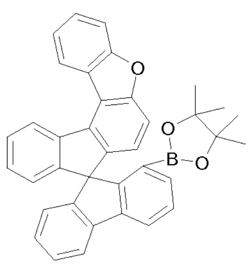 | 85% |

(fortgesetzt)

| | Edukt 1 | Produkt | Ausbeute |
|---|---|---|---|
| **Int-48** | 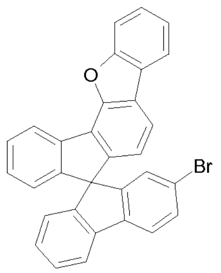 [1376933-75-3] | 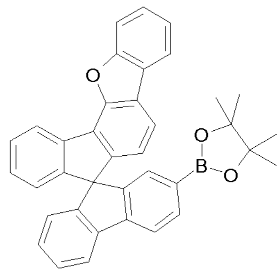 | 89% |

**Synthese der Verbindung (4-1)**

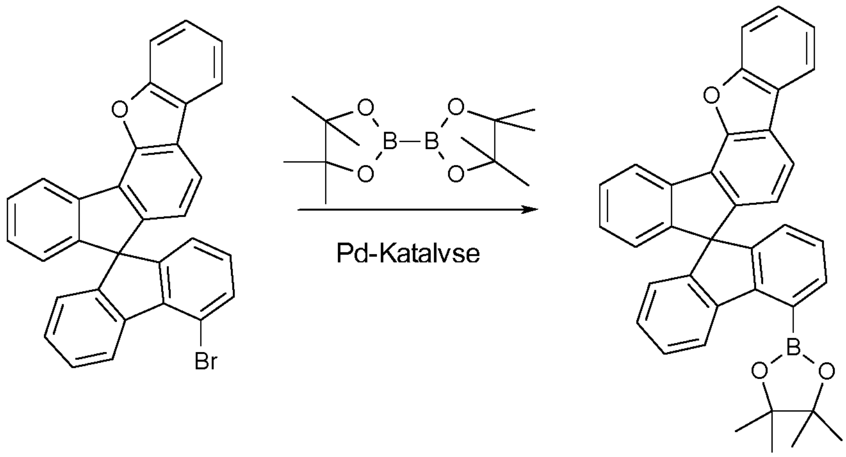


**Int-41**

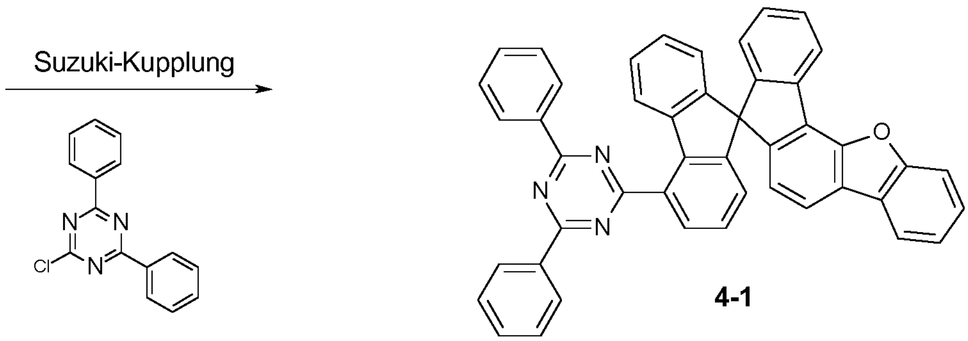


**4-1**

24,6 g (46,3 mmol) Spirofluoren-pinacolboronester Derivat und 20,0 g (46,3 mmol) Chlor-Derivat werden in 300 mL Dioxan und 14.1 g Caesiumfluorid (92,6 mmol) suspendiert. Zu dieser Suspension werden 4,1 g (5,56 mmol) Palladium dichlorid-bis(tricyclohexylphosphin) gegeben, und die Reaktionsmischung wird 24 h unter Rückfluss erhitzt. Nach Erkalten wird die organische Phase abgetrennt, über Kieselgel filtriert, dreimal mit 100 mL Wasser gewaschen und anschließend zur Trockne eingeengt. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 29,7 g (80% d. Th).

**Synthese der Verbindungen (4-2) bis (4-18)**

Analog zu der zuvor beschriebenen Synthese von Verbindung **(4-1)** werden auch die folgenden Verbindungen **(4-2)** bis **(4-18)** hergestellt.

| | Edukt 1 | Edukt 2 | Produkt | Ausbeute |
|---|---|---|---|---|
| **4-2** | 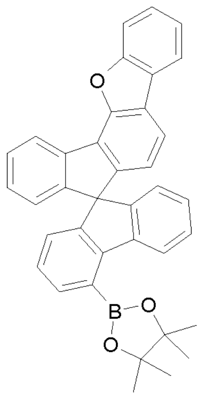 | 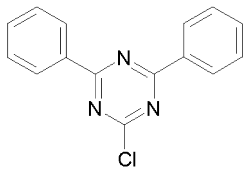 [3842-55-5] | 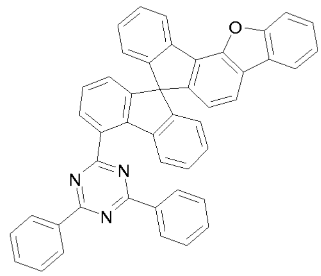 # | 78 % |
| **4-3** | 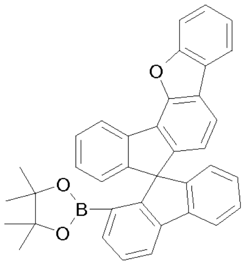 | 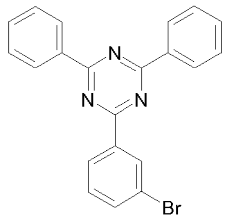 864377-31-1] | 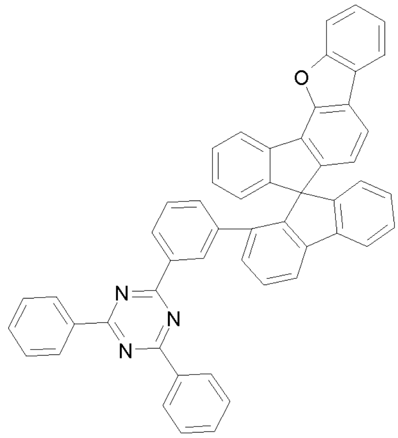 # | 71% |
| **4-4** | 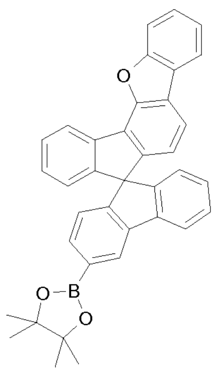 | 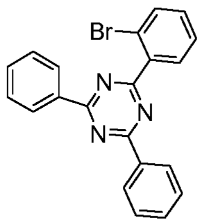 77989-15-2 | 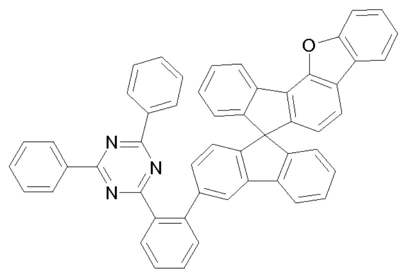 # | 80% |

(fortgesetzt)

| | Edukt 1 | Edukt 2 | Produkt | Ausbeute |
|---|---|---|---|---|
| **4-5** | 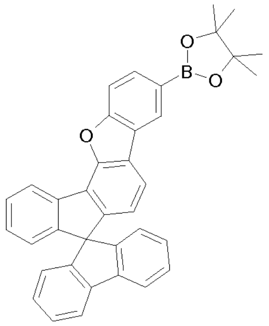 | 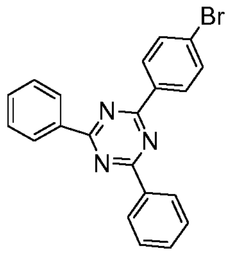 23449-08-3 | 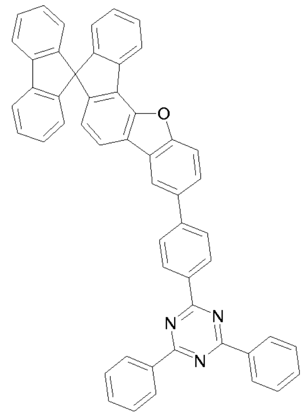 | 83% |
| **4-6** | 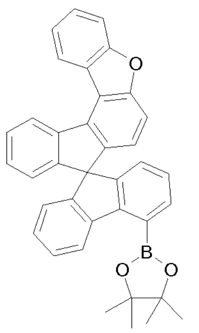 | 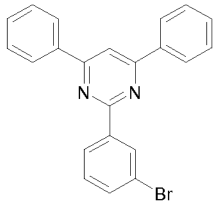 [864377-22-0] | 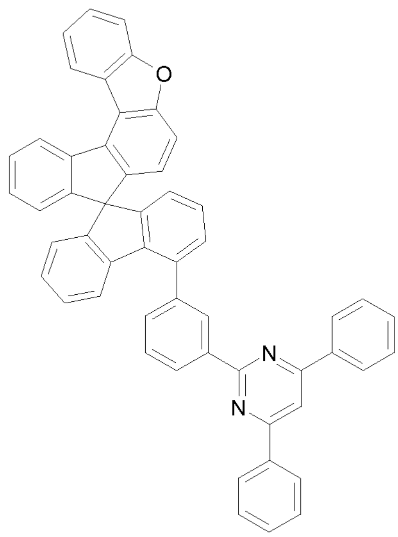 # | 69% |
| **4-7** | 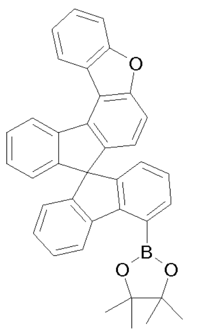 | 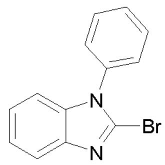 [1418123-78-0] | 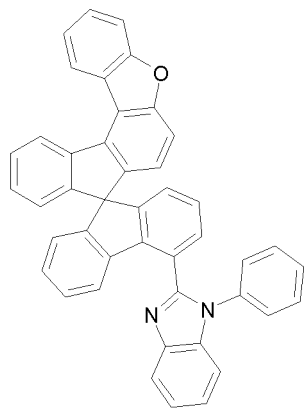 # | 72% |

(fortgesetzt)

| | Edukt 1 | Edukt 2 | Produkt | Ausbeute |
|---|---|---|---|---|
| **4-8** | 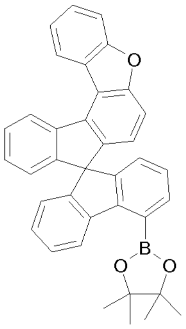 | 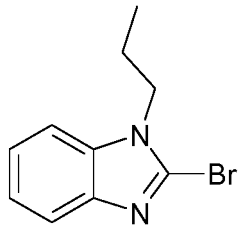 [1379359-00-8] | 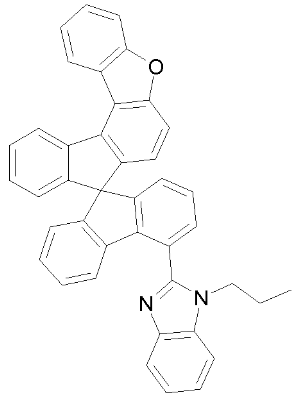 | 76% |
| **4-9** | 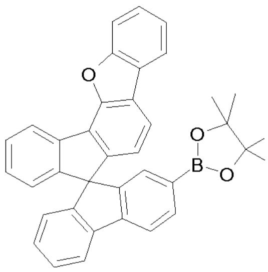 | 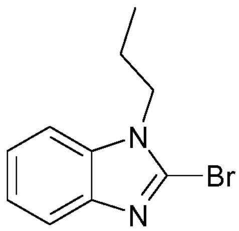 [1379359-00-8] | 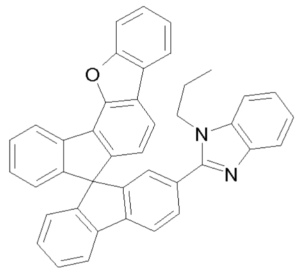 | 74% |
| **4-10** | 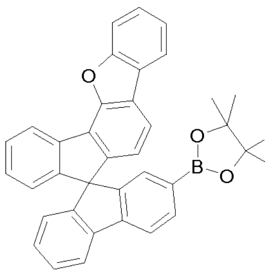 | 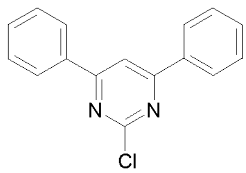 [56181-49-8] | 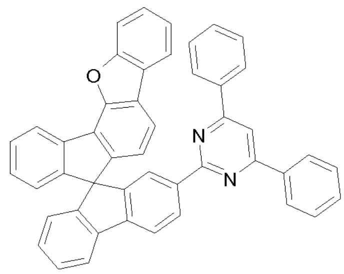 | 73% |
| **4-11** | 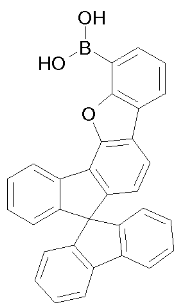 | 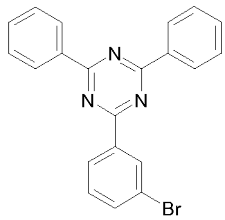 864377-31-1] | 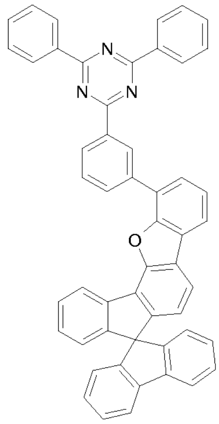 | 76% |

(fortgesetzt)

| | Edukt 1 | Edukt 2 | Produkt | Ausbeute |
|---|---|---|---|---|
| **4-12** | 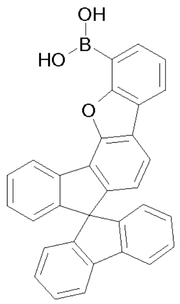 | 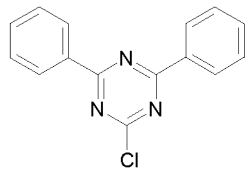 [3842-55-5] | 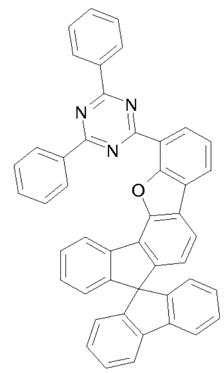 | 74% |
| **4-13** | 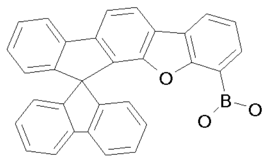 | 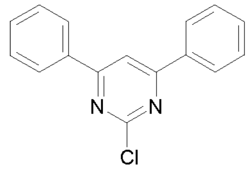 [56181-49-8] | 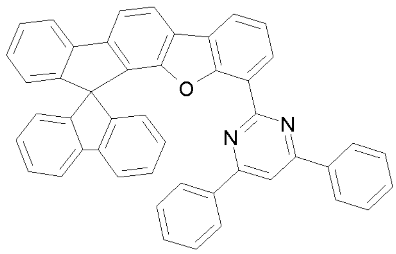 | 71% |
| **4-14** | 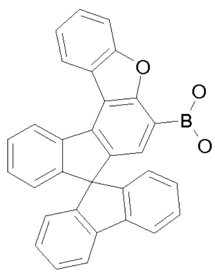 | 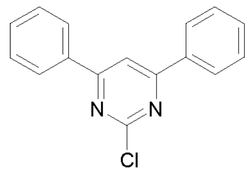 [56181-49-8] | 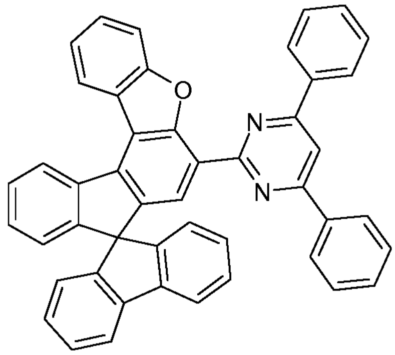 | 68% |
| **4-15** | 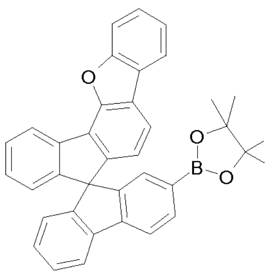 | 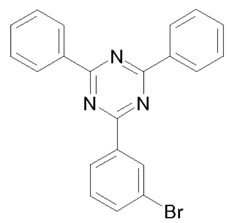 864377-31-1] | 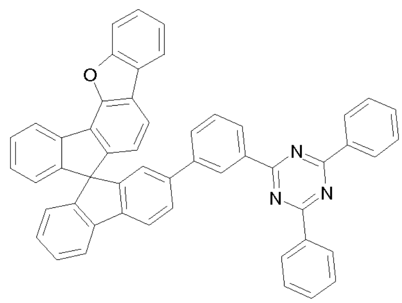 # | 82% |

(fortgesetzt)

| | Edukt 1 | Edukt 2 | Produkt | Ausbeute |
|---|---|---|---|---|
| **4-16** | 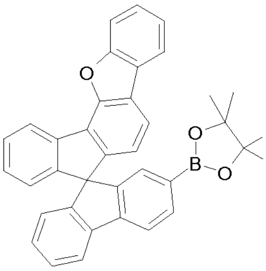 | 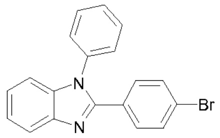 [2620-76-0] | 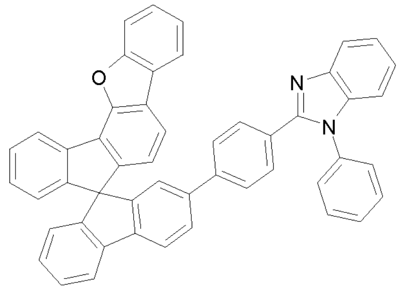 # | 83% |
| **4-17** | 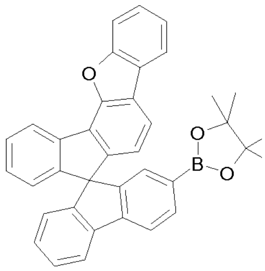 | 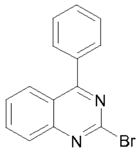 [354574-58-6] | 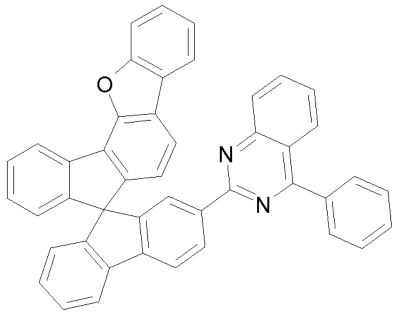 # | 78% |
| **4-18** | 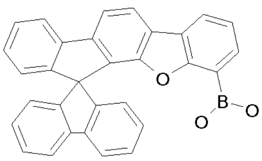 | 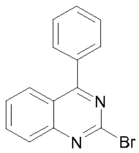 [354574-58-6] | 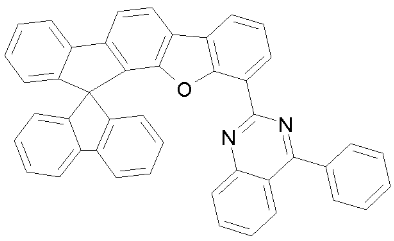 | 86% |
| # nicht erfindungsgemäß | | | | |

**A-5) Beispiel 5: Synthese der Verbindungen 5-1 bis 5-5**

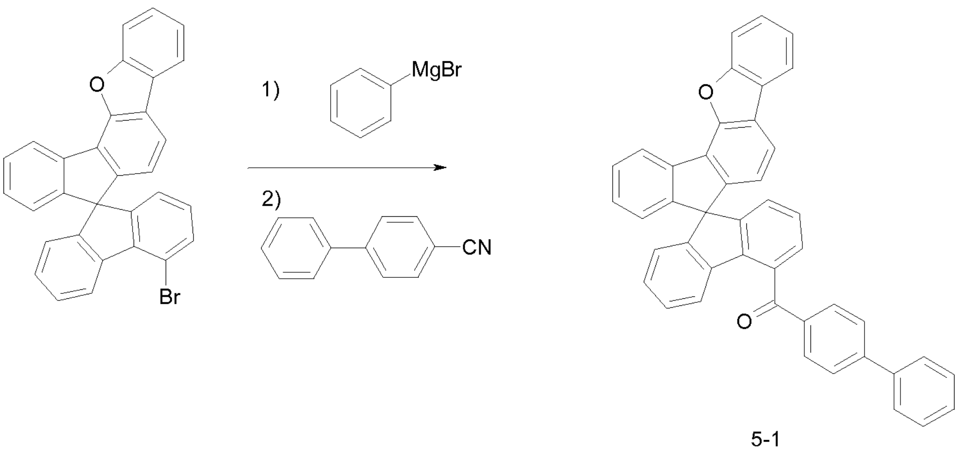


5-1 #

# nicht erfindungsgemäß

Eine Suspension von 48,5 g (100 mmol) Bromspirofluoren-Derivat in 800 ml THF wurde bei Raumtemperatur während 15 min. mit 330 ml einer Phenylmagnesiumbromid-Lösung (1.0 molar in THF) versetzt. Anschließend wurde noch 30 min. bei Raumtemperatur und 5 h unter Rückfluß gerührt. Nach Erkalten wurden 100 ml 5 N HCl und 100 ml Ethanol zugegeben und erneut 16 h unter Rückfluß erhitzt. Nach Erkalten wurde die wäßrige Phase abgetrennt, die organische Phase wurde zur Trockene eingeengt. Der Rückstand wurde in 1000 ml Dichlormethan aufgenommen und fünfmal mit je 500 ml Wasser gewaschen. Nach Trocknen über Magnesiumsulfat wurde die organische Phase zur Trockene eingeengt. Der Rückstand wurde siebenmal aus DMSO (ca. 3 ml/g) umkristallisiert und im Vakuum getrocknet und aus Dichlormethan / *Toluol* umkristallisiert und abschließend im Hochvakuum (p = 5 x $10^{-5}$ mbar) sublimiert. Die Reinheit beträgt 99.9%(HPLC). Die Ausbeute von Verbindung (1-1) beträgt 40 g (68 mmol), entsprechend 70 % der Theorie

**Synthese der Verbindungen (5-2) bis (5-5)**

Analog zu der zuvor beschriebenen Synthese von Verbindung **(5-1)** werden auch die folgenden Verbindungen **(5-2)** bis **(5-5)** hergestellt.

| | Edukt 1 | Edukt 2 | Produkt | Ausbeute |
|---|---|---|---|---|
| **5-2** | 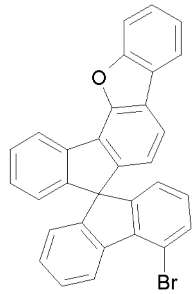 | 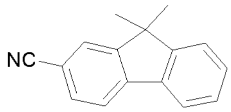 [890134-27-7] | 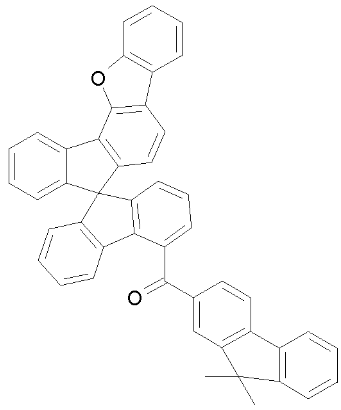 # | 69% |
| **5-3** | 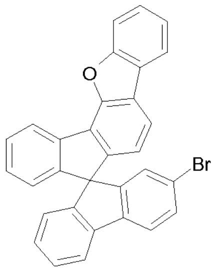 | 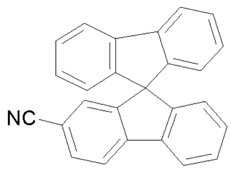 [67665-48-9] | 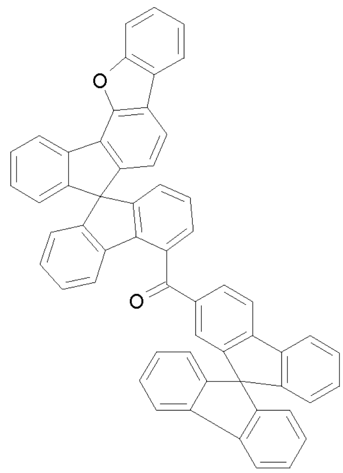 | 65% |

(fortgesetzt)

| | Edukt 1 | Edukt 2 | Produkt | Ausbeute |
|---|---|---|---|---|
| **5-4** | 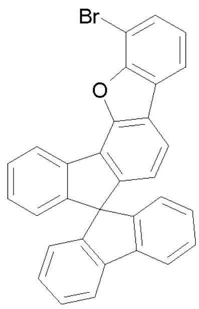 | 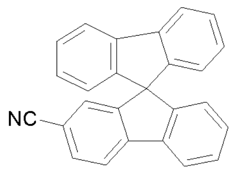 [67665-48-9] | 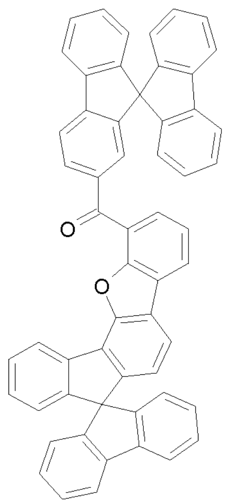 | 72% |
| **5-5** | 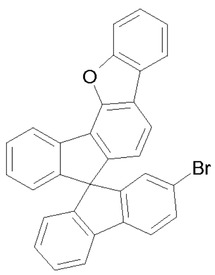 [1376933-75-3] | 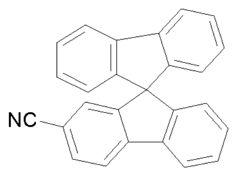 [67665-48-9] | 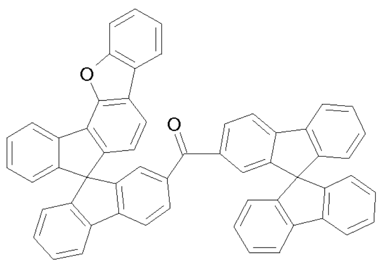 # | 79% |
| # nicht erfindungsgemäß | | | | |

**Herstellung der OLEDs**

In den folgenden Beispielen V1 bis E15 (siehe Tabellen 1 und 2) werden die Daten verschiedener OLEDs vorgestellt.

**Vorbehandlung für die Beispiele V1 bis E15:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$.

Die Daten der verschiedenen OLEDs sind in Tabelle 2 zusammengefasst. Die Beispiele V1 und V2 sind Vergleichsbeispiele gemäß dem Stand der Technik, die Beispiele E1 bis E15 zeigen Daten erfindungsgemäßer OLEDs.

Im Folgenden werden einige der Beispiele näher erläutert, um die Vorteile der erfindungsgemäßen OLEDs zu verdeutlichen.

**Verwendung von erfindungsgemäßen Mischungen in der Emissionsschicht phosphoreszenter OLEDs**

Die erfindungsgemäßen Materialien ergeben bei Einsatz als Matrixmaterialien in phosphoreszierenden OLEDs wesentliche Verbesserungen der Spannung und der externen Quanteneffizienz gegenüber dem Stand der Technik. Durch Einsatz der erfindungsgemäßen Verbindungen 1-18 in Kombination mit dem rot emittierenden Dotanden TER1 lässt sich eine Steigung der externen Quanteneffizienz um ca. 20% gegenüber dem Stand der Technik SdT1 beobachten (Beispiele V1 und E2).

**Verwendung von erfindungsgemäßen Verbindungen als Elektronentransportmaterialien**

Im Vergleich zu einer OLED, in der das Materialien SdT2 gemäß dem Stand der Technik in der ETL verwendet werden, beobachtet man beim Einsatz der erfindungsgemäßen Materialien 1-19 eine deutliche Verbesserung von Spannung und Leistungseffizienz (Beispiele V2 und E2).

Tabelle 1: Aufbau der OLEDs

| HTL/IL(HATCN; 5 nm)/EBL/EML/HBL/ETL/EIL | | | | | | |
|---|---|---|---|---|---|---|
| Bsp | HTL Dicke | EBL Dicke | EML Dicke | HBL Dicke | ETL Dicke | EIL Dicke |
| V1 | SpA1 90nm | SpMA1 130nm | SdT1:TER1 (92%:8%) 40nm | --- | ST2:LiQ (50%:50%) 40nm | --- |
| V2 | SpA1 70nm | SpMA1 90nm | IC1:TEG1 (90%:10%) 30nm | --- | SdT2:ST2 (50%:50%) 40nm | LiQ 3nm |
| E1 | SpA1 90nm | SpMA1 130nm | 1-18:TER1 (92%:8%) 30nm | --- | ST2:LiQ (50%:50%) 30nm | --- |
| E2 | SpA1 70nm | SpMA1 90nm | IC1:TEG1 (90%:10%) 30nm | --- | 1-19:ST2 (50%:50%) 40nm | LiQ 3nm |
| E3 | SpA1 70nm | SpMA1 90nm | 4-12:TEG1 (90%:10%) 30nm | --- | ST2:LiQ (50%:50%) 40nm | --- |
| E4 | SpA1 70nm | SpMA1 90nm | 4-1:TEG1 (90%:10%) 30nm | --- | ST2:LiQ (50%:50%) 40nm | --- |
| E5 | SpA1 70nm | SpMA1 90nm | 1-1:TEG1 (90%:10%) 30nm | --- | ST2:LiQ (50%:50%) 40nm | --- |
| E6 | SpA1 70nm | SpMA1 90nm | 1-5:TEG1 (90%:10%) 30nm | --- | ST2:LiQ (50%:50%) 40nm | --- |
| E7 | SpA1 70nm | SpMA1 90nm | 1-7:TEG1 (90%:10%) 30nm | --- | ST2:LiQ (50%:50%) 40nm | --- |
| E8 | SpA1 70nm | SpMA1 90nm | 1-9:TEG1 (90%:10%) 30nm | --- | ST2:LiQ (50%:50%) 40nm | --- |
| E9 | SpA1 70nm | SpMA1 90nm | IC1:TEG1 (90%:10%) 30nm | --- | 1-11:ST2 (50%:50%) 40nm | LiF 1nm |

(fortgesetzt)

| HTL/IL(HATCN; 5 nm)/EBL/EML/HBL/ETL/EIL | | | | | | |
|---|---|---|---|---|---|---|
| Bsp | HTL Dicke | EBL Dicke | EML Dicke | HBL Dicke | ETL Dicke | EIL Dicke |
| E10 | SpA1 70nm | SpMA1 90nm | 1-14:IC3:TEG1 (45%:45%:10%) 30nm | IC1 10nm | ST2:LiQ (50%:50%) 30nm | --- |
| E11 | SpA1 70nm | SpMA1 90nm | 1-15:IC3:TEG1 (45%:45%:10%) 30nm | IC1 10nm | ST2:LiQ (50%:50%) 30nm | --- |
| E12 | SpA1 70nm | SpMA1 90nm | IC1:3-3:TEG1 (45%:45%:10%) 30nm | IC1 10nm | ST2:LiQ (50%:50%) 30nm | --- |
| E13 | SpA1 90nm | SpMA1 130nm | 4-3:TER1 (92%:8%) 30nm | --- | ST2:LiQ (50%:50%) 30nm | --- |
| E14 | SpA1 70nm | SpMA1 90nm | IC1:TEG1 (90%:10%) 30nm | --- | 4-5:LIQ (50%:50%) 40nm | --- |
| E15 | SpA1 70nm | SpMA1 90nm | 4-11 :TEG1 (90%:10%) 30nm | --- | ST2:LiQ (50%:50%) 40nm | --- |

**Tabelle 2: Daten der OLEDs**

| Bsp. | U1000 (V) | SE1000 (cd/A) | LE1000 (lm/W) | EQE 1000 | CIE x/y bei 1000 $cd/m^2$ |
|---|---|---|---|---|---|
| V1 | 5.4 | 12 | 7 | 10.6% | 0.67/0.33 |
| V2 | 3.7 | 69 | 59 | 18.2% | 0.34/0.62 |
| E1 | 4.9 | 14 | 9 | 13.3% | 0.67/0.33 |
| E2 | 3.4 | 67 | 64 | 18.0% | 0.33/0.62 |
| E3 | 3.6 | 61 | 53 | 16.9% | 0.33/0.62 |
| E4 | 3.3 | 63 | 60 | 17.2% | 0.34/0.62 |
| E5 | 3.5 | 64 | 61 | 17.4% | 0.34/0.62 |
| E6 | 3.6 | 66 | 58 | 17.6% | 0.34/0.62 |
| E7 | 3.3 | 64 | 61 | 17.3% | 0.33/0.62 |
| E8 | 3.8 | 55 | 45 | 15.4% | 0.34/0.62 |
| E9 | 3.4 | 58 | 54 | 16.4% | 0.33/0.62 |
| E10 | 3.5 | 60 | 54 | 16.7% | 0.34/0.63 |
| E11 | 3.7 | 61 | 52 | 16.9% | 0.33/0.62 |
| E12 | 3.6 | 56 | 49 | 16.0% | 0.34/0.62 |
| E13 | 5.1 | 15 | 9 | 13.8% | 0.67/0.33 |
| E14 | 3.6 | 66 | 58 | 18.4% | 0.34/0.62 |
| E15 | 3.9 | 60 | 48 | 16.6% | 0.33/0.62 |

Tabelle 3: Strukturformeln der Materialien für die OLEDs

| | |
|---|---|
| 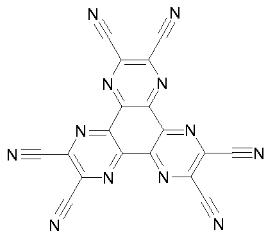 | 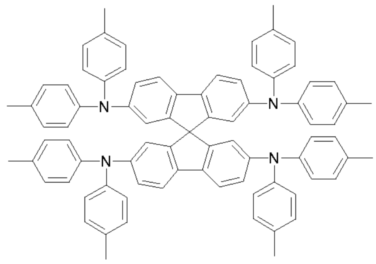 |
| HATCN | SpA1 |
| 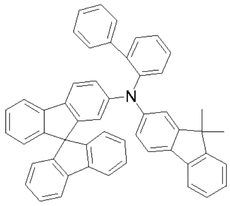 | 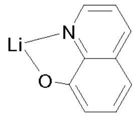 |
| SpMA1 | LiQ |
| 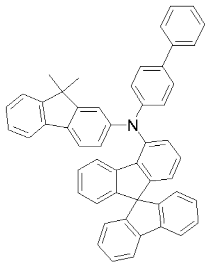 | 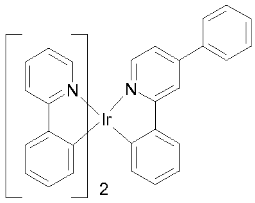 |
| SpMA2 | TEY1 |
| 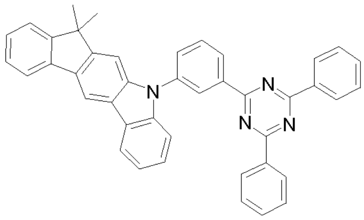 | 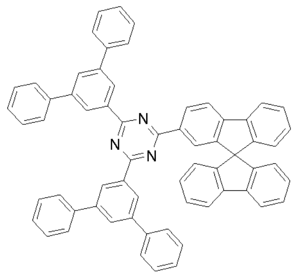 |
| IC2 | ST2 |
| 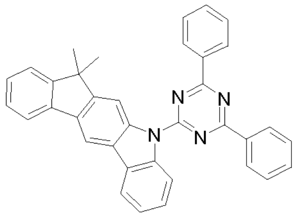 | 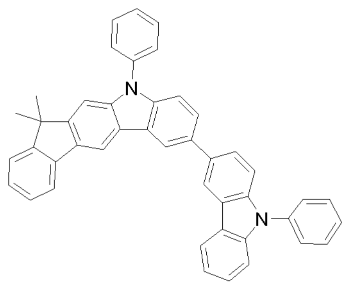 |
| IC1 | IC3 |

(fortgesetzt)

| | |
|---|---|
| 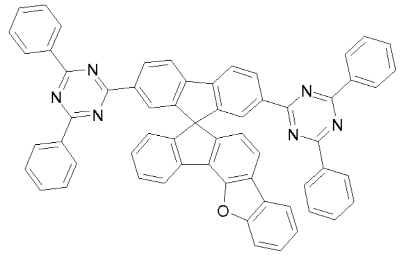 | 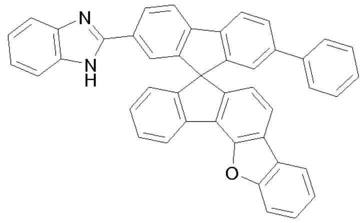 |
| SdT1 | SdT2 |
| 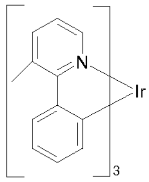 | 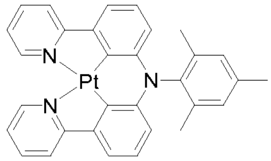 |
| TEG1 | TER1 |
| 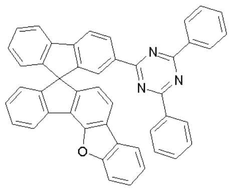 | 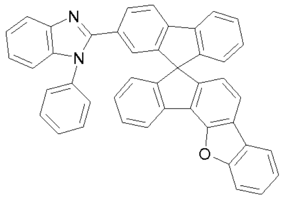 |
| 1-18# | 1-19# |
| 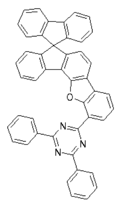 | 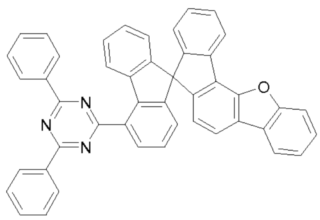 |
| 4-12 # | 4-1 # |
| 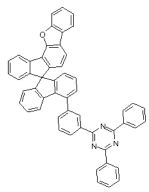 | 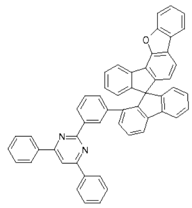 |
| 1-1 # | 1-5 # |
| 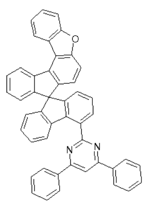 | 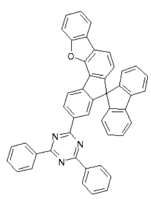 |
| 1-7 # | 1-9 # |

(fortgesetzt)

| | |
|---|---|
| 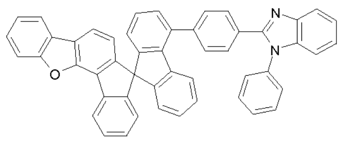 | 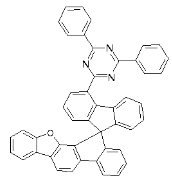 |
| 1-11 # | 1-14 # |
| 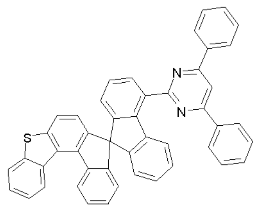 | 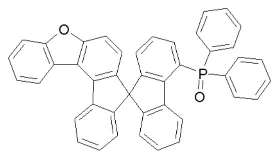 |
| 1-15 # | 3-3 # |
| 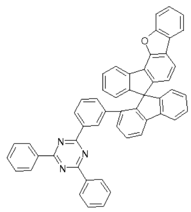 | 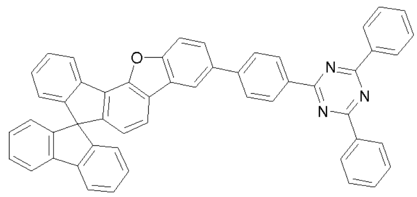 |
| 4-3 # | 4-5 |
| 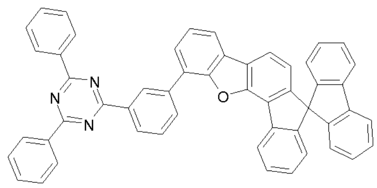 | |
| 4-11 | |
| # nicht erfindungsgemäß | |

## Patentansprüche

**1.** Verbindung, umfassend Strukturen der Formel (I)

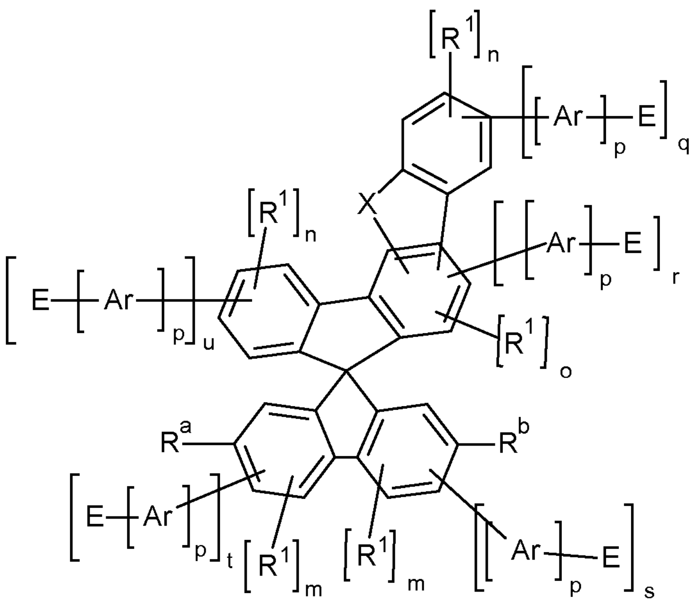

Formel (I)

wobei für die verwendeten Symbole gilt:

X ist O;

Ar ist bei jedem Auftreten gleich oder verschieden eine Arylgruppe mit 6 bis 40 C-Atomen oder eine Heteroarylgruppe mit 3 bis 40 C-Atomen, welche jeweils durch einen oder mehrere Reste $R^1$ substituiert sein kann;

$R^1$ ist bei jedem Auftreten gleich oder verschieden H, D, F, Cl, Br, I, eine geradkettige Alkylgruppe mit 1 bis 40 C-Atomenoder ein aromatisches Ringsystem mit 6 bis 40 aromatischen Ringatomen, das jeweils durch einen oder mehrere Reste $R^2$ substituiert sein kann;

$R^2$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch C=C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei Reste $Ar^1$, welche an dasselbe Phosphoratom binden, durch eine Einfachbindung oder eine Brücke, ausgewählt aus $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ und $P(=O)R^3$, miteinander verknüpft sein;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

n ist bei jedem Auftreten gleich oder verschieden 0, 1, 2, 3 oder 4

m ist bei jedem Auftreten gleich oder verschieden 0, 1, 2 oder 3;

o ist bei jedem Auftreten gleich oder verschieden 0, 1 oder 2;

q ist 1;

p, r, s, t, u ist bei jedem Auftreten gleich 0;

E ist eine elektronentransportierende Gruppe, welche jeweils durch einen oder mehrere Reste $R^1$ substituiert sein kann;

$R^a$ ist $R^1$;

$R^b$ ist $R^1$;

mit der Maßgabe, dass
die Struktur der Formel (I) genau eine elektronentransportierende Gruppe E aufweist;
die Summe von sämtlichen Indices m, s und t kleiner als 7 ist;
die Summe von sämtlichen Indices n, q und u kleiner als 9 ist;
und wobei die Summe der Indices r und o kleiner als 3 ist.

**2.** Verbindungen gemäß Anspruch 1, **dadurch gekennzeichnet, dass** die Summe aller n, m und o kleiner oder gleich 4, vorzugsweise kleiner gleich 2 ist.

**3.** Verbindungen gemäß Anspruch 1 oder 2, **dadurch gekennzeichnet, dass** p 0 ist, so dass E unmittelbar an die Benzofuran- oder Spiro-Gruppe gebunden ist.

**4.** Verbindungen gemäß mindestens einem der vorhergehenden Ansprüche, **dadurch gekennzeichnet, dass** die elektronentransportierende Gruppe E eine Heteroarylgruppe mit 5 bis 60 aromatischen Ringatomen ist.

**5.** Verbindungen gemäß Anspruch 4, **dadurch gekennzeichnet, dass** die elektronentransportierende Gruppe E mindestens eine Struktur umfasst, die aus der Gruppe Triazine, Pyrimidine, Pyrazine, Imidazole, Benzimidazole und Pyridine ausgewählt ist.

**6.** Verbindungen gemäß mindestens einem der vorhergehenden Ansprüche, **dadurch gekennzeichnet, dass** die Verbindung Strukturen der Formeln (II), (III), (IV), (V), (VI), und/oder (VII)

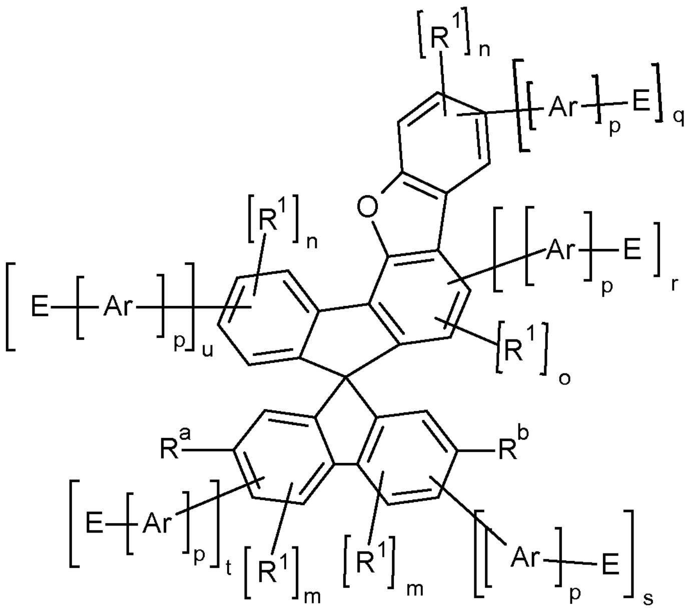

Formel (II)

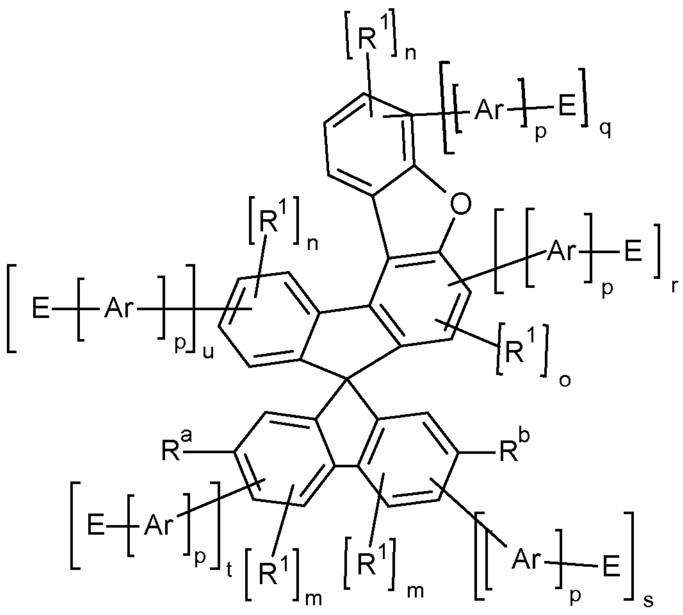

Formel (III)

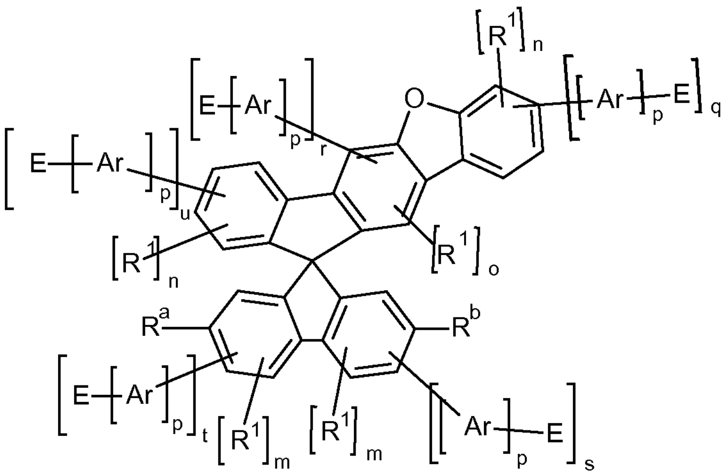

Formel (IV)

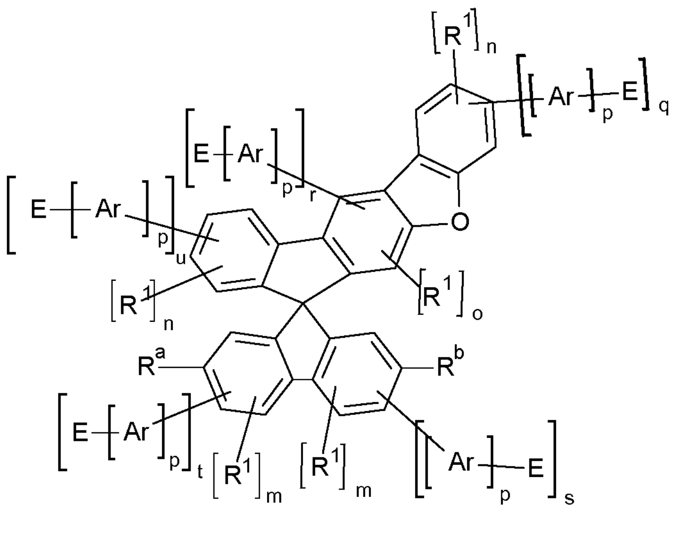

Formel (V)

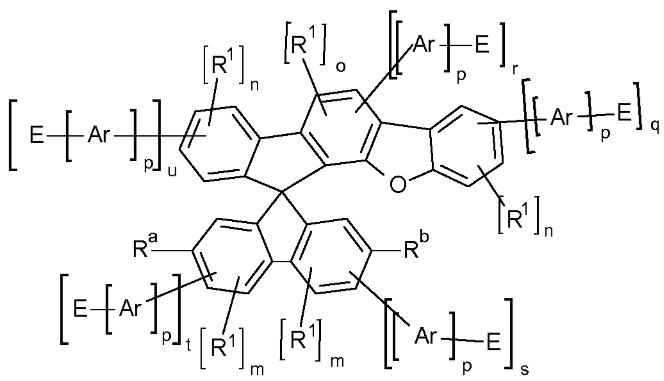

Formel (VI)

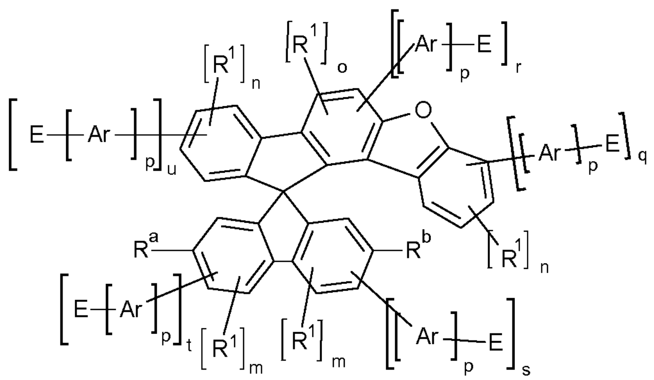

Formel (VII)

umfassen, wobei die dargelegten Symbole die in Anspruch 1 dargelegte Bedeutung haben.

7. Zusammensetzung enthaltend wenigstens eine Verbindung nach einem oder mehreren der Ansprüche 1 bis 6 und wenigstens ein organisch funktionelles Material.

8. Zusammensetzung gemäß Anspruch 7, **dadurch gekennzeichnet, dass** das organisch funktionelle Material ein Elektronentransportmaterial ist.

**9.** Formulierung enthaltend mindestens eine Verbindung nach einem oder mehreren der Ansprüche 1 bis 6 und/oder wenigstens eine Zusammensetzung nach Anspruch 7 oder 8 und mindestens ein Lösungsmittel.

**10.** Verfahren zur Herstellung einer Verbindung nach einem oder mehreren der Ansprüche 1 bis 6 **dadurch gekennzeichnet, dass** zunächst das Spirobifluoren-Grundgerüst hergestellt wird, und in einem späteren Schritt über eine Kupplungsreaktion ein Rest mit einer elektronentransportierenden Gruppe eingeführt wird.

**11.** Verwendung einer Verbindung nach einem oder mehreren der Ansprüche 1 bis 6 oder einer Zusammensetzung nach Anspruch 7 oder 8 in einer elektronischen Vorrichtung als Lochblockiermaterial, Elektroneninjektionsmaterial, und/oder Elektronentransportmaterial.

**12.** Organische Elektrolumineszenzvorrichtung enthaltend mindestens eine Verbindung nach einem oder mehreren der Ansprüche 1 bis 6 oder eine Zusammensetzung nach Anspruch 7 oder 8.

## Claims

**1.** Compound containing structures of the formula (I)

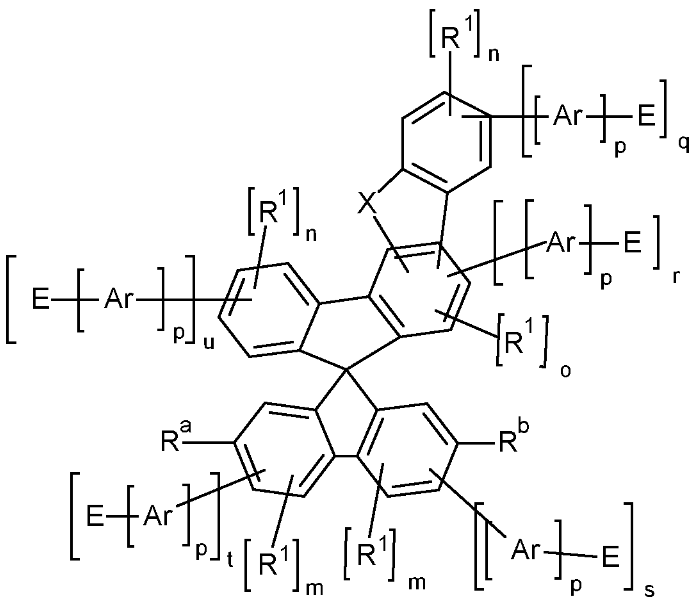

formula (I)

where the following applies to the symbols used:

X is O;
Ar is on each occurrence, identically or differently, an aryl group having 6 to 40 C atoms or a heteroaryl group having 3 to 40 C atoms, which may in each case be substituted by one or more radicals $R^1$;
$R^1$ is on each occurrence, identically or differently, H, D, F, Cl, Br, I, a straight-chain alkyl group having 1 to 40 C atoms or an aromatic ring system having 6 to 40 aromatic ring atoms, which may in each case be substituted by one or more radicals $R^2$;
$R^2$ is on each occurrence, identically or differently, H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by C=C, $Si(R^3)_2$, $Ge(R^3)_2$, $Sn(R^3)_2$, C=O, C=S, C=Se, $P(=O)(R^3)$, SO, $SO_2$, O, S or $CONR^3$ and where one or more H atoms may be replaced by D, F, Cl, Br, I, CN or $NO_2$, or a combination of these systems; two or more adjacent substituents $R^2$ may also form a mono- or polycyclic, aliphatic or aromatic ring system with one another;
$Ar^1$ is on each occurrence, identically or differently, an aromatic or heteroaromatic ring system having 5 to 30

aromatic ring atoms, which may be substituted by one or more radicals $R^2$; two radicals $Ar^1$ which are bonded to the same phosphorus atom may also be linked to one another by a single bond or a bridge selected from $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ and $P(=O)R^3$;
$R^3$ is on each occurrence, identically or differently, H, D, F or an aliphatic, aromatic and/or heteroaromatic hydrocarbon radical having 1 to 20 C atoms, in which, in addition, H atoms may be replaced by F; two or more adjacent substituents $R^3$ may also form a mono- or polycyclic, aliphatic or aromatic ring system with one another;
n is on each occurrence, identically or differently, 0, 1, 2, 3 or 4;
m is on each occurrence, identically or differently, 0, 1, 2 or 3;
o is on each occurrence, identically or differently, 0, 1 or 2;
q is 1;
p, r, s, t, u are on each occurrence equal to 0;
E is an electron-transporting group, which may in each case be substituted by one or more radicals $R^1$;
$R^a$ is $R^1$;
$R^b$ is $R^1$;

with the proviso that
the structure of the formula (I) contains precisely one electron-transporting group E;
the sum of all the indices m, s and t is less than 7;
the sum of all indices n, q and u is less than 9;
and where the sum of the indices r and o is less than 3.

2. Compounds according to Claim 1, **characterised in that** the sum of all n, m and o is less than or equal to 4, preferably less than or equal to 2.

3. Compounds according to Claim 1 or 2, **characterised in that** p is 0, so that E is bonded directly to the benzofuran or spiro group.

4. Compounds according to at least one of the preceding claims, **characterised in that** the electron-transporting group E is a heteroaryl group having 5 to 60 aromatic ring atoms.

5. Compounds according to Claim 4, **characterised in that** the electron-transporting group E contains at least one structure selected from the group triazines, pyrimidines, pyrazines, imidazoles, benzimidazoles and pyridines.

6. Compounds according to at least one of the preceding claims, **characterised in that** the compound contains structures of the formulae (II), (III), (IV), (V), (VI) and/or (VII)

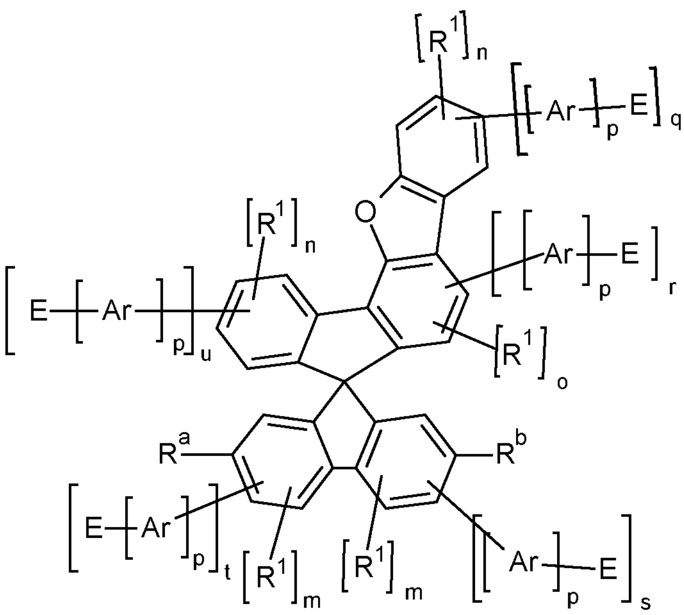

formula (II)

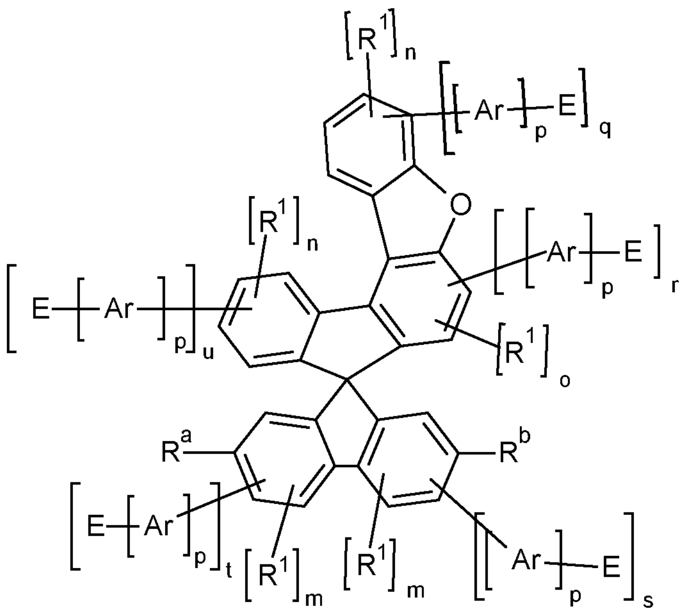

formula (III)

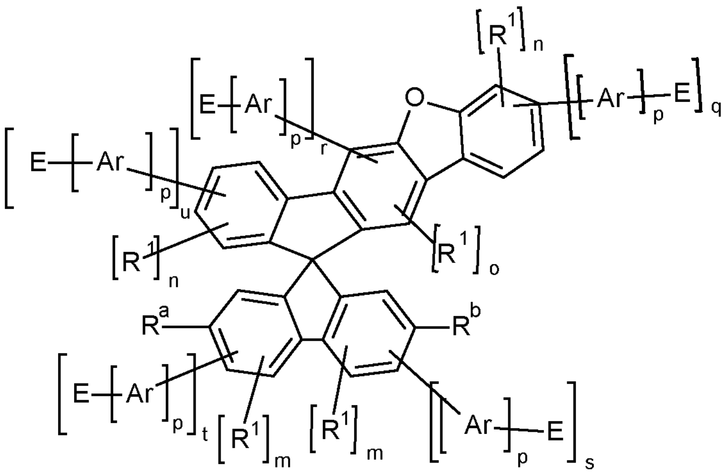

formula (IV)

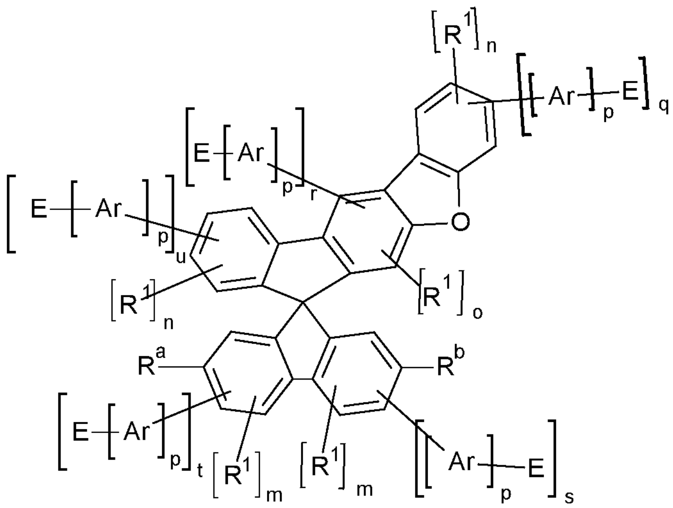

formula (V)

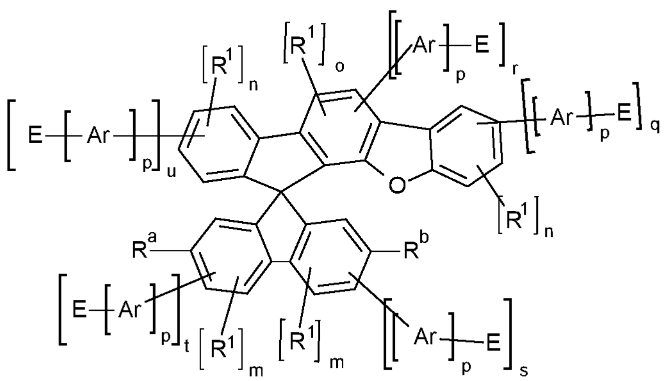

formula (VI)

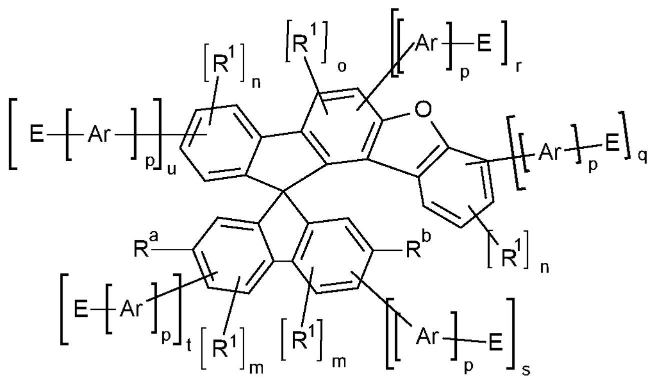

formula (VII),

where the symbols shown have the meaning described in Claim 1.

7. Composition comprising at least one compound according to one or more of Claims 1 to 6 and at least one organofunctional material.

8. Composition according to Claim 7, **characterised in that** the organofunctional material is an electron-transport material.

**9.** Formulation comprising at least one compound according to one or more of Claims 1 to 6 and/or at least one composition according to Claim 7 or 8 and at least one solvent.

**10.** Process for the preparation of a compound according to one or more of Claims 1 to 6, **characterised in that** firstly the spirobifluorene skeleton is prepared, and, in a later step, a radical containing an electron-transporting group is introduced via a coupling reaction.

**11.** Use of a compound according to one or more of Claims 1 to 6 or a composition according to Claim 7 or 8 as hole-blocking material, electron-injection material and/or electron-transport material in an electronic device.

**12.** Organic electroluminescent device containing at least one compound according to one or more of Claims 1 to 6 or a composition according to Claim 7 or 8.

## Revendications

**1.** Composé contenant les structures de la formule (I)

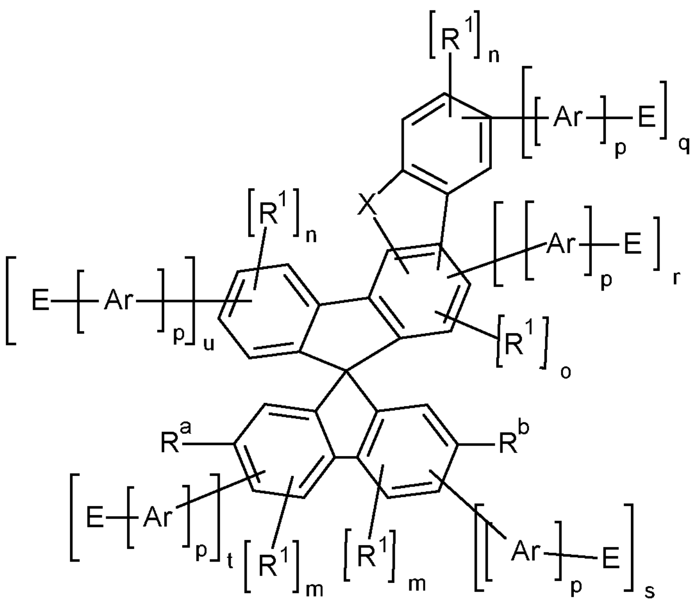

formule (I)

dans laquelle ce qui suit s'applique aux symboles qui sont utilisés :

X est O ;
Ar est pour chaque occurrence, de manière identique ou différente, un groupe aryle qui comporte de 6 à 40 atomes de C ou un groupe hétéroaryle qui comporte de 3 à 40 atomes de C, lequel peut dans chaque cas être substitué par un radical ou par plusieurs radicaux $R^1$ ;
$R^1$ est pour chaque occurrence, de manière identique ou différente, H, D, F, Cl, Br, I, un groupe alkyle en chaîne droite qui comporte de 1 à 40 atome(s) de C ou un système de cycle aromatique qui comporte de 6 à 40 atomes de cycle aromatique, lequel peut dans chaque cas être substitué par un radical ou par plusieurs radicaux $R^2$ ;
$R^2$ est pour chaque occurrence, de manière identique ou différente, H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par C=C, $Si(R^3)_2$, $Ge(R^3)_2$, $Sn(R^3)_2$, C=O, C=S, C=Se, $P(=O)(R^3)$, SO, $SO_2$, O, S ou $CONR^3$ et où un ou plusieurs atome(s) de H peut/peuvent être remplacé(s) par D, F, Cl, Br, I, CN ou $NO_2$, ou une combinaison de ces systèmes ; deux substituants $R^2$ adjacents ou plus peuvent également former un système de cycle aliphatique ou aromatique

mono- ou polycyclique l'un avec l'autre ou les uns avec les autres ;
$Ar^1$ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux $R^2$ ; deux radicaux $Ar^1$ qui sont liés au même atome de phosphore peuvent également être liés l'un à l'autre par une liaison simple ou par un pont qui est sélectionné parmi $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ et $P(=O)R^3$;
$R^3$ est pour chaque occurrence, de manière identique ou différente, H, D, F ou un radical hydrocarbone aliphatique, aromatique et/ou hétéroaromatique qui comporte de 1 à 20 atome(s) de C, où, en outre, des atomes de H peuvent être remplacés par F ; deux substituants $R^3$ adjacents ou plus peuvent également former un système de cycle aliphatique ou aromatique mono- ou polycyclique l'un avec l'autre ou les uns avec les autres ;
n est pour chaque occurrence, de manière identique ou différente, 0, 1, 2, 3 ou 4 ;
m est pour chaque occurrence, de manière identique ou différente, 0, 1, 2 ou 3 ;
o est pour chaque occurrence, de manière identique ou différente, 0, 1 ou 2 ;
q est 1 ;
p, r, s, t, u sont pour chaque occurrence égaux à 0 ;
E est un groupe de transport d'électrons, lequel peut dans chaque cas être substitué par un radical ou par plusieurs radicaux $R^1$ ;
$R^a$ est $R^1$ ;
$R^b$ est $R^1$ ;

étant entendu que
la structure de la formule (I) contient de façon précise un seul groupe de transport d'électrons E ;
la somme de tous les indices m, s et t est inférieure à 7 ;
la somme de tous les indices n, q et u est inférieure à 9 ;
et où la somme des indices r et o est inférieure à 3.

2. Composés selon la revendication 1, **caractérisés en ce que** la somme de tous les indices n, m et o est inférieure ou égale à 4, de préférence inférieure ou égale à 2.

3. Composés selon la revendication 1 ou 2, **caractérisés en ce que** p est 0, de telle sorte que E soit lié directement au groupe benzofurane ou spiro.

4. Composés selon au moins l'une des revendications qui précèdent, **caractérisés en ce que** le groupe de transport d'électrons E est un groupe hétéroaryle qui comporte de 5 à 60 atomes de cycle aromatique.

5. Composés selon la revendication 4, **caractérisés en ce que** le groupe de transport d'électrons E contient au moins une structure qui est sélectionnée parmi le groupe qui est constitué par les triazines, les pyrimidines, les pyrazines, les imidazoles, les benzimidazoles et les pyridines.

6. Composés selon au moins l'une des revendications qui précèdent, **caractérisés en ce que** le composé contient les structures des formules (II), (III), (IV), (V), (VI) et/ou (VII)

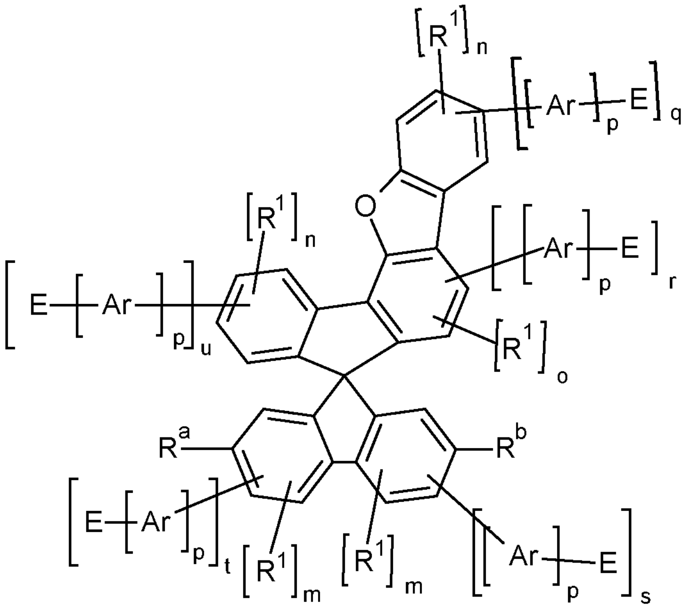

formule (II)

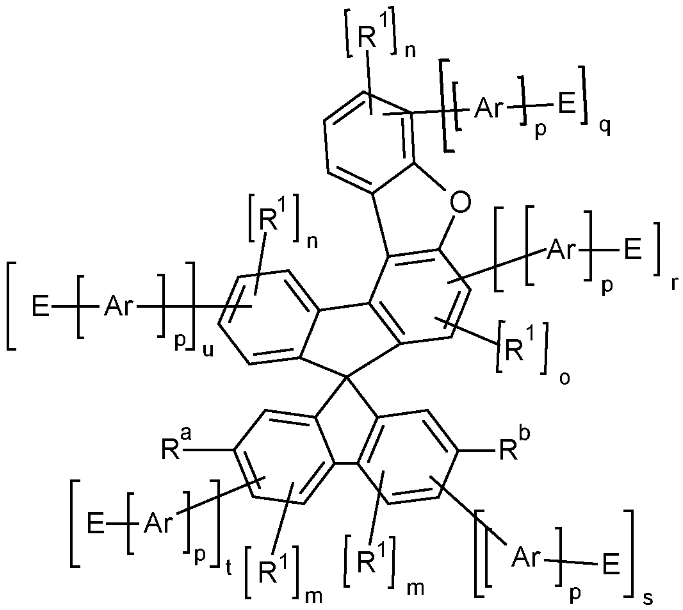

formule (III)

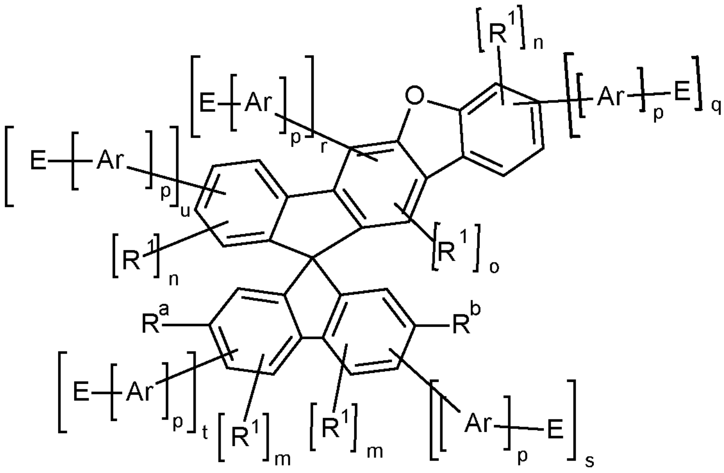

formule (IV)

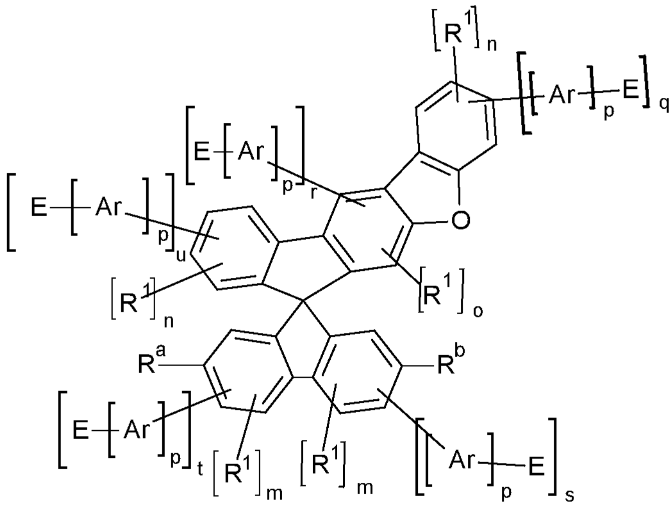

formule (V)

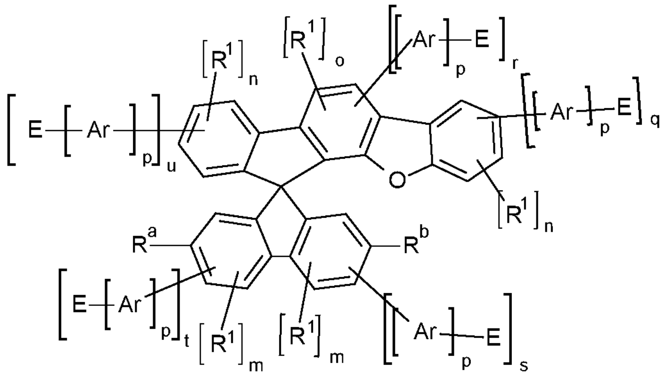

formule (VI)

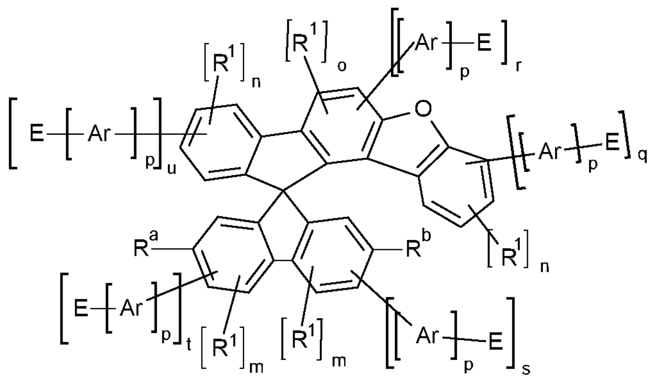

formule (VII),

dans lesquelles les symboles représentés présentent la signification qui a été décrite selon la revendication 1.

**7.** Composition comprenant au moins un composé selon une ou plusieurs des revendications 1 à 6 et au moins un matériau organofonctionnel.

**8.** Composition selon la revendication 7, **caractérisée en ce que** le matériau organofonctionnel est un matériau de transport d'électrons.

**9.** Formulation comprenant au moins un composé selon une ou plusieurs des revendications 1 à 6 et/ou au moins une composition selon la revendication 7 ou 8 et au moins un solvant.

**10.** Procédé pour la préparation d'un composé selon une ou plusieurs des revendications 1 à 6, **caractérisé en ce que**, en premier lieu, le squelette spirobifluorène est préparé et, au niveau d'une étape ultérieure, un radical qui contient un matériau de transport d'électrons est introduit via une réaction de couplage.

**11.** Utilisation d'un composé selon une ou plusieurs des revendications 1 à 6 ou d'une composition selon la revendication 7 ou 8 en tant que matériau de blocage de trous, matériau d'injection d'électrons et/ou matériau de transport d'électrons dans un dispositif électronique.

**12.** Dispositif électroluminescent organique contenant au moins un composé selon une ou plusieurs des revendications 1 à 6 ou une composition selon la revendication 7 ou 8.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- WO 2013100464 A1
- US 2013256645 A1
- DE 102009032922 A1
- JP 2009266927 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- WO 2005086251 A2
- WO 2012175535 A1
- WO 2012175219 A1
- WO 2012168358 A1
- WO 2012031735 A1
- EP 1837926 A1
- WO 2007107306 A1
- EP 2452946 A1
- EP 2463927 A1
- WO 2009000237 A1
- US 2007145355 A1
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2005011013 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- WO 2009148015 A
- US 20090136779 A
- WO 2010050778 A
- WO 2011042107 A
- WO 2011088877 A
- WO 2010108579 A


### In der Beschreibung aufgeführte Nicht-Patentliteratur


- **M. S. ARNOLD et al.** *Appl. Phys. Lett.,* 2008, vol. 92, 053301